# FASCICULE DE BREVET EUROPEEN

(11) **EP 0 578 735 B1**
(45) Date de publication et mention de la délivrance du brevet: **03.01.1996**
(21) Numéro de dépôt: 92909502.4
(22) Date de dépôt: 30.03.1992
(51) Int. Cl.: A61K 7/13, C07D 209/08

(54) **PROCEDE DE TEINTURE DES FIBRES KERATINIQUES AVEC DES AMINOINDOLES, A pH ACIDE ET COMPOSITIONS MISES EN OEUVRE ET NOUVEAUX COMPOSES AMINOINDOLES**
VERFAHREN ZUM FÄRBEN VON KERATIN-FASERN MIT AMINOINDOLEN BEIM SÄURE pH UND VERWENDUNG DAVON IN ZUSAMMENSETZUNGEN UND NEUE AMINOINDOL-VERBINDUNGEN
NOVEL KERATIN FIBRE DYEING METHOD USING AMINOINDOLES, WITH ACID pH, COMPOSITIONS THEREFOR AND NOVEL AMINOINDOLE COMPOUNDS

(30) Priorité: 28.03.1991 FR 9103787
(43) Date de publication de la demande: 19.01.1994
(73) Titulaire: L'OREAL, F-75008 Paris (FR)
(72) Inventeur: LANG, Gérard, F-95210 Saint-Gratien (FR); JUNINO, Alex, F-93180 Livry-Gargan (FR); COTTERET, Jean, F-78480 Verneuil-sur-Seine (FR); LAGRANCE, Alain, 77700 Coupvray (FR)
(74) Mandataire: Casalonga, Axel
(86) Numéro de dépôt international: FR9200288
(87) Numéro de publication internationale: WO9217157

(56) Documents cités:
- EP-A- 0 271 186
- EP-A- 0 348 280
- EP-A- 0 376 776
- EP-A- 0 424 261
- EP-A- 0 425 345
- EP-A- 0 428 442
- EP-A- 0 460 996
- US-A- 4 013 404

## Description

La présente invention est relative à un procédé de teinture des fibres kératiniques, en particulier des fibres kératiniques humaines, mettant en oeuvre des aminoindoles en association avec des bases d'oxydation et un agent oxydant en milieu acide et aux compositions mises en oeuvre au cours de ce procédé ainsi que des nouveaux composés aminoindoles.

Il est connu de teindre les fibres kératiniques et en particulier les cheveux humains avec des compositions tinctoriales contenant en milieu alcalin des précurseurs de colorants d'oxydation et en particulier des p-phénylènediamines, des ortho ou para-aminophénols appelés généralement "bases d'oxydation".

On sait également que l'on peut faire varier les nuances obtenues avec ces bases d'oxydation en les associant à des coupleurs encore appelés modificateurs de coloration, choisis notamment parmi les métadiamines aromatiques, les méta-aminophénols et les métadiphénols.

Certains conposés aminoindoles sont connus en teinture par oxydation des fibres kératiniques comme coupleurs en milieu alcalin, tels que ceux de la demande EP 0 424 261.

La demande EP 0 425 345 décrit des composés aminoindoles utilisés comme précurseurs de colorant par oxydation.

La demande EP 0 428 442 décrit certains aminoindoles tels que le 5-acétylamino 6-hydroxy 2,3-diméthylindole utilisés comme coupleurs en milieu alcalin.

La demande EP 0 460 996 décrit certains composés aminoindoles utilisés dans un procédé de teinture des fibres kératiniques comprenant l, application de l'aminoindole suivie ou précédée de l'application d'un dérivé quinonique ; les aminoindoles et les dérivés quinoniques étant choisis de façon à ce que la différence de potentiels d'oxydo-réduction entre leurs potentiels d'oxydo-réduction respectifs soit inférieure ou égale à 470 mV. Parmi les aminoindoles utilisés est cité le 5-acétylamino 6-méthoxy 2,3-diméthylindole.

Le brevet USP 4 013 404 illustre également des composés aminoindoles essentiellement utilisés comme précurseurs de colorant d'oxydation en teinture des fibres kératiniques.

La demanderesse vient de découvrir' de façon surprenante que l'utilisation de certains composés du type aminoindole associés à des bases d'oxydation, lorsque cette association était appliquée en présence d'un agent oxydant et à pH acide sur les cheveux, conduisait à des colorations présentant une excellente puissance tinctoriale.

Les résultats obtenus sont particulièrement surprenants lorsqu'on les compare à ceux obtenus avec des coupleurs classiques tels que mentionnés ci-dessus où l'on constate souvent une perte de puissance tinctoriale et une ténacité plus faible lorsqu'on opère à pH acide.

Les colorations ainsi obtenues présentent une excellente ténacité à la lumière, aux lavages, à la transpiration et aux intempéries.

La présente invention a donc pour objet un procédé de teinture des fibres kératiniques, en particulier des fibres kératiniques humaines telles que les cheveux, comprenant l'application sur ces fibres d'une composition contenant au moins un aminoindole de formule (I) telle que définie ci-après, d'un précurseur de colorant d'oxydation encore appelé base d'oxydation et d'un agent oxydant, à pH acide,

L'invention a également pour objet un agent de teinture à deux composants, dont l'un des composants comprend l'aminoindole et le précurseur du colorant d'oxydation et l'autre l'agent oxydant à un pH acide, et en des quantités telles que le mélange présente un pH acide.

Un certain nombre de dérivés d'aminoindole de formule (I) telle que définie ci-après, appliqués sur les fibres kératiniques, en association avec des précurseurs de colorant par oxydation, constituent de nouveaux "coupleurs" pour la teinture d'oxydation.

Un autre objet consiste également en des compositions de teinture d'oxydation contenant un précurseur de colorant d'oxydation et l'un de ces composés particuliers comme coupleur.

L'invention a également pour objet la composition prête à l'emploi, contenant les différents agents utilisés pour la teinture, en milieu acide, des cheveux,

L'invention concerne également de nouveaux composés aminoindoles.

D'autres objets de l'invention apparaîtront à la lecture de la description et des exemples qui suivent.

Le procédé de teinture des fibres kératiniques et en particulier des fibres kératiniques humaines telles que les cheveux, conforme à l'invention, est essentiellement caractérisé par le fait que l'on applique sur ces fibres une composition contenant dans un milieu approprié pour la teinture, au moins un coupleur répondant à la formule : 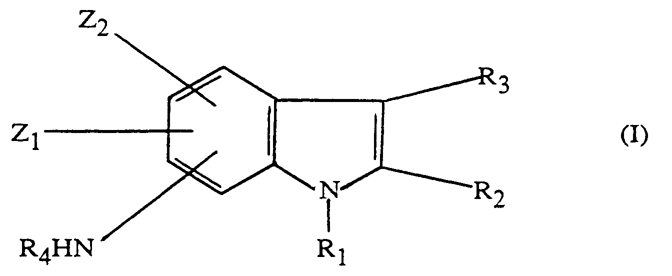
dans laquelle :
R₁ désigne hydrogène ou un groupement alkyle en C₁-C₄;
R₂ et R₃, indépendamment l'un de l'autre, représentent un atome d'hydrogène, un groupement alkyle en C₁-C₄ ou un groupement COOR', R' étant un radical alkyle en C₁-C₄ ou un atome d'hydrogène;
R₄ représente un atome d'hydrogène, un radical alkyle en C₁-C₄, un groupement hydroxyalkyle en C₁-C₄, polyhydroxyalkyle en C₂-C₄, acétyle ou aminoalkyle en C₁-C₆ dont l'amine peut être éventuellement mono- ou disubstituée par un alkyle en C₁-C₄;
Z₁, Z₂, identiques ou différents, représentent un atome d'hydrogène ou un groupement alkyle en C₁-C₄, un hydroxy, un halogène, alcoxy (C₁-C₄);
et les sels de tous ces composés;
- au moins un précurseur de colorant d'oxydation;
- au moins un agent oxydant; le pH de la composition appliquée sur les fibres étant inférieur à 7.

Les composés préférés répondant à la formule (I), utilisés conformément à l'invention, sont les suivants :
(1) le 5-aminoindole,
(2) le 4-aminoindole,
(3) le 2-méthyl 6-aminoindole,
(4) le 2,3,4-triméthyl 6-aminoindole,
(5) le 2,3,7-triméthyl 6-aminoindole,
(6) le 2,3,5-triméthyl 6-aminoindole,
(7) le 6-aminoindole,
(8) le 7-aminoindole,
(9) le 6-(β-hydroxyéthylamino)indole,
(10) le 2-méthyl 5-hydroxy 6-aminoindole,
(11) le 2,3-diméthyl 5-amino 6-hydroxyindole,
(12) le 2,3-diméthyl 5-méthoxy 6-aminoindole,
(15) le 2,3-diméthyl 5-chloro 6-aminoindole,
(16) le 2,3-diméthyl 5-acétylamino 6-méthoxyindole,
(17) le 2,3-diméthyl 5-amino 6-méthoxyindole,
(18) le N-méthyl 6-(β-hydroxyéthyl)aminoindole,
(19) le 2,3,4,5-tétraméthyl 6-aminoindole.

On peut également citer les composés suivants :
(20) le 6-N-méthylaminoindole,
(21) le N-méthyl 5-aminoindole,
(22) le N-méthyl 6-aminoindole,
(23) le N-méthyl 4-aminoindole,
(24) le 2,3-diméthyl 4-aminoindole,
(25) le 2,3-diméthyl 7-aminoindole,
(26) le 3-méthyl 6-aminoindole.
(27) le 2-méthyl 3-éthyl 6-aminoindole,
(28) le 2-methyl 3-éthyl 7-aminoindole.
(29) le 4-N-méthylaminoindole.
(30) le 7-éthyl 4-aminoindole,
(31) le 7-éthyl 6-aminoindole,
(32) le 7-éthyl 6-N,β-hydroxyéthylaminoindole,
(33) le 4-méthyl 6-aminoindole,
(34) le 5-méthyl 6-aminoindole,
(35) le 7-méthyl 4-aminoindole,
(36) le 3-méthyl 7-éthyl 6-aminoindole,
(37) le 5,7-diméthyl 6-aminoindole,
(38) le 5,7-diéthyl 6-aminoindole,
(39) le 2-éthoxycarbonyl 5-méthyl 7-aminoindole.
(40) le 2-éthoxycarbonyl 5-chloro 7-aminoindole,
(41) le 2-éthoxycarbonyl 5-éthoxy 7-aminoindole,
(42) le 2-éthoxycarbonyl 5-méthoxy 7-aminoindole,
(43) le 5-méthoxy 7-(4'-diméthylamino 1'-méthylbutyl)aminoindole,
(44) le 5-méthoxy 7-(4'-diméthylaminobutyl)aminoindole,
(45) le 5-méthoxy 7-(4'-diéthylamino 1'-méthylbutyl)aminoindole.
(46) le 5-fluoro 6-aminoindole,
(47) le 5-fluoro 1-sec-butyl 6-aminoindole,
(48) le 5-fluoro 1-n-propyl 6-aminoindole,
(49) le 1-méthyl 2-méthoxycarbonyl 5-méthoxy 6-aminoindole,
(50) le 2-méthoxycarbonyl 5-méthoxy 6-aminoindole,
(51) le 2-éthoxycarbonyl 5-méthoxy 6-aminoindole,
(52) le 2-carboxy 5-méthoxy 6-aminoindole,
(53) le 1,2-diméthyl 5-hydroxy 6-aminoindole,
(54) le 2-méthoxycarbonyl 4-méthoxy 6-aminoindole.

Parmi ces composés, le 5-aminoindole, le 4-aminoindole, le 2-méthyl 6-aminoindole, le 7-aminoindole, le 6-aminoindole, le 6-(β-hydroxyéthylamino)indole, le 2-méthyl 5-hydroxy 6-aminoindole et le 2,3-diméthyl 5-amino 6-méthoxyindole, sont plus particulièrement préférés.

Les sels sont choisis plus particulièrement parmi les chlorhydrates ou bromhydrates.

Les précurseurs de colorants d'oxydation ou bases d'oxydation sont des composés connus qui ne sont pas des colorants en eux-mêmes et qui forment un colorant par un processus de condensation oxydative, soit sur eux-mêmes, soit en présence d'un coupleur ou modificateur. Ces composés comportent généralement un noyau aromatique portant des groupements fonctionnels, constitués : soit par deux groupements amino; soit par un groupement amino et un groupement hydroxy; ces groupements étant en position para ou ortho, l'un par rapport à l'autre.

Les précurseurs de colorants d'oxydation de type para, utilisés conformément à l'invention, sont choisis parmi les paraphénylènediamines, les bases dites "doubles", les para-amino phénols, les précurseurs hétérocycliques para, comme la 2,5-diamino pyridine, la 2-hydroxy 5-aminopyridine, la 2,4,5,6-tétraaminopyrimidine.

Parmi les paraphénylènediamines, on peut citer les composés répondant à la formule (II) : 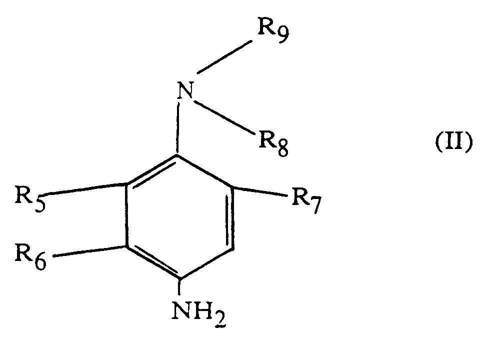
dans laquelle :
R₅, R₆ et R₇, identiques ou différents, représentent un atome d'hydrogène ou d'halogène, un radical alkyle ayant de 1 à 4 atomes de carbone, un radical alcoxy ayant de 1 à 4 atomes de carbone, un radical carboxy, sulfo, hydroxyalkyle ayant de 1 à 4 atomes de carbone;
R₈ et R₉, identiques ou différents, représentent un atome d'hydrogène, un radical alkyle, hydroxyalkyle, alcoxyalkyle, carbamyl alkyle, mésylaminoalkyle, acétylaminoalkyle, uréidoalkyle, carbalcoxy aminoalkyle, pipéridinoalkyle, morpholinoalkyle; ces groupes alkyle ou alcoxy ayant de 1 à 4 atomes de carbone, ou bien R₈ et R₉ forment, conjointement avec l'atome d'azote auquel ils sont liés, un hétérocycle pipéridino ou morpholino, sous réserve que R₅ ou R₇ représente un atome d'hydrogène lorsque R₈ et R₉ ne représentent pas un atome d'hydrogène, ainsi que les sels de ces composés.

Parmi les composés particulièrement préférés répondant à la formule (II), on peut citer la p-phénylènediamine, la 2-méthyl-p-phénylènediamine, la méthoxyparaphénylènediamine, la chloroparaphénylènediamine, la 2,6-diméthylparaphénylènediamine, la 2,5-diméthylparaphénylènediamine, la 2,3-diméthylparaphénylènediamine, la 2-méthyl 5-méthoxyparaphénylènediamine, la 2,6-diméthyl 5-méthoxyparaphénylènediamine, la N,N-diméthylparaphénylène diamine, la 3-méthyl 4-amino N,N-diéthylaniline, la N,N-di-(β-hydroxyéthyl)paraphénylènediamine, la 3-méthyl 4-amino N,N-di-(β-hydroxyéthyl)aniline, la 3-chloro 4-amino N,N-di-(β-hydroxyéthyl)aniline, la 4-amino N,N-(éthyl,carbamylméthyl)aniline, la 3-méthyl 4-amino N,N-(éthyl,carbamylméthyl)aniline, la 4-amino N,N-(éthyl,β-pipéridinoéthyl)aniline, la 3-méthyl 4-amino N,N-(éthyl,β-pipéridinoéthyl) aniline, la 4-amino N,N-(éthyl,β-morpholinoéthyl)aniline, la 3-méthyl 4-amino N,N-(éthyl,β-morpholinoéthyl)aniline, la 4-amino N,N-(éthyl, β-acétylaminoéthyl)aniline, la 4-amino N-(β-méthoxyéthyl)aniline, la 3-méthyl 4-amino N,N-(éthyl,β-acétylaminoéthyl)aniline, la 4-amino N,N-(éthyl,β-mésylaminoéthyl)aniline, la 3-méthyl 4-amino N,N-(éthyl,β-mésylaminoéthyl)aniline la 4-amino N,N-(éthyl,β-sulfoéthyl) aniline, la 3-méthyl 4-amino N,N-(éthyl,β-sulfoéthyl)aniline, la N-[(4'-amino)phényl]morpholine, la N-[(4'-amino)phényl]pipéridine, la 2-hydroxyéthylparaphénylènediamine, la fluoroparaphénylènediamine, la carboxyparaphénylènediamine, la sulfoparaphénylènediamine la 2-isopropylparaphénylènediamine, la 2-n-propylparaphénylènediamine. la 2-hydroxyméthylparaphénylènediamine, la N,N-diméthyl 3-méthyl paraphénylènediamine, la N,N(éthyl,β-hydroxyéthyl)paraphénylène diamine, la N-(dihydroxypropyl)paraphénylènediamine, la N-4'-aminophényl p-phénylènediamine, la N-phényl p-phénylènediamine.

Ces précurseurs de colorants par oxydation de type para peuvent être introduits dans la composition tinctoriale, soit sous forme de base libre, soit sous forme de sels, tels que sous forme de chlorhydrate, de bromhydrate ou de sulfate.

Les bases dites doubles sont des bis-phénylalkylènediamines, répondant à la formule : 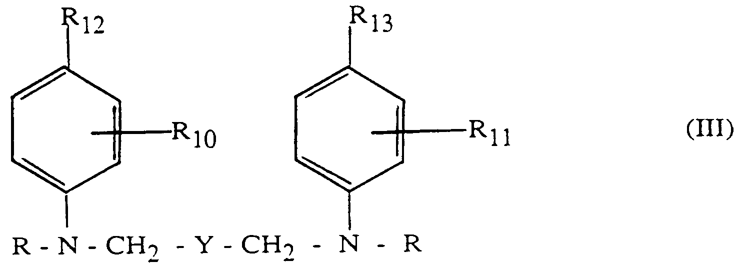
dans laquelle :
R₁₂ et R₁₃, identiques ou différents, représentent des groupements hydroxyle ou NHR₁₄, où R₁₄ désigne un atome d'hydrogène ou un radical alkyle inférieur;
R₁₀ et R₁₁, identiques ou différents, représentent soit des atomes d'hydrogène, soit des atomes d'halogène, soit encore des groupements alkyle;
R représente un atome d'hydrogène, un groupe alkyle, hydroxyalkyle ou aminoalkyle, dont le reste amino peut être substitué;
Y représente un radical pris dans le groupe constitué par les radicaux suivants :
-(CH₂)ₙ-, (CH₂)_{n'}-O-(CH₂)_{n'}-,
(CH₂)_{n'}-CHOH-(CH₂)ₙ,;-(CH₂)ₙ, 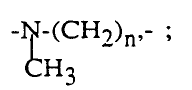
n étant un nombre entier compris entre 0 et 8 et n' un nombre entier compris entre 0 et 4, cette base pouvant se présenter sous forme de ses sels d'addition avec des acides.

Les radicaux alkyle ou alcoxy désignent de préférence un groupement ayant 1 à 4 atomes de carbone et notamment méthyle, éthyle, propyle, méthoxy, éthoxy.

Parmi les composés de formule (III), on peut citer le N,N'bis-(β-hydroxyéthyl)N,N'-bis(4'-aminophényl)1,3-diamino 2-propanol, la N,N'bis-(β-hydroxyéthyl)N,N'-bis(4'-aminophényl)éthylènediamine, la N,N'bis-(4-aminophényl)tétraméthylènediamine, la N,N'bis-(β-hydroxy éthyl)N,N'bis(4-aminophényl)tétraméthylènediamine, la N,N'bis-(4-méthylaminophényl)tétraméthylènediamine, la N,N'bis(éthyl)N,N'-bis (4'-amino 3'-méthylphényl)éthylènediamine.

Parmi les p-aminophénols, on peut citer le p-aminophénol, le 2-méthyl 4-aminophénol, le 3-méthyl 4-aminophénol, le 2-chloro 4-aminophénol, le 3-chloro 4-aminophénol, le 2,6-diméthyl 4-aminophénol, le 3,5-diméthyl 4-aminophénol, le 2,3-diméthyl 4-amino phénol, le 2-hydroxyméthyl 4-aminophénol, le 2-(β-hydroxyéthyl) 4-aminophénol, le 2-méthoxy 4-aminophénol, le 3-méthoxy 4-amino phénol, le 2,5-diméthyl 4-aminophénol, le 2-méthoxyméthyl 4-amino phénol, le 2-β-hydroxyéthoxyméthyl 4-aminophénol.

Les précurseurs de colorants d'oxydation de type ortho sont choisis parmi les orthoaminophénols, comme le 1-amino 2-hydroxy benzène, le 6-méthyl 1-hydroxy 2-aminobenzène, le 4-méthyl 1-amino 2-hydroxybenzène et les orthophénylènediamines.

L'agent oxydant est choisi de préférence parmi le peroxyde d'hydrogène, le peroxyde d'urée, les bromates de métaux alcalins, les persels tels que les perborates et les persulfates. Le peroxyde d'hydrogène est particulièrement préféré.

Le pH de la composition appliquée sur les fibres kératiniques, en particulier les cheveux, a une valeur inférieure à 7 et est compris de préférence entre 3 et 6,9. Ce pH est ajusté par l'utilisation d'agents acidifiants bien connus dans le domaine de la teinture des fibres kératiniques, et en particulier des cheveux humains, tels que des acides minéraux comme l'acide chlorhydrique ou l'acide phosphorique, ou organiques comme les acides carboxyliques tels que l'acide tartrique ou citrique.

Les composés de formule (I) sont présents dans la composition appliquée sur les fibres kératiniques, dans des proportions comprises de préférence entre 0,01 et 3,5% en poids par rapport au poids total de la composition.

Les compositions, définies ci-dessus, appliquées dans la teinture des fibres kératiniques, peuvent également contenir en plus des coupleurs hétérocycliques de formule (I), d'autres coupleurs connus en eux-mêmes tels que des métadiphénols, des métaaminophénols, des métaphénylènediamines, des méta N-acylaminophénols, des métauréidophénols, des métacarbalcoxyaminophénols, l'α-naphtol, des coupleurs possédant un groupement méthylène actif, tels que les composés dicétoniques, les pyrazolones, les coupleurs hétérocycliques ou le 4-hydroxyindole, le 6-hydroxyindole ou le 7-hydroxyindole.

Parmi ces coupleurs pouvant être utilisés en plus des coupleurs de formule (I), on peut citer le 2,4-dihydroxyphénoxyéthanol, le 2,4-dihydroxyanisole, le métaaminophénol, la résorcine, le monométhyl éther de résorcine, la 2-méthylrésorcine, le pyrocatéchol, le 2-méthyl 5-N-(β-hydroxyéthyl)aminophénol, le 2-méthyl 5-N-(β-mésylamino éthyl)aminophénol, la 6-hydroxybenzomorpholine, le 2,4-diamino anisole, le 2,4-diaminophénoxyéthanol, la 6-aminobenzomorpholine, le [2-N-(β-hydroxyéthyl)amino 4-amino]-phénoxyéthanol, le 2-amino 4-N-(β-hydroxyéthyl)aminoanisole, le (2,4-diamino)phényl-β,γ-dihydroxypropyléther, la 2,4-diaminophénoxyéthylamine, le 1,3-diméthoxy 2,4-diaminobenzène, le 2-méthyl 5-aminophénol, le 2,6-diméthyl 3-aminophénol, le 3,4-méthylènedioxyphénol, la 3,4-méthylènedioxy aniline, la 2-chlororésorcine et leurs sels.

Ces compositions peuvent également contenir des agents tensioactifs anioniques, cationiques, non-ioniques, amphotères ou leurs mélanges.

Parmi ces agents tensio-actifs, on peut citer les alkylbenzène sulfonates, les alkylnaphtalènesulfonates, les sulfates, les éthersulfates et les sulfonates d'alcools gras, les sels d'ammonium quaternaires tels que le bromure de triméthylcétylammonium, le bromure de cétyl pyridinium, les éthanolamides d'acides gras éventuellement oxyéthylénés, les acides, les alcools ou les amines polyoxyéthylénés, les alcools ou alphadiols polyglycérolés, les alkylphénols polyoxyéthylénés ou polyglycérolés, ainsi que les alkylsulfates polyoxyéthylénés.

Les compositions tinctoriales sont généralement aqueuses, mais elles peuvent également contenir des solvants organiques pour solubiliser des composés qui ne seraient pas suffisamment solubles dans l'eau. Parmi ces solvants, on peut citer à titre d'exemple, les alcanols inférieurs en C₂-C₄ tels que l'éthanol et l'isopropanol, le glycérol, les glycols ou éthers de glycols, comme le butoxy-2 éthanol, l'éthylèneglycol, le propylèneglycol, le monoéthyléther et le mono méthyléther du diéthylèneglycol, le monoéthyléther et le monométhyl éther du propylèneglycol, ainsi que les alcools aromatiques comme l'alcool benzylique ou le phénoxyéthanol, ou les mélanges de ces solvants.

La composition appliquée sur les cheveux peut également renfermer des agents épaississants choisis en particulier parmi l'alginate de sodium, la gomme arabique, les dérivés de cellulose tels que la méthylcellulose, l'hydroxyéthylcellulose, l'hydroxypropyl cellulose, l'hydroxyméthylcellulose, la carboxyméthylcellulose, les polymères d'acide acrylique éventuellement réticulés, la gomme de xanthane. On peut également utiliser des agents épaississants minéraux tels que la bentonite.

La composition peut également renfermer les agents antioxydants choisis en particulier parmi le sulfite de sodium, l'acide thioglycolique, le bisulfite de sodium, l'acide déhydroascorbique et l'hydroquinone, ainsi que d'autres adjuvants cosmétiquement acceptables lorsque la composition est destinée à être utilisée pour la teinture des fibres kératiniques humaines, tels que des agents de pénétration, des agents séquestrants, des conservateurs, des tampons, des parfums, etc...

La composition appliquée sur les cheveux peut se présenter sous des formes diverses, telles que de liquides, de crèmes, de gels ou sous toute autre forme appropriée pour réaliser une teinture capillaire. Elle peut être conditionnée en flacon aérosol en présence d'un agent propulseur.

L'invention a également pour objet la composition prête à l'emploi utilisée dans le procédé défini ci-dessus.

Selon une forme de lisation préférée, le procédé comporte une étape préliminaire consistant à stocker sous forme séparée, d'une part, la composition contenant dans un milieu approprié pour la teinture, le coupleur répondant à la formule (I) définie ci-dessus et les précurseurs de colorants par oxydation sous forme d'un composant (A) et, d'autre part, une composition renfermant l'agent oxydant tel que défini ci-dessus sous forme d'un composant (B), et à procéder à leur mélange extemporané avant d'appliquer ce mélange sur les fibres kératiniques, comme indiqué ci-dessus.

La composition appliquée sur les fibres kératiniques résulte d'un mélange de 10 à 90% du composant (A) avec 90 à 10% du composant (B) contenant un agent oxydant.

L'invention a également pour objet un agent de teinture des fibres kératiniques, en particulier des cheveux, essentiellement caractérisé par le fait qu'il comporte au moins deux composants, l'un des composants étant constitué par le composant (A) défini ci-dessus et l'autre étant constitué par le composant (B) également défini ci-dessus, le pH des composants (A) et (B) étant tel qu'après mélange dans des proportions de 90 à 10% pour le composant (A) et de 10 à 90% pour le composant (B), la composition résultante ait un pH inférieur à 7.

Dans cette forme de réalisation, le composant (A) qui renferme au moins le coupleur de formule (I) et un précurseur de colorant d'oxydation, peut avoir un pH compris entre 3 et 10,5 et peut être ajusté à la valeur choisie au moyen d'agents alcalinisants habituellement utilisés en teinture des fibres kératiniques, tels que l'ammoniaque, les carbonates alcalins, les alcanolamines tels que les mono-, di- et triéthanolamines, ainsi que leurs dérivés ou des agents acidifiants classiques, tels que les acides minéraux comme l'acide chlorhydrique ou l'acide phosphorique, ou organiques comme les acides carboxyliques tels que l'acide tartrique ou citrique.

Cette composition peut renfermer les différents autres adjuvants mentionnés ci-dessus, notamment des coupleurs différents des coupleurs amino-indoliques, répondant à la formule (I) déjà mentionnée ci-dessus.

L'ensemble des précurseurs de colorants par oxydation ainsi que les coupleurs, sont présents dans des proportions comprises de préférence entre 0,05 et 7% en poids par rapport au poids total du composant (A). La concentration en composés de formule (I) peut varier entre 0,012 et 4% en poids par rapport au poids total du composant (A).

Les agents tensio-actifs sont présents dans le composant (A) dans des proportions de 0,1 à 55% en poids. Lorsque le milieu contient des solvants en plus de l'eau, ceux-ci sont présents dans des proportions comprises entre 0,5 et 40% en poids et en particulier entre 5 et 30% en poids par rapport au poids total du composant (A). Les agents épaississants sont présents de préférence dans des proportions comprises entre 0,1 et 5%, et en particulier entre 0,2 et 3% en poids. Les agents antioxydants mentionnés ci-dessus sont de préférence présents dans le composant (A) dans des proportions comprises entre 0,02 et 1,5% en poids par rapport au poids total du composant (A).

Le composant (B) renfermant l'agent oxydant tel que défini ci-dessus, a un pH inférieur à 7. Ce pH peut avoir une valeur minimum de 1 et il est de préférence compris entre 1,5 et 3,5. Ce composant (B) peut être acidifié avec le même type d'agents acidifiants que ceux utilisés pour le composant (A).

Il peut se présenter sous forme de liquide plus ou moins épaissi, de lait ou de gel.

Cet agent de teinture à deux composants peut être conditionné dans un dispositif à plusieurs compartiments ou kit de teinture, ou tout autre système de conditionnement à plusieurs compartiments dont l'un renferme le composant (A) et le second compartiment renferme le composant (B); ces dispositifs pouvant être équipés d'un moyen permettant de délivrer sur les cheveux le mélange souhaité, tel que les dispositifs décrits dans le brevet US-A-4 823 985 de la demanderesse.

L'invention a également pour objet l'utilisation comme coupleurs d'aminoindoles répondant à la formule (I) pour la teinture en milieu acide des fibres kératiniques, en association avec des précurseurs de colorants d'oxydation.

Le procédé de teinture de l'invention consiste à appliquer sur les cheveux le mélange obtenu, à le laisser poser pendant 3 à 40 minutes, puis à rincer les cheveux et éventuellement effectuer un shampooing.

Il est également possible, conformément à l'invention, d'appliquer séparément une composition contenant le coupleur de formule (I), le précurseur de colorant d'oxydation et l'agent oxydant, de façon à ce que le mélange se formant in-situ au niveau des fibres ait un pH inférieur à 7, comme défini ci-dessus.

L'invention concerne également l'utilisation des composés de formule suivante : 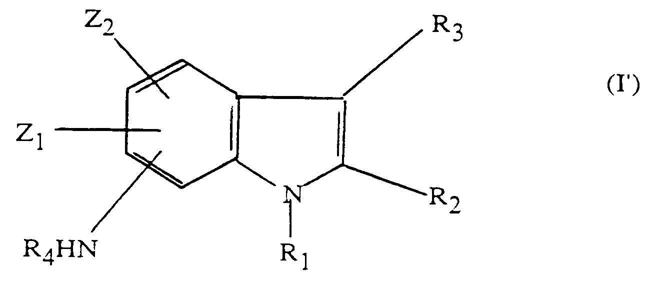
dans laquelle :
R₁, R₂, R₃, R₄, Z₁ et Z₂ ont les mêmes significations qu'indiquées ci-dessus, sous réserve que lorsque R₄HN est en position 4, 6 ou 7, Z₁ et Z₂ ne désignent pas simultanément un atome d'hydrogène, ainsi que leurs sels comme coupleurs pour la teinture par oxydation en association avec des précurseurs de colorants par oxydation.

Les compositions tinctoriales pour les fibres kératiniques, en particulier les cheveux contenant dans un milieu approprié pour la teinture un précurseur de colorant d'oxydation et au moins l'un de ces composés comme coupleur, constituent un autre objet de l'invention.

Ces compositions sont identiques à celles du composant (A) des agents de teinture selon l'invention, décrits précédemment.

Un autre objet de l'invention est constitué par les composés nouveaux choisis parmi :
(30) le 7-éthyl 4-aminoindole,
(31) le 7-éthyl 6-aminoindole,
ou de formule : 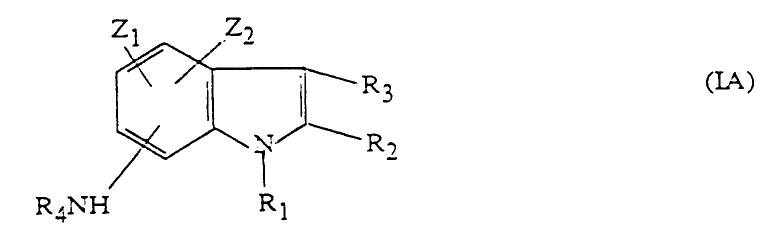
dans laquelle :
R₁ , R₂, R₃ ont les mêmes significations que celles indiquées ci-dessus;
au moins un des groupements R₂ et R₃ représente un atome d'hydrogène;
R₄ désigne un radical alkyle en C₁-C₄, hydroxyalkyle en C₁-C₄, polyhydroxyalkyle en C₂-C₄, -NHR₄ occupant les positions 4, 6 ou 7;
Z₁, Z₂ ayant la même signification que dans la formule (I') définie ci-dessus;
et les sels de ces composés. et les sels de ces composés.

Les composés (30), et (31), peuvent être préparés par réduction des dérivés nitrés correspondants. Certains de ces dérivés nitrés sont connus d'après l'article BERGMAN & SAND, Tetrahedron, Vol. 46, N° 17, pages 6085 à 6112, 1990. Les composés nitrés non décrits dans cet article peuvent être obtenus selon le même procédé, c'est-à-dire par action d'un trialkylorthoformiate sur une dialkyl ou trialkylmétanitroaniline (l'un des groupements alkyle étant situé en position ortho du groupement amino et para du groupement nitro), en présence d'acide p-toluènesulfonique, puis cyclisation du produit obtenu.

La réduction du groupement nitro s'effectue selon les procédés classiques de réduction, comme par exemple par réduction avec le fer dans l'acide acétique ou avec le zinc en présence d'alcool et de chlorure d'ammonium, ou bien par hydrogénation catalytique sous pression d'hydrogène en présence d'un catalyseur d'hydrogénation.

La réduction peut également se faire par transfert d'hydrogène avec un agent de transfert tel que le cyclohexène en présence d'un catalyseur d'hydrogénation.

Le catalyseur d'hydrogénation peut être constitué par exemple par du palladium ou du rhodium sur un support, tel que du charbon.

Dans le cas où Z₁ ou Z₂ = Cl, la réduction se fait de préférence avec le fer dans l'acide acétique ou avec le zinc en présence d'alcool et de chlorure d'ammonium.

Les composés de formule (IA) peuvent être préparés selon le procédé suivant.

Le composé (IA) peut être obtenu à partir du composé (IB) (R₄=H) par les méthodes de substitution des amines aromatiques, selon le schéma réactionnel : 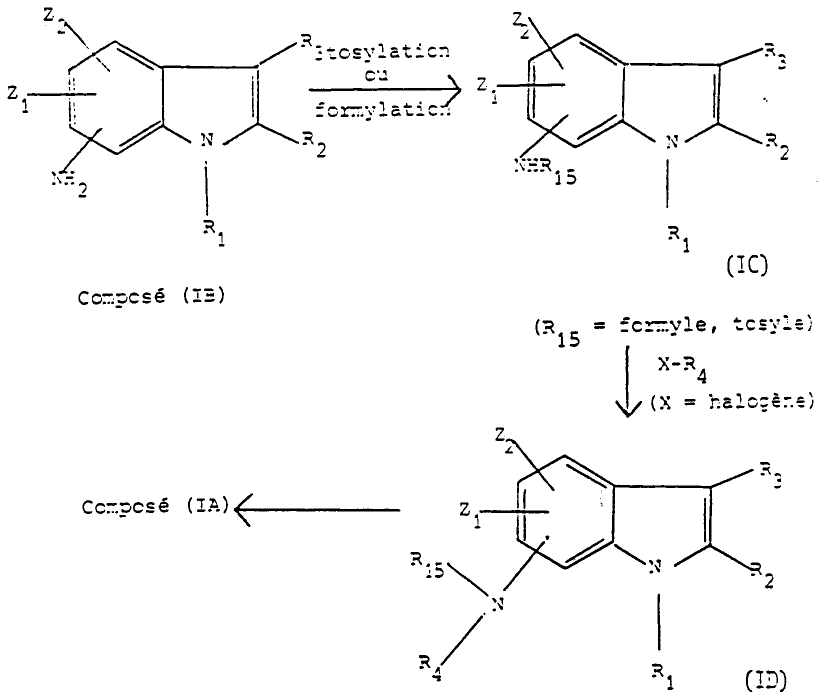
Par formylation ou tosylation, on obtient le composé (IC). Le composé (IC) est alkylé dans un deuxième temps par l'halogénure d'alkyle X-R₄ pour obtenir le composé (ID).

Le produit attendu (IA) est obtenu par déformylation ou détosylation du composé (ID), selon les méthodes conventionnelles.

Le composé (IA) peut également être obtenu, lorsque R₄ désigne un groupement alkyle, par réduction du composé (IE) par un hydrure métallique tel que le bromhydrure d'un métal alcalin en présence ou non d'un acide de Lewis tel que l'éthérate de trifluorure de bore. 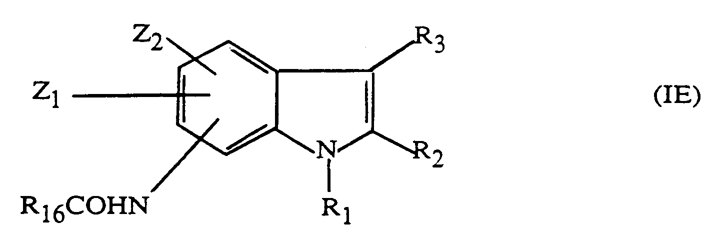
formule dans laquelle R₁₆ représente un groupement alkyle en C₁-C₃ ou un hydrogène.

Le composé (IE) étant obtenu à partir du composé (IB) selon les procédés connus d'obtention des groupements alkylcarbonylamino.

Parmi les méthodes d'hydroxyalkylation, on peut citer l'action du chloroformiate de β-chloralkyle sur le composé (IB) qui permet d'obtenir dans un premier temps le carbamate de β-chloroalkyle correspondant qui, soumis dans un deuxième temps à l'action d'une base minérale forte, permet d'obtenir le composé (IA) pour lequel le radical R₄ est un radical β-hydroxyalkyle.

Les exemples qui suivent sont destinés à illustrer la présente invention sans toutefois présenter un caractère limitatif.

### EXEMPLE DE PREPARATION 1

### SYNTHESE DU 6-AMINO 7-ETHYLINDOLE.

### A/PREPARATION DE LA 7-ETHYLINDOLINE

On prépare une solution de 145 g de 7-éthylindole dans 1,45 l de diméthoxyéthane. Sous azote, on ajoute 49,6 g de borohydrure de sodium puis on additionne sous agitation 854 g d'acide trichloroacétique en 1 heure, en maintenant la température réactionnelle à 20°C, à l'aide d'un bain à carboglace.

Après 2 heures d'agitation à la température ambiante, on verse sous agitation dans 2 litres de soude à 15% glacée. On ajoute 2 litres d'eau puis on extrait la phase aqueuse avec trois fois 0,4 l d'éther isopropylique. Les phases organiques sont lavées, séchées sur sulfate de sodium puis filtrées. Le filtrat est évaporé pour conduire à 150 g d'huile (rendement quantitatif).

### B/SYNTHESE DU 6-NITRO 7-ETHYLINDOLINE

a) On coule 147 a de 7-éthylindoline précédemment synthétisée dans 0,45 l d'acide sulfurique à 98% en maintenant la température à 10°C. On mélange 42 ml d'acide nitrique à 100% et 120 ml d'acide sulfurique à 98%. On ajoute le mélange sulfonitrique goutte à goutte en 1 heure en maintenant la température de la réaction en-dessous de 5°C. Après 1 heure à cette température, on verse sur 4 kg de glace. On neutralise à l'aide d'ammoniaque concentrée sous agitation, en contrôlant la température vers 10°C. On extrait avec trois fois 0,4 l d'acétate d'éthyle, les phases organiques sont lavées, séchées sur sulfate de sodium, puis évaporées.
b) L'huile obtenue est dissoute dans 0,3 l d'acide chlorhydrique 12N, il y a apparition d'un précipité, celui-ci est essoré. Ce solide est lavé à l'éthanol puis à l'éther de pétrole. On reprend ce solide dans 0,3 l d'eau glacée, puis on amène la solution à pH basique à l'aide d'ammoniaque concentrée, il y a formation d'un précipité jaune; celui-ci est essoré, lavé à l'eau puis séché sous vide.
c) On dissout le solide dans 0,6 l d'éther isopropylique, on ajoute 30 g de charbon végétal CECA L2S et 10 g de sulfate de soude. Après filtration, la phase organique est évaporée à sec. L'huile orange est versée dans 0,3 l d'éther de pétrole. Le précipité orange est essoré puis séché.

On obtient 82 g de 6-nitro 7-éthylindoline.

Point de fusion : 50°C
Rendement : 43%

### C/PREPARATION DU 6-AMINO 7-ETHYLINDOLE

On dissout 38,4 g de 6-nitro 7-éthylindoline dans 120 ml d'éthanol absolu auxquels on ajoute 19,2 g de palladium sur charbon à 10% (humide à 50%), puis on porte pendant 2 heures au rèflux. On filtre, on lave le catalyseur avec 100 ml d'éthanol, puis les phases organiques sont évaporées sous vide. L'extrait sec est repris dans 0,4 l d'éther isopropylique au reflux avec 3 g de charbon végétale. On filtre, sèche les phases organiques sur sulfate de sodium puis évapore à sec. Le solide est cristallisé dans 40 ml d'éther isopropylique. Les cristaux blancs sont essorés, séchés, pour obtenir :
25 g
Point de fusion : 108°C
Rendement : 78%

### EXEMPLE DE PREPARATION 2

### SYNTHESE DE LA 4-AMINO 7-ETHYLINDOLE

### A/SYNTHESE DE LA 4-NITRO 7-ETHYLINDOLINE

a) On coule 147 g de 7-éthylindoline précédemment synthétisée dans 0,45 l d'acide sulfurique à 98% en maintenant la température à 10°C. On mélange 42 ml d'acide nitrique à 100% et 120 ml d'acide sulfurique à 98%. On ajoute le mélange sulfonitrique goutte à goutte en 1 heure en maintenant la température de la réaction en-dessous de 5°C. Après 1 heure à cette température, on verse sur 4kg de glace. On neutralise à l'aide d'ammoniaque concentrée sous agitation, en contrôlant la température vers 10°C. On extrait avec trois fois 0,4 l d'acétate d'éthyle, les phases organiques sont lavées, séchées sur sulfate de sodium, puis évaporées.
b) L'huile obtenue est dissoute dans 0,3 l d'acide chlorhydrique 12N, il y a apparition d'un précipité, celui-ci est essoré. Ce solide est lavé à l'éthanol puis à l'éther de pétrole. On reprend ce solide dans 0,3 l d'eau glacée puis on amène la solution à pH basique à l'aide d'ammoniaque concentrée, il y a formation d'un précipité jaune; celui-ci est essoré, lavé à l'eau puis séché sous vide.
c) La phase chlorhydrique obtenue en b) est diluée par 0,5 kg de glace puis amenée à pH basique à l'aide d'ammoniaque concentrée. Le précipité obtenu est essoré, lavé à l'eau, lavé avec deux fois 60 ml d'éther isopropylique et enfin séché.

Rendement : 15%
Point de fusion : 68°C

### B/PREPARATION DU 4-AMIMO 7-ETHYLINDOLE

La 4-nitro 7-éthylindoline est réduite de la même manière que la 6-nitro 7-éthylindoline. On obtient 6 g d'indole recristallisé d'un mélange d'acétate d'éthyle/éther isopropylique (1/3).

Point de fusion : 123°C
Rendement : 75%

### EXEMPLE DE PREPARATION 3

### PREPARATION DU 6(BETACHLOROETHYLURETHAN)-7-ETHYLINDOLE

On coule 8 g de betachloroformiate dans une suspension de 8 g d' 6-amino 7-éthylindole, 5,5 g de carbonate de calcium dans 24 ml de dioxane au reflux en 30 minutes. On verse sur de la glace puis on acidifie le milieu. On essore le précipité, lave à l'eau puis on le sèche. On obtient 13 g de composé blanc.

Point de fusion : 132°C
Rendement : 98%

### EXEMPLE DE PREPARATION 4

### PREPARATION DU 3-(7-ETHYL-1H-INDOL-6-YL)OXAZOLIDIN -2-ONE

On porte au reflux un mélange de 8 g de l'urethane précédent, 8 ml d'éthanol à 96° et de 24 ml de soude 4N pendant 15 minutes. On verse sur glace, on essore le précipité, on lave à l'eau, à l'alcool puis à l'éther de pétrole. Après séchage, on obtient 6,6 g.

Point de fusion : 248°C
Rendement : 96%

### EXEMPLE DE PREPARATION 5

### PREPARATION DU 7-ETHYL 6-N,β-HYDROXYETHYLAMINOINDOLE

On ajoute 12,2 g de l'oxazolidone précédente à 50°C à un mélange de 31,5 g d'hydroxyde de potassium, 8 ml d'eau, 70 ml d'éthanol. Après 20 minutes au reflux, on verse sur 400 g de glace, on amène à pH 5 avec de l'acide acétique. On ajoute de l'ammoniaque jusqu'à pH 7,5-8. On obtient un précipité blanc. Celui-ci est lavé à l'eau et séché. L'extraction des eaux-mères donne 1,6 g supplémentaire. Le tout est cristallisé dans un mélange acétate d'éthyle/éther isopropylique (1/3). On obtient 8,9 g.

Point de fusion : 105°C
Rendement : 82%

### EXEMPLES 1 à 17

On procède à la teinture des cheveux en appliquant sur de cheveux permanentés gris à 90% de blancs, un mélange extemporané de la composition colorante (A) et de la composition oxydante (B).

Ce mélange présente le pH indiqué dans le tableau des exemples qui suivent.

On laisse agir ce mélange pendant 30 minutes, puis on rince les cheveux, on effectue un shampooing. Après séchage, les cheveux sont teints dans la nuance précisée au bas des tableaux ci-après.

| en g | 1 | 2 | 3 | 4 | 5 |
|---|---|---|---|---|---|
| **A) *Composition colorante*** | | | | | |
| 4-aminoindole | | | | | 0,396 |
| 5-aminoindole | | 0,396 | | | |
| 2-méthyl 6-aminoindole | 0,438 | | 0,438 | 0,438 | |
| 2,6-diméthylparaphénylènediamine,2HCl | 0,627 | | | | 0,657 |
| Paraphénylènediamine | | 0,324 | 0,324 | | |
| Paraaminophénol | | | | 0,327 | |
| Monoéthanolamine qs pH | 8,90 | 9,20 | 9,20 | 9,20 | 8,90 |
| Support | X | X | X | X | X |
| Eau qsp | 100 | 100 | 100 | 100 | 100 |

| **B) *Composition oxydante*** | | | | | |
|---|---|---|---|---|---|
| Solution d'eau oxygénée à 20 volumes | 100 | 100 | 100 | 100 | 100 |
| Acide phosphorique | 2,50 | 2,50 | 2,50 | 2,50 | 2,50 |
| pH mélange p/p A + B | 6,50 | 6,60 | 6,50 | 6,50 | 6,50 |
| ***Nuances obtenues :*** | cuivré acajou | châtain | châtain clair cuivré acajou | blond clair cuivré intense | bleu canard cendré puissant |

| en g | 6 | 7 | 8 | 9 | 10 |
|---|---|---|---|---|---|
| ***A) Composition colorante*** | | | | | |
| 2,3,7-triméthyl 6-aminoindole | 0,522 | | | | |
| 2-méthyl 5-hydroxy 6-aminoindole | | | 0,486 | | |
| 5-aminoindole | | 0,396 | | 0,396 | |
| 6-β-hydroxyéthylaminoindole | | | | | 0,528 |
| 2,6-diméthylparaphénylènediamine,2HCl | 0,627 | | | 0,627 | |
| Paraphénylènediamine | | | | | 0,324 |
| Paraaminophénol | | 0,327 | 0,327 | | |
| Monoéthanolamine qs pH | 8,7 | 9,2 | 9,2 | 8,9 | 9,3 |
| Support | X | X | X | X | X |
| Eau qsp | 100 | 100 | 100 | 100 | 100 |

| **B) *Composition oxydante*** | | | | | |
|---|---|---|---|---|---|
| Solution d'eau oxygénée à | 100 | 100 | 100 | 100 | 100 |
| 20 volumes Acide phosphorique | 2,50 | 2,50 | 2,50 | 2,50 | 2,50 |
| pH mélange p/p A + B | 6 | 6,7 | 6,5 | 6,4 | 6,8 |
| ***Nuances obtenues :*** | vert gris assez profond | irisé cuivré | beige cuivré | châtain naturel | châtain cuivre acajou |

| en g | 11 | 12 | 13 | 14 | 15 |
|---|---|---|---|---|---|
| ***A) Composition colorante*** | | | | | |
| 2,3-diméthyl 5-méthoxy 6-aminoindole | 0,571 | | | | |
| 2,3-diméthyl 5-chloro 6-aminoindole | | 0,583 | | | |
| 2,3-diméthyl 5-amino 6-méthoxyindole, HCl | | | 0,680 | | |
| 2,3-diméthyl 5-acétylamino 6-méthoxyindole | | | | 0,697 | |
| 2,3-diméthyl 5-amino 6-hydroxyindole | | | | | 0,529 |
| 2,6-diméthylparaphénylènediamine, 2HCl | | | 0,627 | | 0,627 |
| Paraaminophénol | 0,327 | 0,327 | | | |
| Paraphénylènediamine | | | | 0,324 | |
| Monoéthanolamine qs pH | 9,1 | 9,1 | 8,8 | 9,2 | 8,9 |
| Support | X | X | X | X | X |
| Eau qsp | 100 | 100 | 100 | 100 | 100 |

| **B) *Composition oxydante*** | | | | | |
|---|---|---|---|---|---|
| Solution d'eau oxygénée à 20 volumes | 100 | 100 | 100 | 100 | 100 |
| Acide phosphorique | 2,5 | 2,5 | 2,5 | 2,5 | 2,5 |
| pH mélange p/p A+ B | 6,7 | 6,3 | 6,4 | 6,6 | 6,4 |
| ***Nuances obtenues :*** | beige nacré | blond doré nacré | châtain clair marron irisé | blond foncé mat | gris moyen |

| Exemples en g | 16 | 17 |
|---|---|---|
| ***A) Composition colorante*** | | |
| 7-éthyl 6-aminoindole | 0,48 | - |
| 7-éthyl 6-N,β-hydroxyréthylaminoindole | - | 0,61 |
| Dichlorhydrate de 2,6-diméthylparaphénylènediamine | 0,63 | 0,63 |
| Monoéthanolamine qs pH | 9,8 | 9,8 |
| Support | X | X |
| Eau qsp | 100 | 100 |

| ***B) Composition oxydante*** | | |
|---|---|---|
| Solution d'eau oxygenée à 20 volumes | 100 | 100 |
| Acide phosphorique | 2,5 | 2,5 |
| pH mélange p/p A + B | 6,7 | 6,6 |
| ***Nuances obtenues*** : | châtain irisé acajou rouge | châtain irisé acajou rouge |

### SUPPORT DE COLORATION utilisé dans les exemples 1 à 17.

| | |
|---|---|
| - Alcool oléique polyglycérolé à 2 moles de glycérol | 4,0 g |
| - Alcool oléique polyglycérolé à 4 moles de glycérol à 78% de MA | 5,69 g MA |
| - Acide oléique | 3,0 g |
| - Amine oléique à 2 moles d'oxyde d'éthylène vendue sous la dénomination ETHOMEEN O 12 par la Société AKZO | 7,0 g |
| - Laurylamino succinamate de diéthylaminopropyle, sel de sodium à 55% de MA | 3,0 g MA |
| - Alcool oléique | 5,0 g |
| - Diéthanolamide d'acide oléique | 12,0 g |
| - Propylèneglycol | 3,5 g |
| - Alcool éthylique | 7,0 g |
| - Dipropylèneglycol | 0,5 g |
| - Monométhyléther de propylèneglycol | 9,0 g |
| - Métabisulfite de sodium en solution | |
| aqueuse à 35% de MA | 0,45 g MA |
| - Acétate d'ammonium | 0,8 g |
| - Antioxydant, séquestrant qs | |
| - Parfum, conservateurs qs | |

## Revendications (Revendications pour l'(les) Etat(s) contractant(s) suivant(s): AT, BE, CH, DE, DK, FR, GB, GR, IT, NL, SE)

1. Procédé de teinture des fibres kératiniques, en particulier des fibres kératiniques humaines telles que les cheveux, caractérisé par le fait qu'on applique sur ces fibres une composition contenant dans un milieu approprié pour la teinture, au moins un coupleur répondant à la formule : 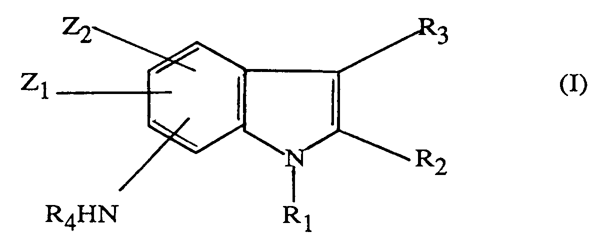 dans laquelle :
R₁ désigne hydrogène ou un groupement alkyle en C₁-C₄;
R₂ et R₃, indépendamment l'un de l'autre, représentent un atome d'hydrogène, un groupement alkyle en C₁-C₄ ou un groupement COOR', R' étant un radical alkyle en C₁-C₄ ou un atome d'hydrogène;
R₄ représente un atome d'hydrogène, un radical alkyle en C₁-C₄, un groupement hydroxyalkyle en C₁-C₄, polyhydroxyalkyle en C₂-C₄, acétyle ou aminoalkyle en C₁-C₆ dont l'amine peut être éventuellement mono- ou disubstituée par un alkyle en C₁-C₄;
Z₁ et Z₂, identiques ou différents, représentent un atome d'hydrogène ou un groupement alkyle en C₁-C₄, un hydroxy, un halogène, alcoxy(C₁-C₄);
et les sels de ces composés;
- au moins un précurseur de colorant d'oxydation;
- au moins un agent oxydant; le pH de la composition appliquée sur les fibres étant inférieur à 7.

2. Procédé selon la revendication 1, caractérisé par le fait que le composé de formule (I) est choisi parmi :
(1) le 5-aminoindole,
(2) le 4-aminoindole,
(3) le 2-méthyl 6-aminoindole,
(4) le 2,3,4-triméthyl 6-aminoindole,
(5) le 2,3,7-triméthyl 6-aminoindole,
(6) le 2,3,5-triméthyl 6-aminoindole,
(7) le 6-aminoindole,
(8) le 7-aminoindole,
(9) le 6-(β-hydroxyéthylamino)indole,
(10) le 2-méthyl 5-hydroxy 6-aminoindole,
(11) le 2,3-diméthyl 5-amino 6-hydroxyindole,
(12) le 2,3-diméthyl 5-méthoxy 6-aminoindole,
(13) le 2,3-diméthyl 5-hydroxy 6-aminoindole,
(14) le 2,3-diméthyl 5-éthyl 6-aminoindole,
(15) le 2,3-diméthyl 5-chloro 6-aminoindole,
(16) le 2,3-diméthyl 5-acétylamino 6-méthoxyindole,
(17) le 2,3-diméthyl 5-amino 6-méthoxyindole,
(18) le N-méthyl 6-(β-hydroxyéthyl)aminoindole,
(19) le 2,3,4,5-tétraméthyl 6-aminoindole.
(20) le 6-N-méthylaminoindole,
(21) le N-méthyl 5-aminoindole,
(22) le N-méthyl 6-aminoindole,
(23) le N-méthyl 4-aminoindole,
(24) le 2,3-diméthyl 4-aminoindole,
(25) le 2,3-diméthyl 7-aminoindole,
(26) le 3-méthyl 6-aminoindole,
(27) le 2-méthyl 3-éthyl 6-aminoindole,
(28) le 2-méthyl 3-éthyl 7-aminoindole,
(29) le 4-N-méthylaminoindole,
(30) le 7-éthyl 6-aminoindole,
(31) le 7-éthyl 4-aminoindole,
(32) le 7-éthyl 6-N,β-hyroxyéthylaminoindole,
(33) le 4-méthyl 6-aminoindole,
(34) le 5-méthyl 6-aminoindole,
(35) le 7-méthyl 4-aminoindole,
(36) le 3-méthyl 7-éthyl 6-aminoindole,
(37) le 5,7-diméthyl 6-aminoindole,
(38) le 5,7-diéthyl 6-aminoindole,
(39) le 2-éthoxycarbonyl 5-méthyl 7-aminoindole,
(40) le 2-éthoxycarbonyl 5-chloro 7-aminoindole,
(41) le 2-éthoxycarbonyl 5-éthoxy 7-aminoindole,
(42) le 2-éthoxycarbonyl 5-méthoxy 7-aminoindole,
(43) le 5-méthoxy 7-(4'-diméthylamino 1'-méthylbutyl)aminoindole,
(44) le 5-méthoxy 7-(4'-diméthylaminobutyl)aminoindole,
(45) le 5-méthoxy 7-(4'-diéthylamino 1'-méthylbutyl)aminoindole,
(46) le 5-fluoro 6-aminoindole,
(47) le 5-fluoro 1-sec-butyl 6-aminoindole,
(48) le 5-fluoro 1-n-propyl 6-aminoindole,
(49) le 1-méthyl 2-méthoxycarbonyl 5-méthoxy 6-aminoindole,
(50) le 2-méthoxycarbonyl 5-méthoxy 6-aminoindole,
(51) le 2-éthoxycarbonyl 5-méthoxy 6-aminoindole,
(52) le 2-carboxy 5-méthoxy 6-aminoindole,
(53) le 1,2-diméthyl 5-hydroxy 6-aminoindole,
(54) le 2-méthoxycarbonyl 4-méthoxy 6-aminoindole,
et les sels de ces composes.

3. Procédé selon la revendication 1 ou 2, caractérisé par le fait que les précurseurs de colorants d'oxydation sont choisis parmi les paraphénylènediamines, les para-aminophénols, les précurseurs hétérocycliques para, les bases doubles.

4. Procédé selon la revendication 3, caractérisé par le fait que les paraphénylènediamines sont choisies parmi les composés répondant à la formule : 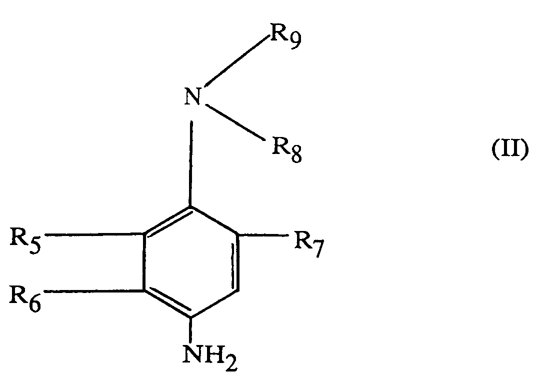 dans laquelle :
R₅, R₆ et R₇, identiques ou différents, représentent un atome d'hydrogène ou d'halogène, un radical alkyle ayant de 1 à 4 atomes de carbone, un radical alcoxy ayant de 1 à 4 atomes de carbone, un radical carboxy, sulfo, hydroxyalkylke ayant de 1 à 4 atomes de carbone;
R₈ et R₉, identiques ou différents, représentent un atome d'hydrogène, un radical alkyle, hydroxyalkyle, alcoxyalkyle, carbamylalkyle, mésylaminoalkyle, acétylaminoalkyle, uréidoalkyle, carbalcoxyaminoalkyle, pipéridinoalkyle, morpholinoalkyle; ces groupes alkyle ou alcoxy ayant de 1 à 4 atomes de carbone, ou bien R₈ et R₉ forment, conjointement avec l'atome d'azote auquel ils sont liés, un hétérocycle pipéridino ou morpholino, sous réserve que R₅ ou R₇ représente un atome d'hydrogène lorsque R₈ et R₉ ne représentent pas un atome d'hydrogène, ainsi que les sels de ces composés.

5. Procédé selon la revendication 4, caractérisé par le fait que les composés de formule (II) sont choisis parmi la p-phénylènediamine, la 2-méthyl-p- phénylènediamine, la méthoxyparaphénylènediamine, la chloropara- phénylènediamine, la 2,6-diméthylparaphénylènediamine, la 2,5- diméthylparaphénylènediamine, la 2,3-diméthylparaphénylènediamine, la 2-méthyl 5-méthoxypara-phénylènediamine, la 2,6-diméthyl 5-méthoxyparaphénylènediamine, la N,N-diméthylparaphénylène diamine, la 3-méthyl 4-amino N,N-diéthylaniline, la N,N-di-(β-hydroxyéthyl)paraphénylènediamine, la 3-méthyl 4-amino N,N-di-(β-hydroxyéthyl)aniline, la 3-chloro 4-amino N,N-di-(β-hydroxyéthyl)aniline, la 4-amino N,N-(éthyl,carbamylméthyl)aniline, la 3-méthyl 4-amino N,N-(éthyl,carbamylméthyl)aniline, la 4-amino N,N-(éthyl,β-pipéridinoéthyl)aniline, la 3-méthyl 4-amino N,N-(éthyl,β-pipéridinoéthyl) aniline, la 4-amino N,N-(éthyl,β-morpholinoéthyl)aniline, la 3-méthyl 4-amino N,N-(éthyl,β-morpholinoéthyl)aniline, la 4-amino N,N-(éthyl, β-acétylaminoéthyl)aniline, la 4-amino N-(β-méthoxyéthyl)aniline, la 3-méthyl 4-amino N,N-(éthyl,β-acétylaminoéthyl)aniline, la 4-amino N,N-(éthyl,β-mésylaminoéthyl)aniline, la 3-méthyl 4-amino N,N- (éthyl,β-mésylaminoéthyl)aniline, la 4-amino N,N-(éthyl,β-sulfoéthyl) aniline, la 3-méthyl 4-amino N,N-(éthyl,β-sulfoéthyl)aniline, la N-[(4'-amino)phényl]-morpholine, la N-[(4'-amino)phényl]pipéridine, la 2-hydroxyéthylparaphénylènediamine, la fluoroparaphénylènediamine, la carboxyparaphénylènediamine, la sulfoparaphénylènediamine, la 2-isopropylparaphénylènediamine, la 2-n-propylparaphénylènediamine, la 2-hydroxyméthylparaphénylènediamine, la N,N-diméthyl 3-méthyl paraphénylènediamine, la N,N(éthyl,β-hydroxyéthyl)paraphénylène diamine, la N-(dihydroxypropyl)paraphénylènediamine, la N-4'-aminophényl p-phénylènediamine, la N-phényl p-phénylènediamine, sous forme de base libre ou de sels.

6. Procédé selon la revendication 3, caractérisé par le fait que les les p-aminophénols, sont choisis parmi le p-aminophénol, le 2-méthyl 4-aminophénol, le 3-méthyl 4-aminophénol, le 2-chloro 4-aminophénol, le 3-chloro 4-aminophénol, le 2,6-diméthyl 4-aminophénol, le 3,5-diméthyl 4-aminophénol, le 2,3-diméthyl 4-amino phénol, le 2-hydroxyméthyl 4-aminophénol, le 2-(β-hydroxyéthyl) 4-aminophénol, le 2-méthoxy 4-aminophénol, le 3-méthoxy 4-amino phénol, le 2,5-diméthyl 4-aminophénol, le 2-méthoxyméthyl 4-amino phénol, le 2-β-hydroxyéthoxyméthyl 4-aminophénol.

7. Procédé selon la revendication 3, caractérisé par le fait que les bases dites doubles sont choisies parmi les bis-phényl alkylène diamines de formule : 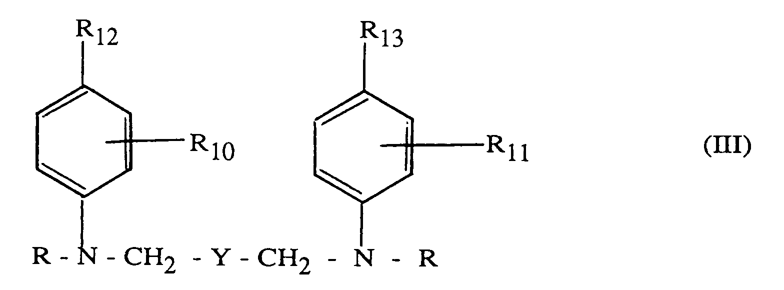 dans laquelle :
R₁₂ et R₁₃, identiques ou différents, représentent des groupements hydroxyle ou NHR₁₄, où R₁₄ désigne un atome d'hydrogène ou un radical alkyle inférieur;
R₁₀ et R₁₁, identiques ou différents, représentent soit des atomes d'hydrogène, soit des atomes d'halogène, soit des groupements alkyle;
R représente un atome d'hydrogène, un groupe alkyle, hydroxyalkyle ou aminoalkyle, dont le reste amino peut être substitué;
Y représente un radical pris dans le groupe constitué par les radicaux suivants : -(CH₂)ₙ-, (CH₂)_{n'}-O-(CH₂)_{n'}-, (CH₂)_{n'}-CHOH-(CH₂)ₙ, ;-(CH₂)ₙ, 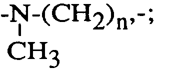 n est un nombre entier compris entre 0 et 8 et n' un nombre entier entre 0 et 4, ainsi que leurs sels d'addition d'acides.

8. Procédé selon la revendication 7, caractérisé par le fait que la base dite double est choisie parmi les composés suivants : le N,N'bis-(β-hydroxyéthyl)N,N'-bis(4'-aminophényl)1,3-diamino 2-propanol, la N,N'bis-(β-hydroxyéthyl)N,N'-bis(4'-aminophényl)éthylènediamine, la N,N'bis-(4-aminophényl)tétraméthylènediamine, la N,N'bis-(β-hydroxy éthyl)N,N'bis(4-aminophényl)tétraméthylènediamine, la N,N'bis-(4-méthylaminophényl)tétraméthylènediamine, la N,N'bis(éthyl)N,N'-bis (4'-amino 3'-méthylphényl)éthylènediamine.

9. Procédé selon la revendication 1 ou 2, caractérisé par le fait que les précurseurs de colorants d'oxydation sont des précurseurs de colorants d'oxydation de type ortho choisis parmi les orthoaminophénols et les orthophénylènediamines.

10. Procédé selon l'une quelconque des revendications 1 à 9, caractérisé par le fait que l'agent oxydant est choisi parmi le peroxyde d'hydrogène, le peroxyde durée, les bromates de métaux alcalins, les persels.

11. Procédé selon l'une quelconque des revendications 1 à 10, caractérisé par le fait que le pH de la composition appliquée sur les fibres kératiniques est compris entre 3 et 6,9.

12. Procédé selon l'une quelconque des revendications 1 à 11, caractérisé par le fait que la composition utilisée pour la teinture des fibres kératiniques contient en plus des coupleurs hétérocyliques de la famille des aminoindoles de formule (I), d'autres coupleurs choisis parmi les métadiphénols, les métaaminophénols, les métaphénylènediamines, les méta N-acylaminophénols, les métauréidophénols, les métacarbalcoxyaminophénols, l'α-naphtol, les coupleurs possédant un groupement méthylène actif choisis parmi les composés dicétoniques et les pyrazolones, les coupleurs hétérocycliques ou le 4-hydroxyindole, le 6-hydroxyindole ou le 7-hydroxyindole.

13. Procédé selon la revendication 12, caractérisé par le fait que les coupleurs sont choisis parmi le 2,4-dihydroxyphénoxyéthanol, le 2,4-dihydroxyanisole, le métaaminophénol, la résorcine, le monométhyléther de résorcine, la 2-méthylrésorcine, le pyrocatéchol, le 2-méthyl 5-N-(β-hydroxyéthyl)aminophénol, le 2-méthyl 5-N-(β-mésylaminoéthyl)aminophénol, la 6-hydroxybenzomorpholine, le 2,4-diaminoanisole, le 2,4-diaminophénoxyéthanol, la 6-aminobenzomorpholine, le [2-N-(β-hydroxyéthyl)amino 4-amino]-phénoxyéthanol, le 2-amino 4-N-(β-hydroxyéthyl)aminoanisole, le (2,4-diamino)phényl-β,γ-dihydroxypropyléther, la 2,4-diaminophénoxyéthylamine, le 1,3-diméthoxy 2,4-diaminobenzène, le 2-méthyl 5-aminophénol, le 2,6-diméthyl 3-aminophénol, le 3,4-méthylènedioxyphénol, la 3,4-méthylènedioxyaniline, la 2-chlororésorcine et leurs sels.

14. Procédé selon l'une quelconque des revendications 1 à 13, caractérisé par le fait que la composition contient des agents tensioactifs anioniques, cationiques, non ioniques, amphotères ou leurs mélanges; des agents épaississants, des agents anti-oxydants et/ou tout autre adjuvant cosmétiquement acceptable.

15. Procédé selon l'une quelconque des revendications 1 à 14, caractérisé par le fait que le milieu approprié pour la teinture est constitué par de l'eau ou par un mélange d'eau et d'un solvant choisi parmi les alcanols inférieurs en C₂-C₄, le glycérol, les glycols, les éthers de glycols, les alcools aromatiques ou leurs mélanges.

16. Agent de teinture des fibres kératiniques et en particulier des cheveux, caractérisé par le fait qu'il comporte au moins deux composants : un composant (A) constitué par une composition contenant dans un milieu approprié pour la teinture, un coupleur amino indolique répondant à la formule (I) tel que défini dans la revendication 1 ou 2 et un précurseur de colorant d'oxydation tel que défini dans l'une quelconque des revendications 1 et 3 à 9, un composant (B) constitué par une composition contenant dans un milieu approprié pour la teinture, un agent oxydant, le pli des composants (A) et (B) étant tel qu'après mélange dans des proportions de 90 à 10% pour le composant (A) et de 10 à 90% pour le composant (B), la composition résultante ait un pH inférieur à 7.

17. Agent selon la revendication 16, caractérisé par le fait que le composant (A) a un pH compris entre 3 et 10,5.

18. Agent selon la revendication 16 ou 17, caractérisé par le fait que le composant (A) contient les précurseurs de colorants par oxydation ainsi que les coupleurs des des proportions comprises entre 0,05 et 7% en poids par rapport au poids total du composant (A).

19. Agent selon l'une quelconque des revendications 16 à 18, caractérisé par le fait que la concentration en composé de formule (I) est comprise entre 0,012 et 4% en poids par rapport au poids total du composant (A).

20. Agent selon l'une quelconque des revendications 16 à 19, caractérisé par le fait que le composant (A) contient des agents tensioactifs dans des proportions de 0,1 à 55% en poids, des solvants en plus de l'eau dans des proportions comprises entre 0,5 et 40% en poids, des agents épaississants dans des proportions comprises entre 0,1 et 5% en poids, des agents antioxydants dans des proportions comprises entre 0,02 et 1,5% en poids, et/ou tout autre adjuvant cosmétiquement acceptable.

21. Agent selon l'une quelconque des revendications 16 à 20, caractérisé par le fait que le composant (B) a un pH qui a une valeur minimum de 1 et inférieure à 7.

22. Procédé de teinture des fibres kératiniques et en particulier des cheveux, caractérisé par le fait qu'il comporte une première étape consistant à stocker un agent de teinture tel que défini dans l'une quelconque des revendications 16 à 21 et à procéder avant application au mélange des composants (A) et (B) dans des proportions de 10 à 90% pour le composant (A) et de 90 à 10% pour le composant (B), de façon à obtenir une composition ayant un pH inférieur à 7 et d'appliquer ce mélange immédiatement après préparation sur les fibres kératiniques.

23. Dispositif à plusieurs compartiments ou kit de teinture, caractérisé par le fait qu'il comporte au moins deux compartiments dont un premier compartiment renferme le composant (A) tel que déini dans l'une quelconque des revendications 16 à 20, et le second compartiment renferme le composant (B) tel que défini dans les revendications 16 et 21.

24. Dispositif selon la revendication 23, caractérisé par le fait qu'il est équipé d'un moyen permettant de délivrer sur les cheveux le mélange souhaité des composants (A) et (B).

25. Procédé de teinture selon l'une quelconque des revendications 1 à 15, caractérisé par le fait que l'on applique sur les cheveux la composition et qu'on la laisse poser pendant 3 à 40 minutes, que l'on rince les cheveux et qu'on précède éventuellement à un shampooing avant un nouveau rinçage et séchage.

26. Utilisation comme coupleurs des composés amino indoliques répondant à la formule (I) tels que définis dans la revendication 1 à 2 pour la teinture en milieu acide des fibres kératiniques, en association avec des précurseurs de colorants d'oxydation.

27. Composition de teinture de fibres kératiniques, prête à l'emploi telle que mise en oeuvre dans le procédé selon l'une quelconque des revendications 1 à 15.

28. Composition selon la revendication 27, contenant le composé de formule (I) dans des proportions de 0,01 à 3,5% en poids par rapport au poids total de la composition.

29. Composition tinctoriale pour les fibres kératiniques, en particulier des cheveux, caractérisé par le fait qu'elle contient dans un milieu approprié pour la teinture au moins un précurseur de colorant d'oxydation et un coupleur répondant à la formule suivante : 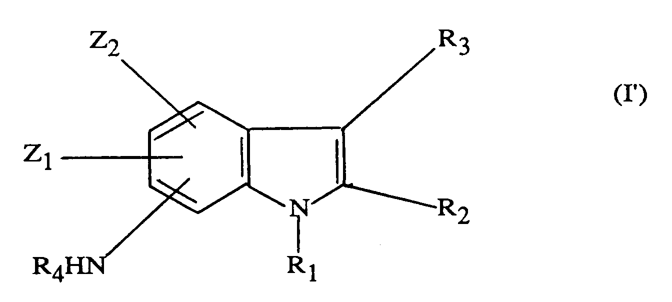 dans laquelle :
R₁ désigne hydrogène ou un groupement alkyle en C₁-C₄;
R₂ et R₃, indépendamment l'un de l'autre, représentent un atome d'hydrogène, un groupement alkyle en C₁-C₄ ou un groupement COOR', R' étant un radical alkyle en C₁-C₄;
dans laquelle :
R₄ représente un atome d'hydrogène, un radical alkyle en C₁-C₄, un groupe hydroxyalkyle en C₁-C₄, polyhydroxyalkyle en C₂-C₄, acétyle ou aminoalkyle en C₁-C₆ dont l'amine peut être éventuellement mono- ou disubstituée par un alkyle en C₁-C₄;
Z₁ et Z₂, identiques ou différents, représentent un atome d'hydrogène, un groupe alkyle en C₁-C₄, hydroxy, un halogène, un alcoxy en C₁-C₄;
sous réserve que lorsque NHR₄ est en position 4, 6 ou 7, Z₁ et Z₂ ne désignent pas simultanément un atome d'hydrogène; ainsi que les sels de ces composés, à l'exception du 6-hydroxy 5-acétylamino 2,3-diméthylindole.

30. Agent de teinture selon l'une quelconque des revendications 16 à 20, caractérisé par le fait que le composant (A) contient comme coupleur un aminoindole répondant à la formule suivante : 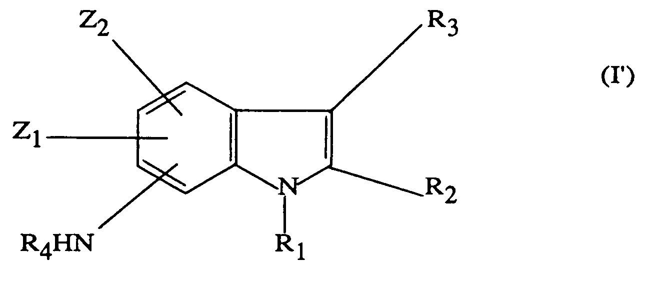 dans laquelle :
R₁ désigne hydrogène ou un groupement alkyle en C₁-C₄;
R₂ et R₃, indépendamment l'un de l'autre, représentent un atome d'hydrogène, un groupe alkyle en C₁-C₄ ou un groupement COOR' où R' désigne un atome d'hydrogène ou un alkyle en C₁-C₄; au moins un des radicaux R₂ et R₃ désigne un atome d'hydrogène;
R₄ représente un groupement alkyle en C₁-C₄, hydroxyalkyle en C₁-C₄ ou polyhydroxyalkyle en C₂-C₄; le groupement -NHR₄ occupant les positions 4, 6 ou 7;
Z₁ et Z₂, identiques ou différents, représentent un atome d'hydrogène, un alkyle en C₁-C₄, un hydroxy, un atome d'halogène ou un alcoxy en C₁-C₄;
sous réserve que lorsque NHR₄ est en position 4, 6 ou 7, Z₁ et Z₂ ne désignent pas simultanément un atome d'hydrogène; ainsi que les sels de ces composés.

31. Composé répondant à la formule suivante : 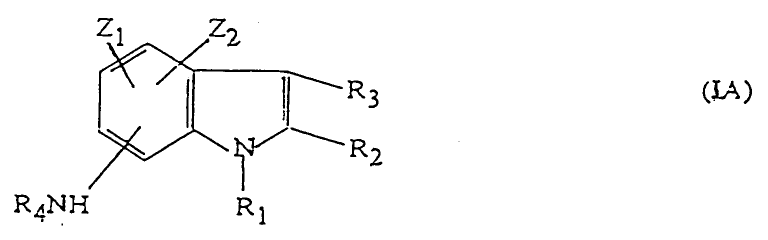 dans laquelle :
R₁ désigne hydrogène ou un groupement alkyle en C₁-C₄;
R₂ et R₃, indépendamment l'un de l'autre, représentent un atome d'hydrogène, un groupe alkyle en C₁-C₄ ou un groupement COOR' où R' désigne un atome d'hydrogène ou un alkyle en C₁-C₄; au moins un des radicaux R₂ et R₃ désigne un atome d'hydrogène;
R₄ représente un groupement alkyle en C₁-C₄, hydroxyalkyle en C₁-C₄ ou polyhydroxyalkyle en C₂-C₄; le groupement -NHR₄ occupant les positions 4, 6 ou 7;
Z₁ et Z₂, identiques ou différents, représentent un atome d'hydrogène, un alkyle en C₁-C₄, un hydroxy, un atome d'halogène ou un alcoxy en C₁-C₄; au moins un des groupements Z₁ et Z₂ est différent d'hydrogène;
ainsi que les sels de ces composés.

32. Composé choisi parmi :
le 7-éthyl 4-aminoindole,
le 7-éthyl 6-aminoindole,
ainsi que les sels de ces composés.

## Revendications (Revendications pour l'(les) Etat(s) contractant(s) suivant(s): ES)

1. Procédé de teinture des fibres kératiniques, en particulier des fibres kératiniques humaines telles que les cheveux, caractérisé par le fait qu'on applique sur ces fibres une composition contenant dans un milieu approprié pour la teinture, au moins un coupleur répondant à la formule : 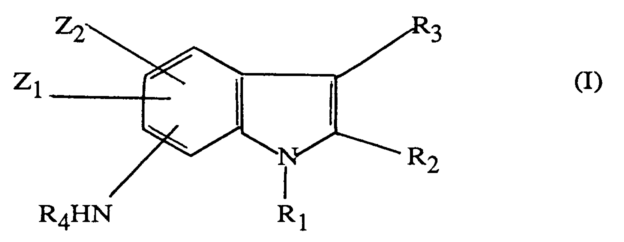 dans laquelle :
R₁ désigne hydrogène ou un groupement alkyle en C₁-C₄;
R₂ et R₃, indépendamment l'un de l'autre, représentent un atome d'hydrogène, un groupement alkyle en C₁-C₄ ou un groupement COOR', R' étant un radical alkyle en C₁-C₄ ou un atome d'hydrogène;
R₄ représente un atome d'hydrogène, un radical alkyle en C₁-C₄, un groupement hydroxyalkyle en C₁-C₄, polyhydroxyalkyle en C₂-C₄, acétyle ou aminoalkyle en C₁-C₆ dont l'amine peut être éventuellement mono- ou disubstituée par un alkyle en C₁-C₄;
Z₁ et Z₂, identiques ou différents, représentent un atome d'hydrogène ou un groupement alkyle en C₁-C₄, un hydroxy, un halogène, alcoxy(C₁-C₄);
et les sels de ces composés;
- au moins un précurseur de colorant d'oxydation;
- au moins un agent oxydant; le pH de la composition appliquée sur les fibres étant inférieur à 7.

2. Procédé selon la revendication 1, caractérisé par le fait que le composé de formule (I) est choisi parmi :
(1) le 5-aminoindole,
(2) le 4-aminoindole,
(3) le 2-méthyl 6-aminoindole,
(4) le 2,3,4-triméthyl 6-aminoindole,
(5) le 2,3,7-triméthyl 6-aminoindole,
(6) le 2,3,5-triméthyl 6-aminoindole,
(7) le 6-aminoindole,
(8) le 7-aminoindole,
(9) le 6-(β-hydroxyéthylamino)indole,
(10) le 2-méthyl 5-hydroxy 6-aminoindole,
(11) le 2,3-diméthyl 5-amino 6-hydroxyindole,
(12) le 2,3-diméthyl 5-méthoxy 6-aminoindole,
(13) le 2,3-diméthyl 5-hydroxy 6-aminoindole,
(14) le 2,3-diméthyl 5-éthyl 6-aminoindole,
(15) le 2,3-diméthyl 5-chloro 6-aminoindole,
(16) le 2,3-diméthyl 5-acétylamino 6-méthoxyindole,
(17) le 2,3-diméthyl 5-amino 6-méthoxyindole,
(18) le N-méthyl 6-(β-hydroxyéthyl)aminoindole,
(19) le 2,3,4,5-tétraméthyl 6-aminoindole.
(20) le 6-N-méthylaminoindole,
(21) le N-méthyl 5-aminoindole,
(22) le N-méthyl 6-aminoindole,
(23) le N-méthyl 4-aminoindole,
(24) le 2,3-diméthyl 4-aminoindole,
(25) le 2,3-diméthyl 7-aminoindole,
(26) le 3-méthyl 6-aminoindole,
(27) le 2-méthyl 3-éthyl 6-aminoindole,
(28) le 2-méthyl 3-éthyl 7-aminoindole,
(29) le 4-N-méthylaminoindole,
(30) le 7-éthyl 6-aminoindole,
(31) le 7-éthyl 4-aminoindole,
(32) le 7-éthyl 6-N,β-hyroxyéthylaminoindole,
(33) le 4-méthyl 6-aminoindole,
(34) le 5-méthyl 6-aminoindole,
(35) le 7-méthyl 4-aminoindole,
(36) le 3-méthyl 7-éthyl 6-aminoindole,
(37) le 5,7-diméthyl 6-aminoindole,
(38) le 5,7-diéthyl 6-aminoindole,
(39) le 2-éthoxycarbonyl 5-méthyl 7-aminoindole,
(40) le 2-éthoxycarbonyl 5-chloro 7-aminoindole,
(41) le 2-éthoxycarbonyl 5-éthoxy 7-aminoindole,
(42) le 2-éthoxycarbonyl 5-méthoxy 7-aminoindole,
(43) le 5-méthoxy 7-(4'-diméthylamino 1'-méthylbutyl)aminoindole,
(44) le 5-méthoxy 7-(4'-diméthylaminobutyl)aminoindole,
(45) le 5-méthoxy 7-(4'-diéthylamino 1'-méthylbutyl)aminoindole,
(46) le 5-fluoro 6-aminoindole,
(47) le 5-fluoro 1-sec-butyl 6-aminoindole,
(48) le 5-fluoro 1-n-propyl 6-aminoindole,
(49) le 1-méthyl 2-méthoxycarbonyl 5-méthoxy 6-aminoindole,
(50) le 2-méthoxycarbonyl 5-méthoxy 6-aminoindole,
(51) le 2-éthoxycarbonyl 5-méthoxy 6-aminoindole,
(52) le 2-carboxy 5-méthoxy 6-aminoindole,
(53) le 1,2-diméthyl 5-hydroxy 6-aminoindole,
(54) le 2-méthoxycarbonyl 4-méthoxy 6-aminoindole,
et les sels de ces composes.

3. Procédé selon la revendication 1 ou 2, caractérisé par le fait que les précurseurs de colorants d'oxydation sont choisis parmi les paraphénylènediamines, les para-aminophénols, les précurseurs hétérocycliques para, les bases doubles.

4. Procédé selon la revendication 3, caractérisé par le fait que les paraphénylènediamines sont choisies parmi les composés répondant à la formule : 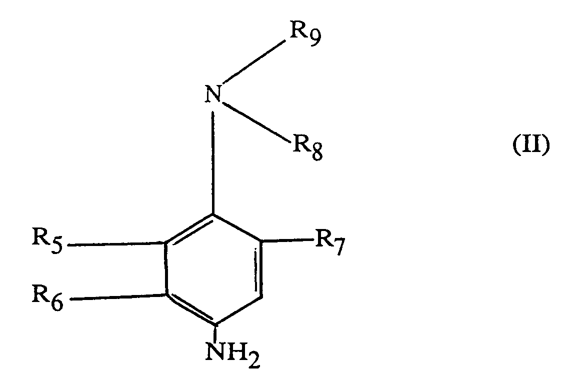 dans laquelle :
R₅, R₆ et R₇, identiques ou différents, représentent un atome d'hydrogène ou d'halogène, un radical alkyle ayant de 1 à 4 atomes de carbone, un radical alcoxy ayant de 1 à 4 atomes de carbone, un radical carboxy, sulfo, hydroxyalkylke ayant de 1 à 4 atomes de carbone;
R₈ et R₉, identiques ou différents, représentent un atome d'hydrogène, un radical alkyle, hydroxyalkyle, alcoxyalkyle, carbamylalkyle, mésylaminoalkyle, acétylaminoalkyle, uréidoalkyle, carbalcoxyaminoalkyle, pipéridinoalkyle, morpholinoalkyle; ces groupes alkyle ou alcoxy ayant de 1 à 4 atomes de carbone, ou bien R₈ et R₉ forment, conjointement avec l'atome d'azote auquel ils sont liés, un hétérocycle pipéridino ou morpholino, sous réserve que R₅ ou R₇ représente un atome d'hydrogène lorsque R₈ et R₉ ne représentent pas un atome d'hydrogène, ainsi que les sels de ces composés.

5. Procédé selon la revendication 4, caractérisé par le fait que les composés de formule (II) sont choisis parmi la p-phénylènediamine, la 2-méthyl-p- phénylènediamine, la méthoxyparaphénylènediamine, la chloropara- phénylènediamine, la 2,6-diméthylparaphénylènediamine, la 2,5- diméthylparaphénylènediamine, la 2,3-diméthylparaphénylènediamine, la 2-méthyl 5-méthoxypara-phénylènediamine, la 2,6-diméthyl 5-méthoxyparaphénylènediamine, la N,N-diméthylparaphénylène diamine, la 3-méthyl 4-amino N,N-diéthylaniline, la N,N-di-(β-hydroxyéthyl)paraphénylènediamine, la 3-méthyl 4-amino N,N-di-(β-hydroxyéthyl)aniline, la 3-chloro 4-amino N,N-di-(β-hydroxyéthyl)aniline, la 4-amino N,N-(éthyl,carbamylméthyl)aniline, la 3-méthyl 4-amino N,N-(éthyl,carbamylméthyl)aniline, la 4-amino N,N-(éthyl,β-pipéridinoéthyl)aniline, la 3-méthyl 4-amino N,N-(éthyl,β-pipéridinoéthyl) aniline, la 4-amino N,N-(éthyl,β-morpholinoéthyl)aniline, la 3-méthyl 4-amino N,N-(éthyl,β-morpholinoéthyl)aniline, la 4-amino N,N-(éthyl, β-acétylaminoéthyl)aniline, la 4-amino N-(β-méthoxyéthyl)aniline, la 3-méthyl 4-amino N,N-(éthyl,β-acétylaminoéthyl)aniline, la 4-amino N,N-(éthyl,β-mésylaminoéthyl)aniline, la 3-méthyl 4-amino N,N- (éthyl,β-mésylaminoéthyl)aniline, la 4-amino N,N-(éthyl,β-sulfoéthyl) aniline, la 3-méthyl 4-amino N,N-(éthyl,β-sulfoéthyl)aniline, la N-[(4'-amino)phényl]-morpholine, la N-[(4'-amino)phényl]pipéridine, la 2-hydroxyéthylparaphénylènediamine, la fluoroparaphénylènediamine, la carboxyparaphénylènediamine, la sulfoparaphénylènediamine, la 2-isopropylparaphénylènediamine, la 2-n-propylparaphénylènediamine, la 2-hydroxyméthylparaphénylènediamine, la N,N-diméthyl 3-méthyl paraphénylènediamine, la N,N(éthyl,β-hydroxyéthyl)paraphénylène diamine, la N-(dihydroxypropyl)paraphénylènediamine, la N-4'-aminophényl p-phénylènediamine, la N-phényl p-phénylènediamine, sous forme de base libre ou de sels.

6. Procédé selon la revendication 3, caractérisé par le fait que les les p-aminophénols, sont choisis parmi le p-aminophénol, le 2-méthyl 4-aminophénol, le 3-méthyl 4-aminophénol, le 2-chloro 4-aminophénol, le 3-chloro 4-aminophénol, le 2,6-diméthyl 4-aminophénol, le 3,5-diméthyl 4-aminophénol, le 2,3-diméthyl 4-amino phénol, le 2-hydroxyméthyl 4-aminophénol, le 2-(β-hydroxyéthyl) 4-aminophénol, le 2-méthoxy 4-aminophénol, le 3-méthoxy 4-amino phénol, le 2,5-diméthyl 4-aminophénol, le 2-méthoxyméthyl 4-amino phénol, le 2-β-hydroxyéthoxyméthyl 4-aminophénol.

7. Procédé selon la revendication 3, caractérisé par le fait que les bases dites doubles sont choisies parmi les bis-phényl alkylène diamines de formule : 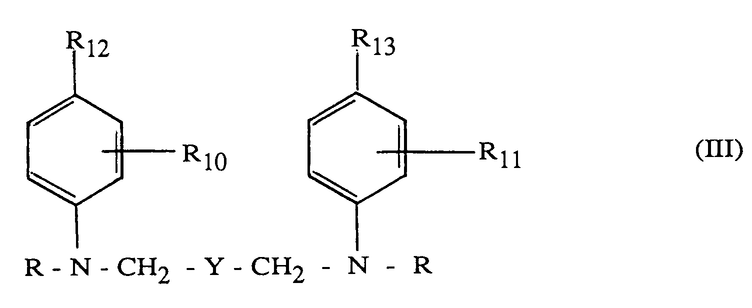 dans laquelle :
R₁₂ et R₁₃, identiques ou différents, représentent des groupements hydroxyle ou NHR₁₄, où R₁₄ désigne un atome d'hydrogène ou un radical alkyle inférieur;
R₁₀ et R₁₁, identiques ou différents, représentent soit des atomes d'hydrogène, soit des atomes d'halogène, soit des groupements alkyle;
R représente un atome d'hydrogène, un groupe alkyle, hydroxyalkyle ou aminoalkyle, dont le reste amino peut être substitué;
Y représente un radical pris dans le groupe constitué par les radicaux suivants : -(CH₂)ₙ-, (CH₂)_{n'}-O-(CH₂)_{n'}-, (CH₂)_{n'}-CHOH-(CH₂)ₙ, ;-(CH₂)ₙ,; 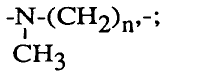 n est un nombre entier compris entre 0 et 8 et n' un nombre entier entre 0 et 4, ainsi que leurs sels d'addition d'acides.

8. Procédé selon la revendicati'on 7, caractérisé par le fait que la base dite double est choisie parmi les composés suivants : le N,N'bis-(β-hydroxyéthyl)N,N'-bis(4'-aminophényl)1,3-diamino 2-propanol, la N,N'bis-(β-hydroxyéthyl)N,N'-bis(4'-aminophényl)éthylènediamine, la N,N'bis-(4-aminophényl)tétraméthylènediamine, la N,N'bis-(β-hydroxy éthyl)N,N'bis(4-aminophényl)tétraméthylènediamine, la N,N'bis-(4-méthylaminophényl)tétraméthylènediamine, la N,N'bis(éthyl)N,N'-bis (4'-amino 3'-méthylphényl)éthylènediamine.

9. Procédé selon la revendication 1 ou 2, caractérisé par le fait que les précurseurs de colorants d'oxydation sont des précurseurs de colorants d'oxydation de type ortho choisis parmi les orthoaminophénols et les orthophénylènediamines.

10. Procédé selon l'une quelconque des revendications 1 à 9, caractérisé par le fait que l'agent oxydant est choisi parmi le peroxyde d'hydrogène, le peroxyde d'urée, les bromates de métaux alcalins, les persels.

11. Procédé selon l'une quelconque des revendications 1 à 10, caractérisé par le fait que le pH de la composition appliquée sur les fibres kératiniques est compris entre 3 et 6,9.

12. Procédé selon l'une quelconque des revendications 1 à 11, caractérisé par le fait que la composition utilisée pour la teinture des fibres kératiniques contient en plus des coupleurs hétérocyliques de la famille des aminoindoles de formule (I), d'autres coupleurs choisis parmi les métadiphénols, les métaaminophénols, les métaphénylènediamines, les méta N-acylaminophénols, les métauréidophénols, les métacarbalcoxyaminophénols, l'α-naphtol, les coupleurs possédant un groupement méthylène actif choisis parmi les composés dicétoniques et les pyrazolones, les coupleurs hétérocycliques ou le 4-hydroxyindole, le 6-hydroxyindole ou le 7-hydroxyindole.

13. Procédé selon la revendication 12, caractérisé par le fait que les coupleurs sont choisis parmi le 2,4-dihydroxyphénoxyéthanol, le 2,4-dihydroxyanisole, le métaaminophénol, la résorcine, le monométhyléther de résorcine, la 2-méthylrésorcine, le pyrocatéchol, le 2-méthyl 5-N-(β-hydroxyéthyl)aminophénol, le 2-méthyl 5-N-(β-mésylaminoéthyl)aminophénol, la 6-hydroxybenzomorpholine, le 2,4-diaminoanisole, le 2,4-diaminophénoxyéthanol, la 6-aminobenzomorpholine, le [2-N-(β-hydroxyéthyl)amino 4-amino]-phénoxyéthanol, le 2-amino 4-N-(β-hydroxyéthyl)aminoanisole, le (2,4-diamino)phényl-β,γ-dihydroxypropyléther, la 2,4-diaminophénoxyéthylamine, le 1,3-diméthoxy 2,4-diaminobenzène, le 2-méthyl 5-aminophénol, le 2,6-diméthyl 3-aminophénol, le 3,4-méthylènedioxyphénol, la 3,4-méthylènedioxyaniline, la 2-chlororésorcine et leurs sels.

14. Procédé selon l'une quelconque des revendications 1 à 13, caractérisé par le fait que la composition contient des agents tensioactifs anioniques, cationiques, non ioniques, amphotères ou leurs mélanges; des agents épaississants, des agents anti-oxydants et/ou tout autre adjuvant cosmétiquement acceptable.

15. Procédé selon l'une quelconque des revendications 1 à 14, caractérisé par le fait que le milieu approprié pour la teinture est constitué par de l'eau ou par un mélange d'eau et d'un solvant choisi parmi les alcanols inférieurs en C₂-C₄, le glycérol, les glycols, les éthers de glycols, les alcools aromatiques ou leurs mélanges.

16. Agent de teinture des fibres kératiniques et en particulier des cheveux, caractérisé par le fait qu'il comporte au moins deux composants : un composant (A) constitué par une composition contenant dans un milieu approprié pour la teinture, un coupleur amino indolique répondant à la formule (I) tel que défini dans la revendication 1 ou 2 et un précurseur de colorant d'oxydation tel que défini dans l'une quelconque des revendications 1 et 3 à 9, un composant (B) constitué par une composition contenant dans un milieu approprié pour la teinture, un agent oxydant, le pH des composants (A) et (B) étant tel qu'après mélange dans des proportions de 90 à 10% pour le composant (A) et de 10 à 90% pour le composant (B), la composition résultante ait un pH inférieur à 7.

17. Agent selon la revendication 16, caractérisé par le fait que le composant (A) a un pH compris entre 3 et 10,5.

18. Agent selon la revendication 16 ou 17, caractérisé par le fait que le composant (A) contient les précurseurs de colorants par oxydation ainsi que les coupleurs dans des proportions comprises entre 0,05 et 7% en poids par rapport au poids total du composant (A).

19. Agent selon l'une quelconque des revendications 16 à 18, caractérisé par le fait que la concentration en composé de formule (I) est comprise entre 0,012 et 4% en poids par rapport au poids total du composant (A).

20. Agent selon l'une quelconque des revendications 16 à 19, caractérisé par le fait que le composant (A) contient des agents tensioactifs dans des proportions de 0,1 à 55% en poids, des solvants en plus de l'eau dans des proportions comprises entre 0,5 et 40% en poids, des agents épaississants dans des proportions comprises entre 0,1 et 5% en poids, des agents antioxydants dans des proportions comprises entre 0,02 et 1,5% en poids, et/ou tout autre adjuvant cosmétiquement acceptable.

21. Agent selon l'une quelconque des revendications 16 à 20, caractérisé par le fait que le composant (B) a un pH qui a une valeur minimum de 1 et inférieure à 7.

22. Procédé de teinture des fibres kératiniques et en particulier des cheveux, caractérisé par le fait qu'il comporte une première étape consistant à stocker un agent de teinture tel que défini dans l'une quelconque des revendications 16 à 21 et à procéder avant application au mélange des composants (A) et (B) dans des proportions de 10 à 90% pour le composant (A) et de 90 à 10% pour le composant (B), de façon à obtenir une composition ayant un pH inférieur à 7 et d'appliquer ce mélange immédiatement après préparation sur les fibres kératiniques.

23. Dispositif à plusieurs compartiments ou kit de teinture, caractérisé par le fait qu'il comporte au moins deux compartiments dont un premier compartiment renferme le composant (A) tel que déini dans l'une quelconque des revendications 16 à 20, et le second compartiment renferme le composant (B) tel que défini dans les revendications 16 et 21.

24. Dispositif selon la revendication 23, caractérisé par le fait qu'il est équipé d'un moyen permettant de délivrer sur les cheveux le mélange souhaité des composants (A) et (B).

25. Procédé de teinture selon l'une quelconque des revendications 1 à 15, caractérisé par le fait que l'on applique sur les cheveux la composition et qu'on la laisse poser pendant 3 à 40 minutes, que l'on rince les cheveux et qu'on précède éventuellement à un shampooing avant un nouveau rinçage et séchage.

26. Utilisation comme coupleurs des composés amino indoliques répondant à la formule (I) tels que définis dans la revendication 1 à 2 pour la teinture en milieu acide des fibres kératiniques, en association avec des précurseurs de colorants d'oxydation.

27. Composition de teinture de fibres kératiniques, prête à l'emploi telle que mise en oeuvre dans le procédé selon l'une quelconque des revendications 1 à 15.

28. Composition selon la revendication 27, contenant le composé de formule (I) dans des proportions de 0,01 à 3,5% en poids par rapport au poids total de la composition.

29. Composition tinctoriale pour les fibres kératiniques, en particulier des cheveux, caractérisé par le fait qu'elle contient dans un milieu approprié pour la teinture au moins un précurseur de colorant d'oxydation et un coupleur répondant à la formule suivante : 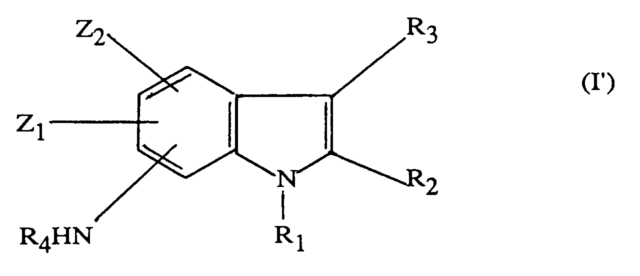 dans laquelle :
R₁ désigne hydrogène ou un groupement alkyle en C₁-C₄;
R₂ et R₃, indépendamment l'un de l'autre, représentent un atome d'hydrogène, un groupement alkyle en C₁-C₄ ou un groupement COOR', R' étant un radical alkyle en C₁-C₄;
dans laquelle :
R₄ représente un atome d'hydrogène, un radical alkyle en C₁-C₄, un groupe hydroxyalkyle en C₁-C₄, polyhydroxyalkyle en C₂-C₄, acétyle ou aminoalkyle en C₁-C₆ dont l'amine peut être éventuellement mono- ou disubstituée par un alkyle en C₁-C₄;
Z₁ et Z₂, identiques ou différents, représentent un atome d'hydrogène, un groupe alkyle en C₁-C₄, hydroxy, un halogène, un alcoxy en C₁-C₄;
sous réserve que lorsque NHR₄ est en position 4, 6 ou 7, Z₁ et Z₂ ne désignent pas simultanément un atome d'hydrogène; ainsi que les sels de ces composés, à l'exception du 6-hydroxy 5-acétylamino 2,3-diméthylindole.

30. Agent de teinture selon l'une quelconque des revendications 16 à 20, caractérisé par le fait que le composant (A) contient comme coupleur un aminoindole répondant à la formule suivante : 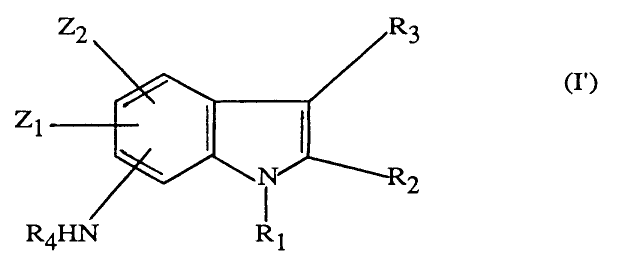 dans laquelle :
R₁ désigne hydrogène ou un groupement alkyle en C₁-C₄;
R₂ et R₃, indépendamment l'un de l'autre, représentent un atome d'hydrogène, un groupe alkyle en C₁-C₄ ou un groupement COOR' où R' désigne un atome d'hydrogène ou un alkyle en C₁-C₄; au moins un des radicaux R₂ et R₃ désigne un atome d'hydrogène;
R₄ représente un groupement alkyle en C₁-C₄, hydroxyalkyle en C₁-C₄ ou polyhydroxyalkyle en C₂-C₄; le groupement -NHR₄ occupant les positions 4, 6 ou 7;
Z₁ et Z₂, identiques ou différents, représentent un atome d'hydrogène, un alkyle en C₁-C₄, un hydroxy, un atome d'halogène ou un alcoxy en C₁-C₄;
sous réserve que lorsque NHR₄ est en position 4, 6 ou 7, Z₁ et Z₂ ne désignent pas simultanément un atome d'hydrogène; ainsi que les sels de ces composés.

31. Procédé de préparation d'un composé répondant à la formule suivante : 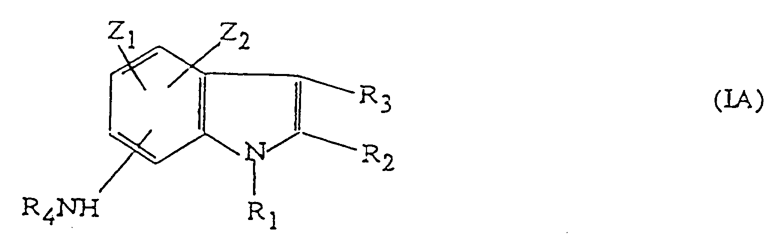 dans laquelle :
R₁ désigne hydrogène ou un groupement alkyle en C₁-C₄;
R₂ et R₃, indépendamment l'un de l'autre, représentent un atome d'hydrogène, un groupe alkyle en C₁-C₄ ou un groupement COOR' où R' désigne un atome d'hydrogène ou un alkyle en C₁-C₄; au moins un des radicaux R₂ et R₃ désigne un atome d'hydrogène;
R₄ représente un groupement alkyle en C₁-C₄, hydroxyalkyle en C₁-C₄ ou polyhydroxyalkyle en C₂-C₄; le groupement -NHR₄ occupant les positions 4, 6 ou 7;
Z₁ et Z₂, identiques ou différents, représentent un atome d'hydrogène, un alkyle en C₁-C₄, un hydroxy, un atome d'halogène ou un alcoxy en C₁-C₄; au moins un des groupements Z₁ et Z₂ est différent d'hydrogène;
ainsi que les sels de ce composé,
caractérisé par le fait qu'ils sont obtenus par tosylation ou formylation d'un composé de formule : 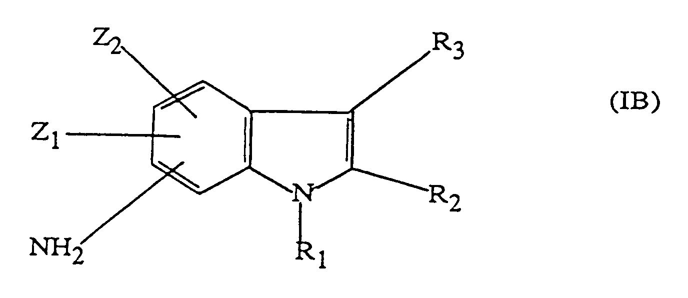 où R₁, R₂, R₃, Z₁ et Z₂ ont les mêmes significations indiquées ci-dessus, suivie d'une alkylation par un halogénure d'alkyle puis par une détosylation ou détosylation selon les méthodes conventionnelles.

32. Procédé de préparation des composés 7-éthyl 4-aminoindole et 7-éthyl 6-aminoindole, caractérisé par le fait qu'ils sont obtenus par réduction respectivement du 7-éthyl 4-nitroindole ou du 7-éthyl 6-nitroindole.

## Claims (Claims for the following Contracting State(s): AT, BE, CH, DE, DK, FR, GB, GR, IT, NL, SE)

1. Process for dyeing keratinous fibres, in particular human keratinous fibres such as hair, characterized in that there is applied to these fibres a composition containing, in a suitable medium for dyeing, at least one coupler corresponding to the formula: 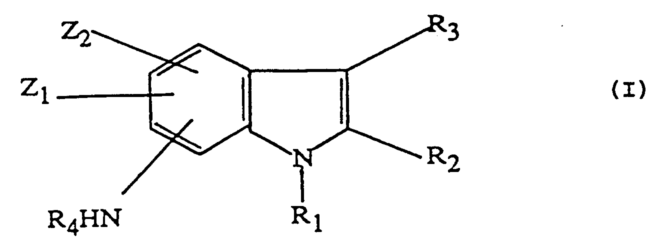 in which:
R₁ denotes hydrogen or a C₁-C₄ alkyl group,
R₂ and R₃, independently of each other, denote a hydrogen atom, a C₁-C₄ alkyl group or a COOR' group, R' being a C₁-C₄ alkyl radical or a hydrogen atom,
R₄ denotes a hydrogen atom, a C₁-C₄ alkyl radical, a C₁-C₄ hydroxyalkyl, C₂-C₄ polyhydroxyalkyl, acetyl or C₁-C₆ aminoalkyl group, in which the amine may be optionally mono- or disubstituted by a C₁-C₄ alkyl,
Z₁ and Z₂, which are identical or different, denote a hydrogen atom or a C₁-C₄ alkyl, hydroxyl, halogen or alkoxy (C₁-C₄) group,
and the salts of these compounds;
- at least one oxidation dye precursor,
- at least one oxidizing agent, the pH of the composition applied to the fibres being lower than 7.

2. Process according to Claim 1, characterized in that the compound of formula (I) is chosen from:
(1) 5-aminoindole,
(2) 4-aminoindole,
(3) 2-methyl-6-aminoindole,
(4) 2,3,4-trimethyl-6-aminoindole,
(5) 2,3,7-trimethyl-6-aminoindole,
(6) 2,3,5-trimethyl-6-aminoindole,
(7) 6-aminoindole,
(8) 7-aminoindole,
(9) 6-(β-hydroxyethylamino)indole,
(10) 2-methyl-5-hydroxy-6-aminoindole,
(11) 2,3-dimethyl-5-amino-6-hydroxyindole,
(12) 2,3-dimethyl-5-methoxy-6-aminoindole,
(13) 2,3-dimethyl-5-hydroxy-6-aminoindole,
(14) 2,3-dimethyl-5-ethyl-6-aminoindole,
(15) 2,3-dimethyl-5-chloro-6-aminoindole,
(16) 2,3-dimethyl-5-acetylamino-6-methoxyindole,
(17) 2,3-dimethyl-5-amino-6-methoxyindole,
(18) N-methyl-6-(β-hydroxyethyl)aminoindole,
(19) 2,3,4,5-tetramethyl-6-aminoindole,
(20) 6-N-methylaminoindole,
(21) N-methyl-5-aminoindole,
(22) N-methyl-6-aminoindole,
(23) N-methyl-4-aminoindole,
(24) 2,3-dimethyl-4-aminoindole,
(25) 2,3-dimethyl-7-aminoindole,
(26) 3-methyl-6-aminoindole,
(27) 2-methyl-3-ethyl-6-aminoindole,
(28) 2-methyl-3-ethyl-7-aminoindole,
(29) 4-N-methylaminoindole,
(30) 7-ethyl-4-aminoindole,
(31) 7-ethyl-6-aminoindole,
(32) 7-ethyl-6-N-β-hydroxyethylaminoindole,
(33) 4-methyl-6-aminoindole,
(34) 5-methyl-6-aminoindole,
(35) 7-methyl-4-aminoindole,
(36) 3-methyl-7-ethyl-6-aminoindole,
(37) 5,7-dimethyl-6-aminoindole,
(38) 5,7-diethyl-6-aminoindole,
(39) 2-ethoxycarbonyl-5-methyl-7-aminoindole,
(40) 2-ethoxycarbony1-5-chloro-7-aminoindole,
(41) 2-ethoxycarbonyl-5-ethoxy-7-aminoindole,
(42) 2-ethoxycarbonyl-5-methoxy-7-aminoindole,
(43) 5-methoxy-7-(4'-dimethylamino-1'-methylbutyl)-aminoindole,
(44) 5-methoxy-7-(4'-dimethylaminobutyl)aminoindole,
(45) 5-methoxy-7-(4'-diethylamino-1'-methylbutyl)-aminoindole,
(46) 5-fluoro-6-aminoindole,
(47) 5-fluoro-1-sec-butyl-6-aminoindole,
(48) 5-fluoro-1-n-propyl-6-aminoindole,
(49) 1-methyl-2-methoxycarbonyl-5-methoxy-6-aminoindole,
(50) 2-methoxycarbonyl-5-methoxy-6-aminoindole,
(51) 2-ethoxycarbonyl-5-methoxy-6-aminoindole,
(52) 2-carboxy-5-methoxy-6-aminoindole,
(53) 1,2-dimethyl-5-hydroxy-6-aminoindole,
(54) 2-methoxycarbonyl-4-methoxy-6-aminoindole,
and the salts of these compounds.

3. Process according to Claim 1 or 2, characterized in that the oxidation dye precursors are chosen from para-phenylenediamines, para-aminophenols, para-heterocyclic precursors and double bases.

4. Process according to Claim 3, characterized in that the para-phenylenediamines are chosen from the compounds corresponding to the formula: 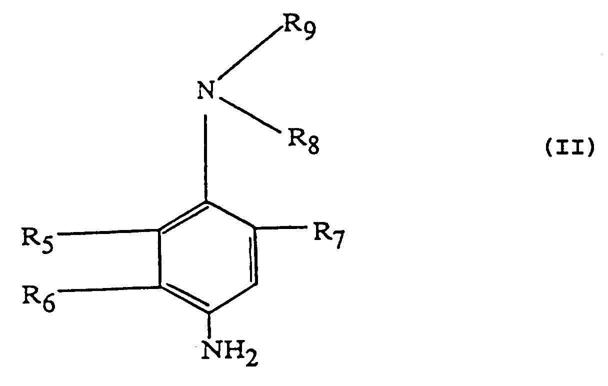 in which:
R₅, R₆ and R₇, which are identical or different, denote a hydrogen or halogen atom, an alkyl radical containing from 1 to 4 carbon atoms, an alkoxy radical containing from 1 to 4 carbon atoms, a carboxyl, sulpho or hydroxyalkyl radical containing from 1 to 4 carbon atoms,
R₈ and R₉, which are identical or different, denote a hydrogen atom or an alkyl, hydroxyalkyl, alkoxyalkyl, carbamylalkyl, mesylaminoalkyl, acetylaminoalkyl, ureidoalkyl, carbalkoxyaminoalkyl, piperidinoalkyl or morpholinoalkyl radical, these alkyl or alkoxy groups containing from 1 to 4 carbon atoms, or else R₈ and R₉, together with the nitrogen atom to which they are linked, form a piperidino or morpholino heterocyclic ring, provided that R₅ or R₇ denotes a hydrogen atom when R₈ and R₉ do not denote a hydrogen atom, and the salts of these compounds.

5. Process according to Claim 4, characterized in that the compounds of formula (II) are chosen from p-phenylenediamine, 2-methyl-p-phenylenediamine, methoxy-para-phenylenediamine, chloro-para-phenylenediamine, 2,6-dimethyl-para-phenylenediamine, 2,5-dimethyl-para-phenylenediamine, 2,3-dimethyl-para-phenylenediamine, 2-methyl-5-methoxy-para-phenylenediamine, 2,6-dimethyl-5-methoxy-para-phenylenediamine, N,N-dimethyl-para-phenylenediamine, 3-methyl-4-amino-N,N-diethylaniline, N,N-di(β-hydroxyethyl)-para-phenylenediamine, 3-methyl-4-amino-N,N-di(β-hydroxyethyl)aniline, 3-chloro-4-amino-N,N-di(β-hydroxyethyl)aniline, 4-amino-N,N-(ethyl,carbamylmethyl)aniline, 3-methyl-4-amino-N,N-(ethyl,carbamylmethyl)aniline, 4-amino-N,N-(ethyl,β-piperidinoethyl)aniline, 3-methyl-4-amino-N,N-(ethyl,β-piperidinoethyl)aniline, 4-amino-N,N-(ethyl,β-morpholinoethyl)-aniline, 3-methyl-4-amino-N,N-(ethyl,β-morpholinoethyl)-aniline, 4-amino-N,N-(ethyl,β-acetylaminoethyl)aniline, 4-amino-N-(β-methoxyethyl)aniline, 3-methyl-4-amino-N,N-(ethyl,β-acetylaminoethyl)aniline, 4-amino-N,N-(ethyl,β-mesylaminoethyl)aniline, 3-methyl-4-amino-N,N-(ethyl,β-mesylaminoethyl)aniline, 4-amino-N,N-(ethyl,β-sulphoethyl)aniline, 3-methyl-4-amino-N,N-(ethyl,β-sulphoethyl)aniline, N-[(4'-amino)phenyl]morpholine, N-[(4'-amino)phenyl]piperidine, 2-hydroxyethyl-para-phenylenediamine, fluoro-para-phenylenediamine, carboxy-para-phenylenediamine, sulpho-para-phenylenediamine, 2-isopropyl-para-phenylenediamine, 2-n-propyl-para-phenylenediamine, 2-hydroxymethyl-para-phenylenediamine, N,N-dimethyl-3-methyl-para-phenylenediamine, N,N-(ethyl,β-hydroxyethyl)-para-phenylenediamine, N-(dihydroxypropyl)-para-phenylenediamine, N-4'-aminophenyl-p-phenylenediamine and N-phenyl-p-phenylenediamine, in the form of free base or salts.

6. Process according to Claim 3, characterized in that the p-aminophenols are chosen from p-aminophenol, 2-methyl-4-aminophenol, 3-methyl-4-aminophenol, 2-chloro-4-aminophenol, 3-chloro-4-aminophenol, 2,6-dimethyl-4-aminophenol, 3,5-dimethyl-4-aminophenol, 2,3-dimethyl-4-aminophenol, 2-hydroxymethyl-4-aminophenol, 2-(β-hydroxyethyl)-4-aminophenol, 2-methoxy-4-aminophenol, 3-methoxy-4-aminophenol, 2,5-dimethyl-4-aminophenol, 2-methoxymethyl-4-aminophenol and 2-β-hydroxyethoxymethyl-4-aminophenol,

7. Process according to Claim 3, characterized in that the so-called double bases are chosen from bis-phenylalkylenediamines of formula: 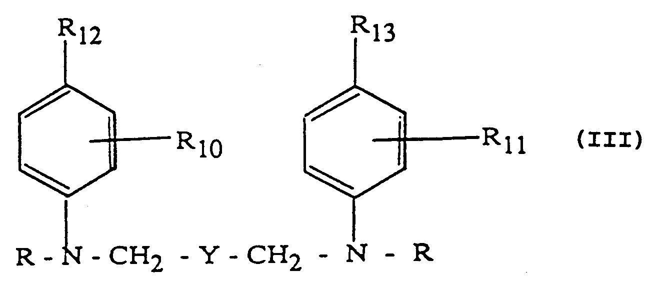 in which:
R₁₂ and R₁₃, which are identical or different, denote hydroxyl or NHR₁₄ groups, where R₁₄ denotes a hydrogen atom or a lower alkyl radical,
R₁₀ and R₁₁, which are identical or different, denote either hydrogen atoms or halogen atoms or alkyl groups,
R denotes a hydrogen atom or an alkyl, hydroxyalkyl or aminoalkyl group in which the amino residue can be substituted,
Y denotes a radical taken from the group consisting of the following radicals:
-(CH₂)ₙ-,(CH₂)_{n'}-O-(CH₂)_{n'}-,
(CH₂)_{n'}-CHOH-(CH₂)_{n'};-(CH₂)_{n'} 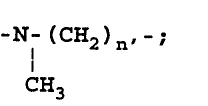 n being an integer between 0 and 8 and n' an integer between 0 and 4, and their addition salts with acids.

8. Process according to Claim 7, characterized in that the so-called double base is chosen from the following compounds: N,N'-bis(β-hydroxyethyl)-N,N'-bis(4'-aminophenyl)-1,3-diamino-2-propanol, N,N'-bis(β-hydroxyethyl)-N,N'-bis(4'-aminophenyl)ethylenediamine, N,N'-bis(4-aminophenyl)tetramethylenediamine, N,N'-bis(β-hydroxyethyl)-N,N'-bis(4-aminophenyl)tetramethylenediamine, N,N'-bis(4-methylaminophenyl)tetramethylene diamine and N,N'-bis(ethyl)-N,N'-bis(4'-amino-3'-methylphenyl)ethylenediamine.

9. Process according to Claim 1 or 2, characterized in that the oxidation dye precursors are oxidation dye precursors of ortho type which are chosen from ortho-aminophenols and ortho-phenylenediamines.

10. Process according to any one of Claims 1 to 9, characterized in that the oxidizing agent is chosen from hydrogen peroxide, urea peroxide, alkali metal bromates and persalts.

11. Process according to any one of Claims 1 to 10, characterized in that the pH of the composition applied to the keratinous fibres is between 3 and 6.9.

12. Process according to any one of Claims 1 to 11, characterized in that the composition employed for dyeing keratinous fibres additionally contains heterocyclic couplers of the class of aminoindoles of formula (I), other couplers chosen from meta-diphenols, meta-aminophenols, meta-phenylenediamines, meta-N-acylaminophenols, meta-ureidophenols, meta-carbalkoxyaminophenols, α-naphthol, couplers containing an active methylene group and chosen from diketonic compounds and pyrazolones, heterocyclic couplers or 4-hydroxyindole, 6-hydroxyindole or 7-hydroxyindole.

13. Process according to Claim 12, characterized in that the couplers are chosen from 2,4-dihydroxyphenoxy-ethanol, 2,4-dihydroxyanisole, meta-aminophenol, resorcinol, resorcinol monomethyl ether, 2-methyl-resorcinol, pyrocatechol, 2-methyl-5-N-(β-hydroxyethyl)-aminophenol, 2-methyl-5-N-(β-mesylaminoethyl)aminophenol, 6-hydroxybenzomorpholine, 2,4-diaminoanisole, 2,4-diaminophenoxyethanol, 6-aminobenzomorpholine, [2-N-(β-hydroxyethyl)amino-4-amino]-phenoxyethanol, 2-amino-4-N-(β-hydroxyethyl)aminoanisole, (2,4-diamino)-phenyl-β,γ-dihydroxypropyl ether, 2,4-diaminophenoxyethylamine, 1,3-dimethoxy-2,4-diaminobenzene, 2-methyl-5-aminophenol, 2,6-dimethyl-3-aminophenol, 3,4-methylenedioxyphenol, 3,4-methylenedioxyaniline, 2-chlororesorcinol and their salts.

14. Process according to any one of Claims 1 to 13, characterized in that the composition contains anionic, cationic, nonionic or amphoteric surface-active agents or mixtures thereof, thickening agents, antioxidant agents and/or any other cosmetically acceptable adjuvant.

15. Process according to any one of Claims 1 to 14, characterized in that the suitable medium for dyeing consists of water or of a mixture of water and of a solvent chosen from C₂-C₄ lower alkanols, glycerol, glycols, glycol ethers, aromatic alcohols or their mixtures.

16. Agent for dyeing keratinous fibres and in particular hair, characterized in that it comprises at least two components: a component (A) consisting of a composition containing, in a suitable medium for dyeing, an aminoindole coupler corresponding to the formula (I) as defined in Claim 1 or 2 and an oxidation dye precursor as defined in any one of Claims 1 and 3 to 9, a component (B) consisting of a composition containing, in a suitable medium for dyeing, an oxidizing agent, the pH of the components (A) and (B) being such that after mixing in proportions of 90 to 10 % in the case of the component (A) and of 10 to 90 % in the case of the component (B), the resulting composition has a pH lower than 7.

17. Agent according to Claim 16, characterized in that the component (A) has a pH of between 3 and 10.5.

18. Agent according to Claim 16 or 17, characterized in that the component (A) contains oxidation dye precursors and couplers in proportions of between 0.05 and 7 % by weight relative to the total weight of the component (A).

19. Agent according to any one of Claims 16 to 18, characterized in that the concentration of compound of formula (I) is between 0.012 and 4 % by weight relative to the total weight of the component (A).

20. Agent according to any one of Claims 16 to 19, characterized in that the component (A) contains surface-active agents in proportions of 0.1 to 55 % by weight, solvents in addition to water in proportions of between 0.5 and 40 % by weight, thickening agents in proportions of between 0.1 and 5 % by weight, antioxidant agents in proportions of between 0.02 and 1.5 % by weight, and/or any other cosmetically acceptable adjuvant.

21. Agent according to any one of Claims 16 to 20, characterized in that the component (B) has a pH which has a minimum value of 1 and lower than 7.

22. Process for dyeing keratinous fibres and in particular hair, characterized in that it comprises a first stage consisting in storing a dyeing agent as defined in any one of Claims 16 to 21 and in mixing the components (A) and (B) before application, in proportions of 10 to 90 % in the case of the component (A) and of 90 to 10 % in the case of the component (B), so as to obtain a composition which has a pH lower than 7 and in applying this mixture to the keratinous fibres immediately after preparation.

23. Multicompartment device or dyeing kit, characterized in that it comprises at least two compartments, of which a first compartment contains the component (A) as defined in any one of Claims 16 to 20, and the second compartment contains the component (B) as defined in Claims 16 and 21.

24. Device according to Claim 23, characterized in that it is provided with a means permitting the desired mixture of the components (A) and (B) to be delivered onto the hair.

25. Dyeing process according to any one of Claims 1 to 15, characterized in that the composition is applied to the hair and is left in place for 3 to 40 minutes, that the hair is rinsed and that it is optionally shampooed before a new rinsing and drying.

26. Use as couplers of the aminoindole compounds corresponding to the formula (I) as defined in Claim 1 to 2 for dyeing keratinous fibres in acidic medium, in combination with oxidation dye precursors.

27. Composition for dyeing keratinous fibres, ready for use as used in the process according to any one of Claims 1 to 15.

28. Composition according to Claim 27, containing the compound of formula (I) in proportions of 0.01 to 3.5 % by weight relative to the total weight of the composition.

29. Dyeing composition for keratinous fibres, in particular hair, characterized in that it contains, in a suitable medium for dyeing, at least one oxidation dye precursor and a coupler corresponding to the following formula: 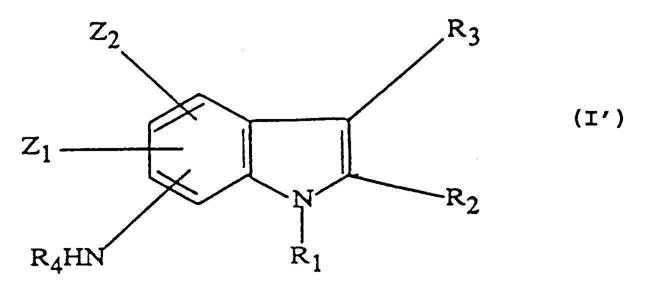 in which:
R₁ denotes hydrogen or a C₁-C₄ alkyl group,
R₂ and R₃, independently of each other, denote a hydrogen atom, a C₁-C₄ alkyl group or a COOR' group, R' being a C₁-C₄ alkyl radical, in which:
R₄ denotes a hydrogen atom, a C₁-C₄ alkyl radical, a C₁-C₄ hydroxyalkyl, C₂-C₄ polyhydroxyalkyl, acetyl or C₁-C₆ aminoalkyl group in which the amine may be optionally mono- or disubstituted by a C₁-C₄ alkyl,
Z₁ and Z₂, which are identical or different, denote a hydrogen atom or a C₁-C₄ alkyl, hydroxyl, a halogen or a C₁-C₄ alkoxy group,
provided that, when NHR₄ is in position 4, 6 or 7, Z₁ and Z₂ do not simultaneously denote a hydrogen atom; and the salts of these compounds, with the exception of 6-hydroxy-5-acetylamino-2,3-dimethylindole.

30. Dyeing agent according to any one of Claims 16 to 20, characterized in that the component (A) contains as coupler an aminoindole corresponding to the following formula: 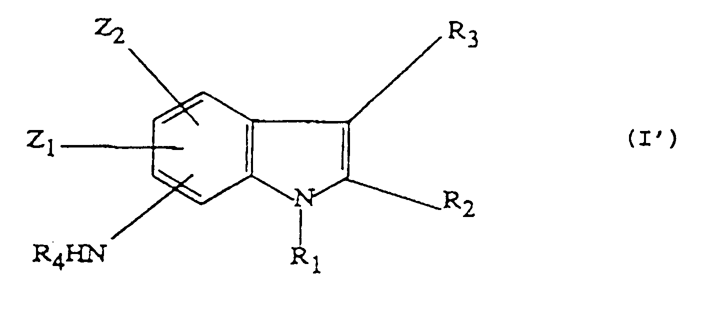 in which:
R₁ denotes hydrogen or a C₁-C₄ alkyl group,
R₂ and R₃, independently of each other, denote a hydrogen atom, a C₁-C₄ alkyl group or a COOR' group where R' denotes a hydrogen atom or a C₁-C₄ alkyl; at least one of the radicals R₂ and R₃ denotes a hydrogen atom,
R₄ denotes a C₁-C₄ alkyl, C₁-C₄ hydroxyalkyl or C₂-C₄ polyhydroxyalkyl group, the group -NHR₄ occupying positions 4, 6 or 7,
Z₁ and Z₂, which are identical or different, denote a hydrogen atom, a C₁-C₄-alkyl, a hydroxyl, a halogen atom or a C₁-C₄ alkoxy,
provided that, when NHR₄ is in position 4, 6 or 7, Z₁ and Z₂ do not simultaneously denote a hydrogen atom; and the salts of these compounds.

31. Compound corresponding to the following formula: 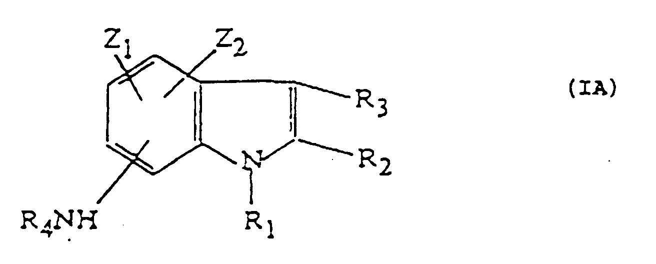 in which:
R₁ denotes hydrogen or a C₁-C₄ alkyl group,
R₂ and R₃, independently of each other, denote a hydrogen atom, a C₁-C₄ alkyl group or a COOR' group where R' denotes a hydrogen atom or a C₁-C₄ alkyl; at least one of the radicals R₂ and R₃ denotes a hydrogen atom,
R₄ denotes a C₁-C₄ alkyl, C₁-C₄ hydroxyalkyl or C₂-C₄ polyhydroxyalkyl group, the group -NHR₄ occupying positions 4, 6 or 7,
Z₁ and Z₂, which are identical or different, denote a hydrogen atom, a C₁-C₄ alkyl, a hydroxyl, a halogen atom or a C₁-C₄ alkoxy; at least one of the groups Z₁ and Z₂ is other than hydrogen,
and the salts of these compounds.

32. Compound chosen from:
7-ethyl-4-aminoindole,
7-ethyl-6-aminoindole,
and the salts of these compounds.

## Claims (Claims for the following Contracting State(s): ES)

1. Process for dyeing keratinous fibres, in particular human keratinous fibres such as hair, characterized in that there is applied to these fibres a composition containing, in a suitable medium for dyeing, at least one coupler corresponding to the formula: 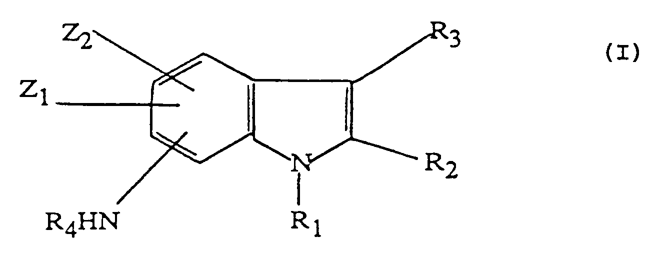 in which:
R₁ denotes hydrogen or a C₁-C₄ alkyl group,
R₂ and R₃, independently of each other, denote a hydrogen atom, a C₁-C₄ alkyl group or a COOR' group, R' being a C₁-C₄ alkyl radical or a hydrogen atom,
R₄ denotes a hydrogen atom, a C₁-C₄ alkyl radical, a C₁-C₄ hydroxyalkyl, C₂-C₄ polyhydroxyalkyl, acetyl or C₁-C₆ aminoalkyl group, in which the amine may be optionally mono- or disubstituted by a C₁-C₄ alkyl,
Z₁ and Z₂, which are identical or different, denote a hydrogen atom or a C₁-C₄ alkyl, hydroxyl, halogen or alkoxy (C₁-C₄) group,
and the salts of these compounds;
- at least one oxidation dye precursor,
- at least one oxidizing agent, the pH of the composition applied to the fibres being lower than 7.

2. Process according to Claim 1, characterized in that the compound of formula (I) is chosen from:
(1) 5-aminoindole,
(2) 4-aminoindole,
(3) 2-methyl-6-aminoindole,
(4) 2,3,4-trimethyl-6-aminoindole,
(5) 2,3,7-trimethyl-6-aminoindole,
(6) 2,3,5-trimethyl-6-aminoindole,
(7) 6-aminoindole,
(8) 7-aminoindole,
(9) 6-(β-hydroxyethylamino)indole,
(10) 2-methyl-5-hydroxy-6-aminoindole,
(11) 2,3-dimethyl-5-amino-6-hydroxyindole,
(12) 2,3-dimethyl-5-methoxy-6-aminoindole,
(13) 2,3-dimethyl-5-hydroxy-6-aminoindole,
(14) 2,3-dimethyl-5-ethyl-6-aminoindole,
(15) 2,3-dimethyl-5-chloro-6-aminoindole,
(16) 2,3-dimethyl-5-acetylanino-6-methoxyindole,
(17) 2,3-dimethyl-5-amino-6-methoxyindole,
(18) N-methyl-6-(β-hydroxyethyl)aminoindole,
(19) 2,3,4,5-tetramethyl-6-aminoindole,
(20) 6-N-methylaminoindole,
(21) N-methyl-5-aminoindole,
(22) N-methyl-6-aminoindole,
(23) N-methyl-4-aminoindole,
(24) 2,3-dimethyl-4-aminoindole,
(25) 2,3-dimethyl-7-aminoindole,
(26) 3-methyl-6-aminoindole,
(27) 2-methyl-3-ethyl-6-aminoindole,
(28) 2-methyl-3-ethyl-7-aminoindole,
(29) 4-N-methylaminoindole,
(30) 7-ethyl-4-aminoindole,
(31) 7-ethyl-6-aminoindole,
(32) 7-ethyl-6-N-β-hydroxyethylaminoindole,
(33) 4-methyl-6-aminoindole,
(34) 5-methyl-6-aminoindole,
(35) 7-methyl-4-aminoindole,
(36) 3-methyl-7-ethyl-6-aminoindole,
(37) 5,7-dimethyl-6-aminoindole,
(38) 5,7-diethyl-6-aminoindole,
(39) 2-ethoxycarbonyl-5-methyl-7-aminoindole,
(40) 2-ethoxycarbonyl-5-chloro-7-aminoindole,
(41) 2-ethoxycarbonyl-5-ethoxy-7-aminoindole,
(42) 2-ethoxycarbonyl-5-methoxy-7-aminoindole,
(43) 5-methoxy-7-(4'-dimethylamino-1'-methylbutyl)-aminoindole,
(44) 5-methoxy-7-(4'-dimethylaminobutyl)aminoindole,
(45) 5-methoxy-7-(4'-diethylamino-1'-methylbutyl)-aminoindole,
(46) 5-fluoro-6-aminoindole,
(47) 5-fluoro-1-sec-butyl-6-aminoindole,
(48) 5-fluoro-1-n-propyl-6-aminoindole,
(49) 1-methyl-2-methoxycarbonyl-5-methoxy-6-aminoindole,
(50) 2-methoxycarbonyl-5-methoxy-6-aminoindole,
(51) 2-ethoxycarbonyl-5-methoxy-6-aminoindole,
(52) 2-carboxy-5-methoxy-6-aminoindole,
(53) 1,2-dimethyl-5-hydroxy-6-aminoindole,
(54) 2-methoxycarbonyl-4-methoxy-6-aminoindole, and
the salts of these compounds.

3. Process according to Claim 1 or 2, characterized in that the oxidation dye precursors are chosen from para-phenylenediamines, para-aminophenols, para-heterocyclic precursors and double bases.

4. Process according to Claim 3, characterized in that the para-phenylenediamines are chosen from the compounds corresponding to the formula: 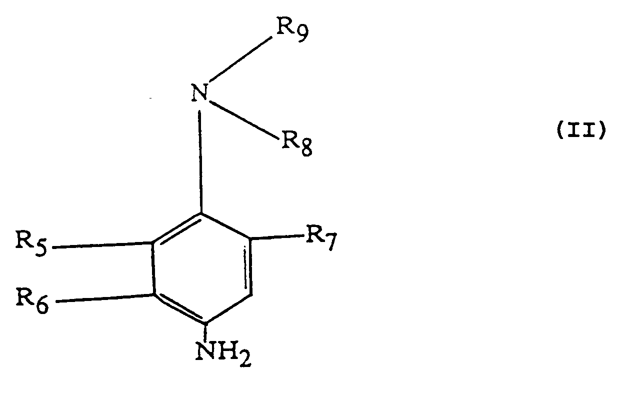 in which:
R₅, R₆ and R₇, which are identical or different, denote a hydrogen or halogen atom, an alkyl radical containing from 1 to 4 carbon atoms, an alkoxy radical containing from 1 to 4 carbon atoms, a carboxyl, sulpho or hydroxyalkyl radical containing from 1 to 4 carbon atoms,
R₈ and R₉, which are identical or different, denote a hydrogen atom or an alkyl, hydroxyalkyl, alkoxyalkyl, carbamylalkyl, mesylaminoalkyl, acetylaminoalkyl, ureidoalkyl, carbalkoxyaminoalkyl, piperidinoalkyl' or morpholinoalkyl radical, these alkyl or alkoxy groups containing from 1 to 4 carbon atoms, or else R₈ and R₉, together with the nitrogen atom to which they are linked, form a piperidino or morpholino heterocyclic ring, provided that R₅ or R₇ denotes a hydrogen atom when R₈ and R₉ do not denote a hydrogen atom, and the salts of these compounds.

5. Process according to Claim 4, characterized in that the compounds of formula (II) are chosen from p-phenylenediamine, 2-methyl-p-phenylenediamine, methoxy-para-phenylenediamine, chloro-para-phenylenediamine, 2,6-dimethyl-para-phenylenediamine, 2,5-dimethyl-para-phenylenediamine, 2,3-dimethyl-para-phenylenediamine, 2-methyl-5-methoxy-para-phenylenediamine, 2,6-dimethyl-5-methoxy-para-phenylenediamine, N,N-dimethyl-para-phenylenediamine, 3-methyl-4-amino-N,N-diethylaniline, N,N-di(β-hydroxyethyl)-para-phenylenediamine, 3-methyl-4-amino-N,N-di(β-hydroxyethyl)aniline, 3-chloro-4-amino-N,N-di(β-hydroxyethyl)aniline, 4-amino-N,N-(ethyl,carbamylmethyl)aniline, 3-methyl-4-amino-N,N-(ethyl,carbamylmethyl)aniline, 4-amino-N,N-(ethyl,β-piperidinoethyl)aniline, 3-methyl-4-amino-N,N-(ethyl,β-piperidinoethyl)aniline, 4-amino-N,N-(ethyl,β-morpholinoethyl)-aniline, 3-methyl-4-amino-N,N-(ethyl,β-morpholinoethyl)-aniline, 4-amino-N,N-(ethyl,β-acetylaminoethyl)aniline, 4-amino-N-(β-methoxyethyl)aniline, 3-methyl-4-amino-N,N-(ethyl,β-acetylaminoethyl)aniline, 4-amino-N,N-(ethyl,β-mesylaminoethyl)aniline, 3-methyl-4-amino-N,N-(ethyl,β-mesylaminoethyl)aniline, 4-amino-N,N-(ethyl,β-sulphoethyl)aniline, 3-methyl-4-amino-N,N-(ethyl,β-sulphoethyl)aniline, N-[(4'-amino)phenyl]morpholine, N-[(4'-amino)phenyl]piperidine, 2-hydroxyethyl-para-phenylenediamine, fluoro-para-phenylenediamine, carboxy-para-phenylenediamine, sulpho-para-phenylenediamine, 2-isopropyl-para-phenylenediamine, 2-n-propyl-para-phenylenediamine, 2-hydroxymethyl-para-phenylenediamine, N,N-dimethyl-3-methyl-para-phenylenediamine, N,N-(ethyl,β-hydroxyethyl)-para-phenylenediamine, N-(dihydroxypropyl)-para-phenylenediamine, N-4'-aminophenyl-p-phenylenediamine and N-phenyl-p-phenylenediamine, in the form of free base or salts.

6. Process according to Claim 3, characterized in that the p-aminophenols are chosen from p-aminophenol, 2-methyl-4-aminophenol, 3-methyl-4-aminophenol, 2-chloro-4-aminophenol, 3-chloro-4-aminophenol, 2,6-dimethyl-4-aminophenol, 3,5-dimethyl-4-aminophenol, 2,3-dimethyl-4-aminophenol, 2-hydroxymethyl-4-aminophenol, 2-(β-hydroxyethyl)-4-aminophenol, 2-methoxy-4-aminophenol, 3-methoxy-4-aminophenol, 2,5-dimethyl-4-aminophenol, 2-methoxymethyl-4-aminophenol and 2-β-hydroxyethoxy-methyl-4-aminophenol.

7. Process according to Claim 3, characterized in that the so-called double bases are chosen from bis-phenylalkylenedianines of formula: 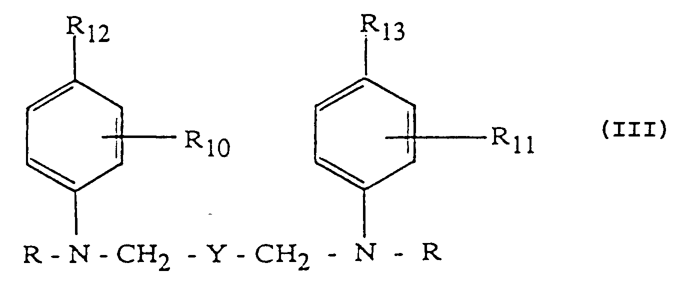 in which:
R₁₂ and R₁₃, which are identical or different, denote hydroxyl or NHR₁₄ groups, where R₁₄ denotes a hydrogen atom or a lower alkyl radical,
R₁₀ and R₁₁, which are identical or different, denote either hydrogen atoms or halogen atoms or alkyl groups,
R denotes a hydrogen atom or an alkyl, hydroxyalkyl or aminoalkyl group in which the amino residue can be substituted,
Y denotes a radical taken from the group consisting of the following radicals:
-(CH₂)ₙ-,(CH₂)ₙ,-O-(CH₂)_{n'}-,
(CH₂)_{n'}-CHOH-(CH₂)_{n'};-(CH₂)_{n'} 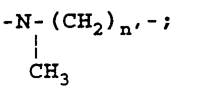 n being an integer between 0 and 8 and n' an integer between 0 and 4, and their addition salts with acids.

8. Process according to Claim 7, characterized in that the so-called double base is chosen from the following compounds: N,N'-bis(β-hydroxyethyl)-N,N'-bis(4'-aminophenyl)-1,3-diamino-2-propanol, N,N'-bis(β-hydroxyethyl)-N,N'-bis(4'-aminophenyl)ethylenediamine, N,N'-bis(4-aminophenyl)tetramethylenediamine, N,N'-bis(β-hydroxyethyl)-N,N'-bis(4-aminophenyl)tetramethylenediamine, N,N'-bis(4-methylaminophenyl)tetramethylenediamine and N,N'-bis(ethyl)-N,N'-bis(4'-amino-3'-methylphenyl)ethylenediamine.

9. Process according to Claim 1 or 2, characterized in that the oxidation dye precursors are oxidation dye precursors of ortho type which are chosen from ortho-aminophenols and ortho-phenylenediamines.

10. Process according to any one of Claims 1 to 9, characterized in that the oxidizing agent is chosen from hydrogen peroxide, urea peroxide, alkali metal bromates and persalts.

11. Process according to any one of Claims 1 to 10, characterized in that the pH of the composition applied to the keratinous fibres is between 3 and 6.9.

12. Process according-to any one of Claims 1 to 11, characterized in that the composition employed for dyeing keratinous fibres additionally contains heterocyclic couplers of the class of aminoindoles of formula (I), other couplers chosen from meta-diphenols, meta-aminophenols, meta-phenylenediamines, meta-N-acylaminophenols, meta-ureidophenols, meta-carbalkoxyaminophenols, α-naphthol, couplers containing an active methylene group and chosen from diketonic compounds and pyrazolones, heterocyclic couplers or 4-hydroxyindole, 6-hydroxyindole or 7-hydroxyindole.

13. Process according to Claim 12, characterized in that the couplers are chosen from 2,4-dihydroxyphenoxyethanol, 2,4-dihydroxyanisole, meta-aminophenol, resorcinol, resorcinol monomethyl ether, 2-methylresorcinol, pyrocatechol, 2-methyl-5-N-(β-hydroxyethyl)-aminophenol, 2-methyl-5-N-(β-mesylaminoethyl)aminophenol, 6-hydroxybenzomorpholine, 2,4-diaminoanisole, 2,4-diaminophenoxyethanol, 6-aminobenzomorpholine, [2-N-(β-hydroxyethyl)amino-4-amino]-phenoxyethanol, 2-amino-4-N-(β-hydroxyethyl)aminoanisole, (2,4-diamino)-phenyl-β,γ-dihydroxypropyl ether, 2,4-diaminophenoxyethylanine, 1,3-dimethoxy-2,4-diaminobenzene, 2-methyl-5-aminophenol, 2,6-dimethyl-3-aminophenol, 3,4-methylenedioxyphenol, 3,4-methylenedioxyaniline, 2-chlororesorcinol and their salts.

14. Process according to any one of Claims 1 to 13, characterized in that the composition contains anionic, cationic, nonionic or amphoteric surface-active agents or mixtures thereof, thickening agents, antioxidant agents and/or any other cosmetically acceptable adjuvant.

15. Process according to any one of Claims 1 to 14, characterized in that the suitable medium for dyeing consists of water or of a mixture of water and of a solvent chosen from C₂-C₄ lower alkanols, glycerol, glycols, glycol ethers, aromatic alcohols or their mixtures.

16. Agent for dyeing keratinous fibres and in particular hair, characterized in that it comprises at least two components: a component (A) consisting of a composition containing, in a suitable medium for dyeing, an aminoindole coupler corresponding to the formula (I) as defined in Claim 1 or 2 and an oxidation dye precursor as defined in any one of Claims 1 and 3 to 9, a component (B) consisting of a composition containing, in a suitable medium for dyeing, an oxidizing agent, the pH of the components (A) and (B) being such that after mixing in proportions of 90 to 10 % in the case of the component (A) and of 10 to 90 % in the case of the component (B), the resulting composition has a pH lower than 7.

17. Agent according to Claim 16, characterized in that the component (A) has a pH of between 3 and 10.5.

18. Agent according to Claim 16 or 17, characterized in that the component (A) contains oxidation dye precursors and couplers in proportions of between 0.05 and 7 % by weight relative to the total weight of the component (A).

19. Agent according to any one of Claims 16 to 18, characterized in that the concentration of compound of formula (I) is between 0.012 and 4 % by weight relative to the total weight of the component (A).

20. Agent according to any one of Claims 16 to 19, characterized in that the component (A) contains surface-active agents in proportions of 0.1 to 55 % by weight, solvents in addition to water in proportions of between 0.5 and 40 % by weight, thickening agents in proportions of between 0.1 and 5 % by weight, antioxidant agents in proportions of between 0.02 and 1.5 % by weight, and/or any other cosmetically acceptable adjuvant.

21. Agent according to any one of Claims 16 to 20, characterized in that the component (B) has a pH which has a minimum value of 1 and lower than 7.

22. Process for dyeing keratinous fibres and in particular hair, characterized in that it comprises a first stage consisting in storing a dyeing agent as defined in any one of Claims 16 to 21 and in mixing the components (A) and (B) before application, in proportions of 10 to 90 % in the case of the component (A) and of 90 to 10 % in the case of the component (B), so as to obtain a composition which has a pH lower than 7 and in applying this mixture to the keratinous fibres immediately after preparation.

23. Multicompartment device or dyeing kit, characterized in that it comprises at least two compartments, of which a first compartment contains the component (A) as defined in any one of Claims 16 to 20, and the second compartment contains the component (B) as defined in Claims 16 and 21.

24. Device according to Claim 23, characterized in that it is provided with a means permitting the desired mixture of the components (A) and (B) to be delivered onto the hair.

25. Dyeing process according to any one of Claims 1 to 15, characterized in that the composition is applied to the hair and is left in place for 3 to 40 minutes, that the hair is rinsed and that it is optionally shampooed before a new rinsing and drying.

26. Use as couplers of the aminoindole compounds corresponding to the formula (I) as defined in Claim 1 to 2 for dyeing keratinous fibres in acidic medium, in combination with oxidation dye precursors.

27. Composition for dyeing keratinous fibres, ready for use as used in the process according to any one of Claims 1 to 15.

28. Composition according to Claim 27, containing the compound of formula (I) in proportions of 0.01 to 3.5 % by weight relative to the total weight of the composition.

29. Dyeing composition for keratinous fibres, in particular hair, characterized in that it contains, in a suitable medium for dyeing, at least one oxidation dye precursor and a coupler corresponding to the following formula: 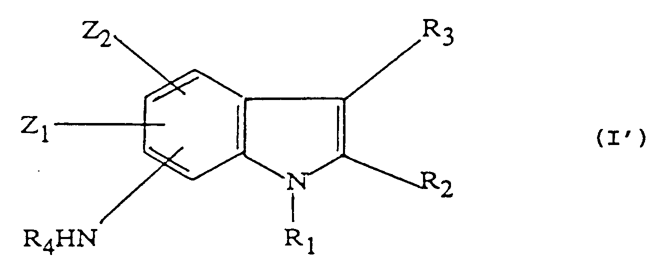 in which:
R₁ denotes hydrogen or a C₁-C₄ alkyl group,
R₂ and R₃, independently of each other, denote a hydrogen atom, a C₁-C₄ alkyl group or a COOR' group, R' being a C₁-C₄ alkyl radical, in which:
R₄ denotes a hydrogen atom, a C₁-C₄ alkyl radical, a C₁-C₄ hydroxyalkyl, C₂-C₄ polyhydroxyalkyl, acetyl or C₁-C₆ aminoalkyl group in which the amine may be optionally mono- or disubstituted by a C₁-C₄ alkyl,
Z₁ and Z₂, which are identical or different, denote a hydrogen atom or a C₁-C₄ alkyl, hydroxyl, a halogen or a C₁-C₄ alkoxy group,
provided that, when NHR₄ is in position 4, 6 or 7, Z₁ and Z₂ do not simultaneously denote a hydrogen atom; and the salts of these compounds, with the exception of 6-hydroxy-5-acetylamino-2,3-dimethylindole.

30. Dyeing agent according to any one of Claims 16 to 20, characterized in that the component (A) contains as coupler an aminoindole corresponding to the following formula: 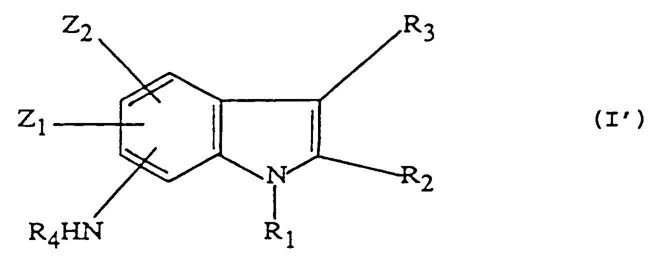 in which:
R₁ denotes hydrogen or a C₁-C₄ alkyl group,
R₂ and R₃, independently of each other, denote a hydrogen atom, a C₁-C₄ alkyl group or a COOR' group where R' denotes a hydrogen atom or a C₁-C₄ alkyl; at least one of the radicals R₂ and R₃ denotes a hydrogen atom,
R₄ denotes a C₁-C₄ alkyl, C₁-C₄ hydroxyalkyl or C₂-C₄ polyhydroxyalkyl group, the group -NHR₄ occupying positions 4, 6 or 7,
Z₁ and Z₂, which are identical or different, denote a hydrogen atom, a C₁-C₄-alkyl, a hydroxyl, a halogen atom or a C₁-C₄ alkoxy,
provided that, when NHR₄ is in position 4, 6 or 7, Z₁ and Z₂ do not simultaneously denote a hydrogen atom; and the salts of these compounds.

31. Process for the preparation of a compound corresponding to the following formula: 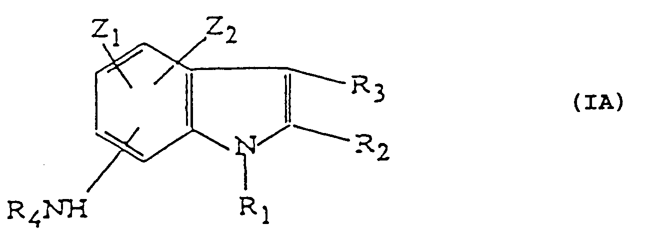 in which:
R₁ denotes hydrogen or a C₁-C₄ alkyl group,
R₂ and R₃, independently of each other, denote a hydrogen atom, a C₁-C₄ alkyl group or a COOR' group where R' denotes a hydrogen atom or a C₁-C₄ alkyl; at least one of the radicals R₂ and R₃ denotes a hydrogen atom,
R₄ denotes a C₁-C₄ alkyl, C₁-C₄ hydroxyalkyl or C₂-C₄ polyhydroxyalkyl group, the group -NHR₄ occupying positions 4, 6 or 7,
Z₁ and Z₂, which are identical or different, denote a hydrogen atom, a C₁-C₄ alkyl, a hydroxyl, a halogen atom or a C₁-C₄ alkoxy, at least one of the groups Z₁ and Z₂ is other than hydrogen,
and the salts of this compound,
characterized in that they are obtained by tosylation or formylation of a compound of formula: 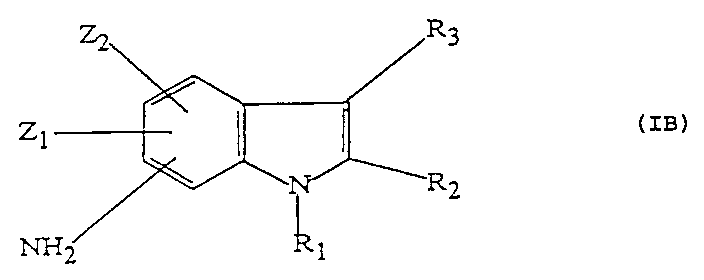 where R₁, R₂, R₃, Z₁ and Z₂ have the same meanings shown above, followed by an alkylation with an alkyl halide and then by a detosylation or detosylation according to the conventional methods.

32. Process for the preparation of the compounds 7-ethyl-4-aminoindole and 7-ethyl-6-aminoindole, characterized in that they are obtained by reduction of 7-ethyl-4-nitroindole or of 7-ethyl-6-nitroindole respectively.

## Patentansprüche (Patentansprüche für folgende(n) Vertragsstaat(en): AT, BE, CH, DE, DK, FR, GB, GR, IT, NL, SE)

1. Verfahren zur Färbung keratinischer Fasern, insbesondere menschlicher keratinischer Fasern wie der Haare,
dadurch **gekennzeichnet**, daß
man auf diese Fasern eine Zusammensetzung aufbringt, die in einem zur Färbung geeigneten Milieu mindestens einen Kuppler der Formel: 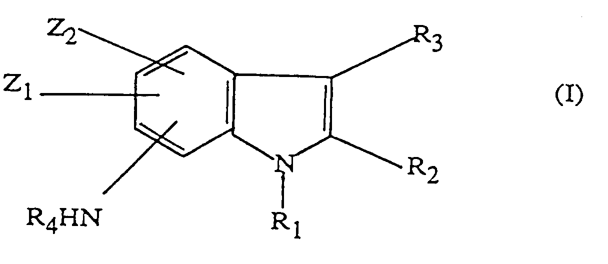 worin gilt:
R₁ bedeutet ein Wasserstoffatom oder eine C₁₋₄-Alkylgruppe;
R₂ und R₃ stellen, unabhängig voneinander, ein Wasserstoffatom, eine C₁₋₄-Alkyl- oder eine COOR'-Gruppe dar, wobei R' ein C₁₋₄-Alkylrest oder ein Wasserstoffatom ist;
R₄ stellt ein Wasserstoffatom, einen C₁₋₄-Alkylrest, eine Hydroxy-C₁₋₄-alkyl-, Polyhydroxy-C₂₋₄-alkyl-, Acetyl- oder eine Amino-C₁₋₆-alkylgruppe dar, wobei das Amin gegebenenfalls mit einem C₁₋₄-Alkylrest mono- oder disubstituiert sein kann;
Z₁ und Z₂ stellen, gleich oder verschieden, ein Wasserstoffatom oder eine C₁₋₄-Alkyl-, Hydroxy-, Halogen- oder eine C₁₋₄-Alkoxygruppe dar;
oder die Salze dieser Verbindungen,
- mindestens eine Oxidationsfarbstoff-Vorstufenverbindung und
- mindestens ein oxidierendes Mittel enthält,
wobei der pH-Wert der auf die Fasern aufgebrachten Zusammensetzung unterhalb 7 liegt.

2. Verfahren gemäß Anspruch 1,
dadurch **gekennzeichnet**, daß
die Verbindung der Formel (I) ausgewählt ist aus:
(1) 5-Aminoindol,
(2) 4-Aminoindol,
(3) 2-Methyl-6-aminoindol,
(4) 2,3,4-Trimethyl-6-aminoindol,
(5) 2,3,7-Trimethyl-6-aminoindol,
(6) 2,3,5-Trimethyl-6-aminoindol,
(7) 6-Aminoindol,
(8) 7-Aminoindol,
(9) 6-(β-Hydroxyethylamino)indol,
(10) 2-Methyl-5-hydroxy-6-aminoindol,
(11) 2,3-Dimethyl-5-amino-6-hydroxyindol,
(12) 2,3-Dimethyl-5-methoxy-6-aminoindol,
(13) 2,3-Dimethyl-5-hydroxy-6-aminoindol,
(14) 2,3-Dimethyl-5-ethyl-6-aminoindol,
(15) 2,3-Dimethyl-5-chlor-6-aminoindol,
(16) 2,3-Dimethyl-5-acetylamino-6-methoxyindol,
(17) 2,3-Dimethyl-5-amino-6-methoxyindol,
(18) N-Methyl-6-(β-hydroxyethyl)aminoindol,
(19) 2,3,4,5-Tetramethyl-6-aminoindol
(20) 6-N-Methylaminoindol,
(21) N-Methyl-5-aminoindol,
(22) N-Methyl-6-aminoindol,
(23) N-Methyl-4-aminoindol,
(24) 2,3-Dimethyl-4-aminoindol,
(25) 2,3-Dimethyl-7-aminoindol,
(26) 3-Methyl-6-aminoindol,
(27) 2-Methyl-3-ethyl-6-aminoindol,
(28) 2-Methyl-3-ethyl-7-aminoindol,
(29) 4-N-Methylaminoindol,
(30) 7-Ethyl-4-aminoindol,
(31) 7-Ethyl-6-aminoindol,
(32) 7-Ethyl-6-N-β-hydroxyethylaminoindol,
(33) 4-Methyl-6-aminoindol,
(34) 5-Methyl-6-aminoindol,
(35) 7-Methyl-4-aminoindol,
(36) 3-Methyl-7-ethyl-6-aminoindol,
(37) 5,7-Dimethyl-6-aminoindol,
(38) 5,7-Diethyl-6-aminoindol,
(39) 2-Ethoxycarbonyl-5-methyl-7-aminoindol,
(40) 2-Ethoxycarbonyl-5-chlor-7-aminoindol,
(41) 2-Ethoxycarbonyl-5-ethoxy-7-aminoindol,
(42) 2-Ethoxycarbonyl-5-methoxy-7-aminoindol,
(43) 5-Methoxy-7-(4'-dimethylamino-1'-methylbutyl)aminoindol,
(44) 5-Methoxy-7-(4'-dimethylaminobutyl)aminoindol,
(45) 5-Methoxy-7-(4'-diethylamino-1'-methylbutyl)aminoindol,
(46) 5-Fluor-6-aminoindol,
(47) 5-Fluor-1-sec-butyl-6-aminoindol,
(48) 5-Fluor-1-n-propyl-6-aminoindol,
(49) 1-Methyl-2-methoxycarbonyl-5-methoxy-6-aminoindol,
(50) 2-Methoxycarbonyl-5-methoxy-6-aminoindol,
(51) 2-Ethoxycarbonyl-5-methoxy-6-aminoindol,
(52) 2-Carboxy-5-methoxy-6-aminoindol,
(53) 1,2-Dimethyl-5-hydroxy-6-aminoindol,
(54) 2-Methoxycarbonyl-4-methoxy-6-aminoindol
und aus den Salzen dieser Verbindungen.

3. Verfahren gemäß Anspruch 1 oder 2,
dadurch **gekennzeichnet**, daß
die Oxidationsfarbstoff-Vorstufenverbindungen aus p-Phenylendiaminen, p-Aminophenolen, heterozyklischen Vorstufenverbindungen vom para-Typ und aus Doppelbasen ausgewählt sind.

4. Verfahren gemäß Anspruch 3,
dadurch **gekennzeichnet**, daß
die p-Phenylendiamine aus Verbindungen der Formel: 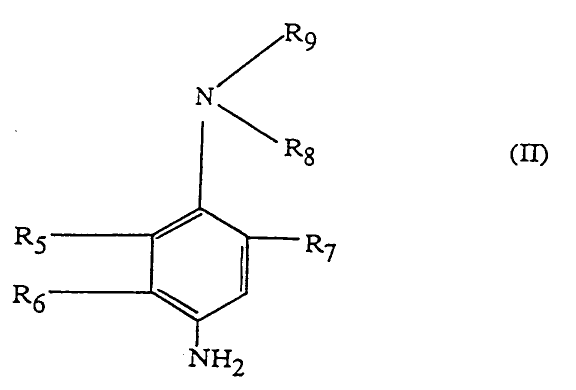 worin gilt:
R₅, R₆ und R₇ stellen, gleich oder verschieden, ein Wasserstoff- oder Halogenatom, einen Alkylrest mit 1 bis 4 Kohlenstoffatomen, einen Alkoxyrest mit 1 bis 4 Kohlenstoffatomen, einen Carboxy-, Sulfo- oder Hydroxyalkylrest mit 1 bis 4 Kohlenstoffatomen dar;
R₈ und R₉ stellen, gleich oder verschieden, ein Wasserstoffatom, einen Alkyl-, Hydroxyalkyl-, Alkoxyalkyl-, Carbamylalkyl-, Mesylaminoalkyl-, Acetylaminoalkyl-, Ureidoalkyl-, Carbalkoxyaminoalkyl-, Piperidinoalkyl- oder einen Morpholinoalkylrest dar, wobei diese Alkyl- oder Alkoxygruppen 1 bis 4 Kohlenstoffatome aufweisen, oder R₈ und R₉ bilden, zusammen mit dem Stickstoffatom, an das sie gebunden sind, auch einen Piperidino- oder Morpholino-Heterozyklus, mit der Maßgabe, daß R₅ oder R₇ ein Wasserstoffatom darstellen, wenn R₈ und R₉ kein Wasserstoffatom darstellen,
sowie aus den Salzen dieser Verbindungen ausgewählt sind.

5. Verfahren gemäß Anspruch 4,
dadurch **gekennzeichnet**, daß
die Verbindungen der Formel (II) aus p-Phenylendiamin, 2-Methyl-p-phenylendiamin, Methoxy-p-phenylendiamin, Chlor-p-phenylendiamin, 2,6-Dimethyl-p-phenylendiamin, 2,5-Dimethyl-p-phenylendiamin, 2,3-Dimethyl-p-phenylendiamin, 2-Methyl-5-methoxy-p-phenylendiamin, 2,6-Dimethyl-5-methoxy-p-phenylendiamin, N,N-Dimethyl-p-phenylendiamin, 3-Methyl-4-amino-N,N-diethylanilin, N,N-Di(β-hydroxyethyl)-p-phenylendiamin, 3-Methyl-4-amino-N,N-di(β-hydroxyethyl)anilin, 3-Chlor-4-amino-N,N-di(β-hydroxyethyl)anilin, 4-Amino-N,N-(ethyl, carbamylmethyl)anilin, 3-Methyl-4-amino-N,N-(ethyl,carbamylmethyl)anilin, 4-Amino-N,N-(ethyl,β-piperidinoethyl)anilin, 3-Methyl-4-amino-N,N-(ethyl,β-piperidinoethyl)anilin, 4-Amino-N,N-(ethyl, β-morpholinoethyl)anilin, 3-Methyl-4-amino,N,N-(ethyl, β-morpholinoethyl)anilin, 4-Amino-N,N-(ethyl, β-acetylaminoethyl)anilin, 4-Amino-N-(β-methoxyethyl)anilin, 3-Methyl-4-amino-N,N-(ethyl,β-acetylaminoethyl)anilin, 4-Amino-N,N-(ethyl,β-mesylaminoethyl)anilin, 3-Methyl-4-amino-N,N-(ethyl,β-mesylaminoethyl)anilin, 4-Amino-N,N-(ethyl,β-sulfoethyl)anilin, 3-Methyl-4-amino-N,N-(ethyl,β-sulfoethyl)anilin, N-((4'-Amino)phenyl)morpholin, N-((4'-Amino)phenyl)piperidin, 2-Hydroxyethyl-p-phenylendiamin, Fluor-p-phenylendiamin, Carboxy-p-phenylendiamin, Sulfo-p-phenylendiamin, 2-Isopropyl-p-phenylendiamin, 2-n-Propyl-p-phenylendiamin, 2-Hydroxmethyl-p-phenylendiamin, N,N-Dimethyl-3-methyl-p-phenylendiamin, N,N-(Ethyl,β-hydroxyethyl)-p-phenylendiamin, N-(Dihydroxypropyl)-p-phenylendiamin, N-4'-Aminophenyl-p-phenylendiamin, N-Phenyl-p-phenylendiamin in Form einer freien Base oder von Salzen ausgewählt sind.

6. Verfahren gemäß Anspruch 3,
dadurch **gekennzeichnet**, daß
die p-Aminophenole aus p-Aminophenol, 2-Methyl-4-aminophenol, 3-Methyl-4-aminophenol, 2-Chlor-4-aminophenol, 3-Chlor-4-aminophenol, 2,6-Dimethyl-4-aminophenol, 3,5-Dimethyl-4-aminophenol, 2,3-Dimethyl-4-aminophenol, 2-Hydroxymethyl-4-aminophenol, 2-(β-Hydroxyethyl)-4-aminophenol, 2-Methoxy-4-aminophenol, 3-Methoxy-4-aminophenol, 2,5-Dimethyl-4-aminophenol, 2-Methoxymethyl-4-aminophenol und aus 2-β-Hydroxyethoxymethyl-4-aminophenol ausgewählt sind.

7. Verfahren gemäß Anspruch 3,
dadurch **gekennzeichnet**, daß
die als doppelte bezeichneten Basen aus Bisphenylalkylendiaminen der Formel: 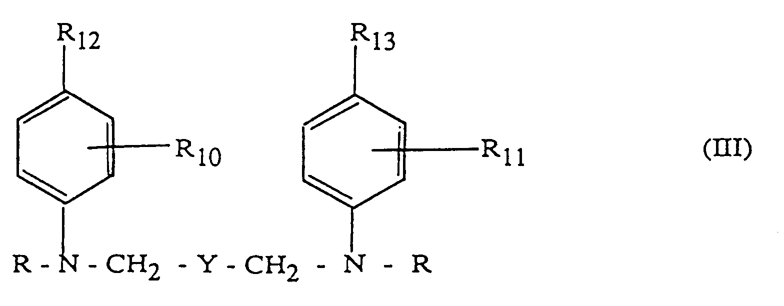 worin gilt:
R₁₂ und R₁₃ stellen, gleich oder verschieden, Hydroxyl- oder NHR₁₄-Gruppen dar, worin R₁₄ ein Wasserstoffatom oder eine Niedrigalkylrest bedeutet;
R₁₀ und R₁₁ stellen, gelich oder verschieden, entweder Wasserstoffatome oder Halogenatome oder auch Alkylgruppen dar;
R stellt ein Wasserstoffatom, eine Alkyl-, Hydroxyalkyl- oder Aminoalkylgruppe dar, deren Aminorest substituiert sein kann;
Y stellt einen Rest aus der Gruppe aus den folgenden Resten dar:
-(CH₂)ₙ-, -(CH₂)_{n'}-O-(CH₂)_{n'}-,
- (CH₂)_{n'}-CHOH-(CH₂)_{n'},-(CH₂)_{n'}-N-CH₃-(CH₂)_{n'}-,
worin n eine ganze Zahl von 0 bis 8 und n' eine ganze Zahl von 0 bis 4 sind,
sowie aus deren Säureadditionssalzen ausgewählt sind.

8. Verfahren gemäß Anspruch 7,
dadurch **gekennzeichnet**, daß
die als doppelte bezeichnete Base aus den folgenden Verbindungen ausgewählt ist: N,N'-Bis(β-hydroxyethyl)-N,N'-bis(4'-aminophenyl)-1,3-diaminopropan-2-ol, N,N'-Bis(β-hydroxyethyl)-N,N'-bis(4'-aminophenyl)ethylendiamin, N,N'-Bis(4-aminophenyl)tetramethylendiamin, N,N'-Bis(β-hydroxyethyl)-N,N'-bis(4-aminophenyl)tetramethylendiamin, N,N'-Bis(4-methylaminophenyl)tetramethylendiamin und aus N,N'-Bis(ethyl)-N,N'-bis(4'-amino-3'-methylphenyl)ethylendiamin.

9. Verfahren gemäß Anspruch 1 oder 2,
dadurch **gekennzeichnet**, daß
die Oxidationsfarbstoff-Vorstufenverbindungen Oxidationsfarbstoff-Vorstufenverbindungen vom ortho-Typ sind, ausgewählt aus o-Aminophenolen und o-Phenylendiaminen.

10. Verfahren gemäß jedem der Ansprüche 1 bis 9,
dadurch **gekennzeichnet**, daß
das oxidierende Mittel aus Wasserstoffperoxid, Harnstoffperoxid, Alkalimetallbromaten und aus Persalzen ausgewählt ist.

11. Verfahren gemäß jedem der Ansprüche 1 bis 10,
dadurch **gekennzeichnet**, daß
der pH-Wert der auf die keratinischen Fasern aufgetragenen Zusammensetzung 3 bis 6,9 beträgt.

12. Verfahren gemäß jedem der Ansprüche 1 bis 11,
dadurch **gekennzeichnet**, daß
die zur Färbung der keratinischen Fasern eingesetzte Zusammensetzung zusätzlich zu den heterozyklischen Kupplern der Familie der Aminoindole der Formel (I) weitere Kuppler enthält, die aus m-Diphenolen, m-Aminophenolen, m-Phenylendiaminen, m-N-Acylaminophenolen, m-Ureidophenolen, m-Carbalkoxyaminophenolen, α-Naphthol, aus Kupplern mit einer aktiven Methylengruppe, ausgewählt aus Diketoverbindungen und Pyrazolonen, aus heterozyklischen Kupplern oder aus 4-Hydroxyindol, 6-Hydroxyindol oder 7-Hydroxyindol ausgewählt sind.

13. Verfahren gemäß Anspruch 12,
dadurch **gekennzeichnet**, daß
die Kuppler aus 2,4-Dihydroxyphenoxyethanol, 2,4-Dihydroxyanisol, m-Aminophenol, Resorcin, den Monomethylether von Resorcin, 2-Methylresorcin, Pyrocatechol, 2-Methyl-5-N-(β -hydroxyethyl)aminophenol, 2-Methyl-5-N-(β-mesylaminoethyl)aminophenol, 6-Hydroxybenzomorpholin, 2,4-Diaminoanisol, 2,4-Diaminophenoxyethanol, 6-Aminobenzomorpholin, (2-N-(β-Hydroxyethyl)amino-4-amino)-phenoxyethanol, 2-Amino-4-N-(β-hydroxyethyl)aminoanisol, (2,4-Diamino)phenyl-β,γ-dihydroxypropylether, 2,4-Diaminophenoxyethylamin, 1,3-Dimethoxy-2,4-diaminobenzol, 2-Methyl-5-aminophenol, 2,6-Dimethyl-3-aminophenol, 3,4-Methylendioxyphenol, 3,4-Methylendioxyanilin, 2-Chlorresorcin und deren Salzen ausgewählt sind.

14. Verfahren gemäß jedem der Ansprüche 1 bis 13,
dadurch **gekennzeichnet**, daß
die Zusammensetzung anionische, kationische, nicht-ionische oder amphotere oberflächenaktive Mittel oder deren Mischungen, Verdickungsmittel, Antioxidantien und/oder jeden weiteren kosmetisch geeigneten Hilfsstoff enthält.

15. Verfahren gemäß jedem der Ansprüche 1 bis 14,
dadurch **gekennzeichnet**, daß
das zur Färbung geeignete Milieu aus Wasser oder aus einer Mischung aus Wasser und einem Lösungsmittel zusammengesetzt ist, welches aus C₂₋₄-Niedrigalkanolen, Glycerin, Glycolen, Ethern von Glycolen, aromatischen Alkoholen oder aus deren Mischungen ausgewählt ist.

16. Mittel zur Färbung keratinischer Fasern und insbesondere der Haare,
dadurch **gekennzeichnet**, daß
es mindestens zwei Bestandteile umfaßt: einen Bestandteil (A) aus einer Zusammensetzung, die in einem zur Färbung geeigneten Milieu einen Aminoindol-Kuppler der in Anspruch 1 oder 2 definierten Formel (I) und eine in jedem der Ansprüche 1 und 3 bis 9 definierte Oxidationsfarbstoff-Vorstufenverbindung enthält, sowie einen Bestandteil (B) aus einer Zusammensetzung, die in einem zur Färbung geeigneten Milieu ein oxidierendes Mittel enthält, wobei der pH-Wert der Bestandteile (A) und (B) so eingestellt ist, daß nach Mischung in Mengenanteilen von 90 bis 10% für den Bestandteil (A) und von 10 bis 90% für den Bestandteil (B) die sich ergebende Zusammensetzung einen pH-Wert von weniger als 7 aufweist.

17. Mittel gemäß Anspruch 16,
dadurch **gekennzeichnet**, daß
der Bestandteil (A) einen pH-Wert von 3 bis 10,5 hat.

18. Mittel gemäß Anspruch 16 oder 17,
dadurch **gekennzeichnet**, daß
der Bestandteil (A) die Oxidationsfarbstoff-Vorstufenverbindungen sowie die Kuppler in Mengenanteilen von 0,05 bis 7 Gew.% enthält, bezogen auf das Gesamtgewicht des Bestandteils (A).

19. Mittel gemäß jedem der Ansprüche 16 bis 18,
dadurch **gekennzeichnet**, daß
die Konzentration an Verbindung der Formel (I) 0,012 bis 4 Gew.% ausmacht, bezogen auf das Gesamtgewicht des Bestandteils (A).

20. Mittel gemäß jedem der Ansprüche 16 bis 19,
dadurch **gekennzeichnet**, daß
der Bestandteil (A) oberflächenaktive Mittel in Mengenanteilen von 0,1 bis 55 Gew.%, Lösungsmittel zusätzlich zum Wasser in Mengenanteilen von 0,5 bis 40 Gew.%, Verdickungsmittel in Mengenanteilen von 0,1 bis 5 Gew.%, Antioxidantien in Mengenanteilen von 0,02 bis 1,5 Gew.% und/oder jeden weiteren kosmetisch geeigneten Hilfsstoff enthält.

21. Mittel gemäß jedem der Anspüche 16 bis 20,
dadurch **gekennzeichnet**, daß
der Bestandteil (B) einen pH-Wert in einem Bereich von mindestens 1 bis unterhalb 7 aufweist.

22. Verfahren zur Färbung keratinischer Fasern und insbesondere der Haare,
dadurch **gekennzeichnet**, daß
es eine erste Stufe umfaßt, wobei man ein in jedem der Ansprüche 16 bis 21 definiertes Färbemittel aufbewahrt und vorhält, und wobei man vor der Aufbringung die Bestandteile (A) und (B) in Mengenanteilen von 10 bis 90% für den Bestandteil (A) und von 90 bis 10% für den Bestandteil (B) vermischt, und zwar so, daß man eine Zusammensetzung erhält, die einen pH-Wert von weniger als 7 aufweist, und wobei man diese Mischung unmittelbar nach ihrer Zubereitung auf die keratinischen Fasern aufbringt.

23. Vorrichtung aus mehreren Teilen oder Kit zur Färbung,
dadurch **gekennzeichnet**, daß
sie mindestens zwei Teile umfassen, wovon ein erster Teil den in jedem der Ansprüche 16 bis 20 definierten Bestandteil (A) und der zweite Teil den in jedem der Ansprüche 16 bis 21 definierten Bestandteil (B) einschließen.

24. Vorrichtung gemäß Anspruch 23,
dadurch **gekennzeichnet**, daß
sie mit einem Element ausgerüstet ist, welches es ermöglicht, die erwünschte Mischung aus den Bestandteilen (A) und (B) freizusetzen und auf die Haare aufzubringen.

25. Verfahren gemäß jedem der Ansprüche 1 bis 15,
dadurch **gekennzeichnet**, daß
man auf die Haare die Zusammensetzung aufbringt und sie 3 bis 40 Minuten lang verweilen läßt, die Haare spült und gegebenenfalls eine Schamponierung vor einer erneuten Spülung durchführt und die Haare dann trocknet.

26. Verwendung der Aminoindol-Verbindungen der in Anspruch 1 oder 2 definierten Formel (I) als Kuppler zur Färbung keratinischer Fasern in saurem Milieu, zusammen mit Oxidationsfarbstoff-Vorstufenverbindungen.

27. Gebrauchsfertige Zusammensetzung zur Färbung keratinischer Fasern zur Anwendung in dem Verfahren gemäß jedem der Ansprüche 1 bis 15.

28. Zusammensetzung gemäß Anspruch 27,
welche die Verbindung der Formel (I) in Mengenanteilen von 0,01 bis 3,5 Gew.% enthält, bezogen auf das Gesamtgewicht der Zusammensetzung.

29. Färbezusammensetzung für keratinische Fasern, insbesondere für Haare,
dadurch **gekennzeichnet**, daß
sie in einem zur Färbung geeigneten Milieu mindestens eine Oxidationsfarbstoff-Vorstufenverbindung oder einen Kuppler der folgenden Formel: 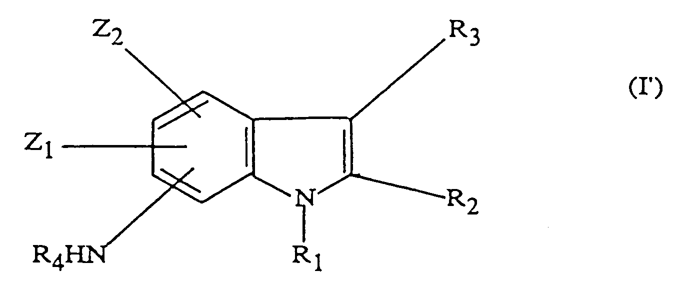 worin gilt:
R₁ bedeutet ein Wasserstoffatom oder eine C₁₋₄-Alkylgruppe;
R₂ und R₃ stellen, unabhängig voneinander, ein Wasserstoffatom, eine C₁₋₄-Alkyl- oder eine COOR'-Gruppe dar, wobei R' ein C₁₋₄-Alkylrest oder ein Wasserstoffatom ist;
R₄ stellt ein Wasserstoffatom, einen C₁₋₄-Alkylrest, eine Hydroxy-C₁₋₄-alkyl-, Polyhydroxy-C₂₋₄-alkyl-, Acetyl- oder eine Amino-C₁₋₆-alkylgruppe dar, wobei das Amin gegebenenfalls mit einem C₁₋₄-Alkylrest mono- oder disubstituiert sein kann;
Z₁ und Z₂ stellen, gleich oder verschieden, ein Wasserstoffatom oder eine C₁₋₄-Alkyl-, Hydroxy-, Halogen- oder eine C₁₋₄-Alkoxygruppe dar; mit der Maßgabe, daß, wenn NHR₄ in Position 4, 6 oder 7 vorliegt, Z₁ und Z₂ nicht gleichzeitig ein Wasserstoffatom bedeuten, sowie die Salze dieser Verbndungen enthält, ausgenommen 6-Hydroxy-5-acetylamino-2,3-dimethylindol.

30. Färbemittel gemäß jedem der Ansprüche 16 bis 20,
dadurch **gekennzeichnet**, daß
der Bestandteil (A) als Kuppler ein Aminoindol der folgenden Formel: 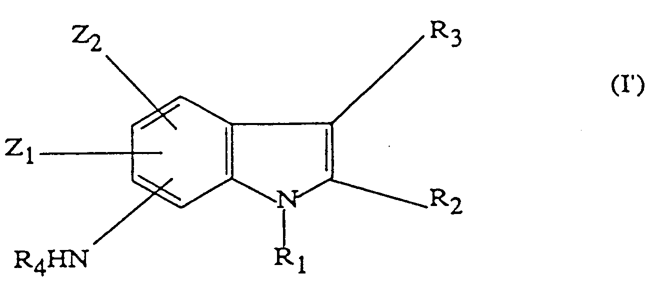 worin gilt:
R₁ bedeutet ein Wasserstoffatom oder eine C₁₋₄-Alkylgruppe;
R₂ und R₃ stellen, unabhängig voneinander, ein Wasserstoffatom, eine C₁₋₄-Alkyl- oder eine COOR'-Gruppe dar, wobei R' ein C₁₋₄-Alkylrest oder ein Wasserstoffatom ist; wobei mindestens einer der Reste R₂ und R₃ ein Wasserstoffatom bedeutet;
R₄ stellt eine C₁₋₄-Alkyl-, Hydroxy-C₁₋₄-alkyl- oder eine Polyhydroxy-C₂₋₄-alkylgruppe dar, wobei die -NHR₄-Gruppe die Positionen 4, 6 oder 7 besetzt;
Z₁ und Z₂ stellen, gleich oder verschieden, ein Wasserstoffatom, einen C₁₋₄-Alkylrest, einen Hydroxyrest, ein Halogenatom oder eine C₁₋₄-Alkoxygruppe dar;
mit der Maßgabe, daß, wenn NHR₄ in Position 4, 6 oder 7 vorliegt, Z₁ und Z₂ nicht gleichzeitig ein Wasserstoffatom bedeuten, sowie die Salze dieser Verbindungen enthält.

31. Verbindung der folgenden Formel: 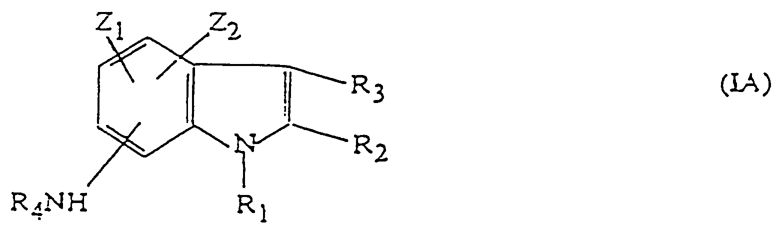 worin gilt:
R₁ bedeutet ein Wasserstoffatom oder eine C₁₋₄-Alkylgruppe;
R₂ und R₃ stellen, unabhängig voneinander, ein Wasserstoffatom, eine C₁₋₄-Alkykl- oder COOR'-Gruppe dar, worin R' ein Wasserstoffatom oder einen C₁₋₄-Alkylrest bedeutet, wobei mindestens einer der Reste R₂ und R₃ ein Wasserstoffatom bedeutet;
R₄ stellt eine C₁₋₄-Alkyl-, Hydroxy-C₁₋₄-alkyl- oder Polyhydroxy-C₂₋₄-alkylgruppe dar, wobei die -NHR₄-Gruppe die Positionen 4, 6 oder 7 besetzt;
Z₁ und Z₂ stellen, gleich oder verschieden, ein Wasserstoffatom, einen C₁₋₄-Alkylrest, einen Hydroxyrest, ein Halogenatom oder eine C₁₋₄-Alkoxygruppe dar, wobei mindestens eine der Gruppierungen Z₁ und Z₂ von Wasserstoff verschieden ist,
sowie die Salze dieser Verbindungen.

32. Verbindung, ausgewählt aus:
7-Ethyl-4-aminoindol,
7-Ethyl-6-aminoindol
sowie aus den Salzen dieser Verbindungen.

## Patentansprüche (Patentansprüche für folgende(n) Vertragsstaat(en): ES)

1. Verfahren zur Färbung keratinischer Fasern, insbesondere menschlicher keratinischer Fasern wie der Haare,
dadurch **gekennzeichnet**, daß
man auf diese Fasern eine Zusammensetzung aufbringt, die in einem zur Färbung geeigneten Milieu mindestens einen Kuppler der Formel: 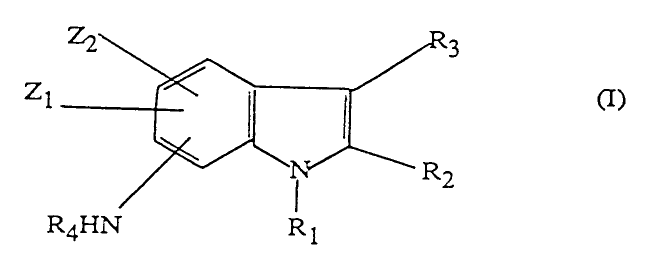 worin gilt:
R₁ bedeutet ein Wasserstoffatom oder eine C₁₋₄-Alkylgruppe;
R₂ und R₃ stellen, unabhängig voneinander, ein Wasserstoffatom, eine C₁₋₄-Alkyl- oder eine COOR'-Gruppe dar, wobei R' ein C₁₋₄-Alkylrest oder ein Wasserstoffatom ist;
R₄ stellt ein Wasserstoffatom, einen C₁₋₄-Alkylrest, eine Hydroxy-C₁₋₄-alkyl-, Polyhydroxy-C₂₋₄-alkyl-, Acetyl- oder eine Amino-C₁₋₆-alkylgruppe dar, wobei das Amin gegebenenfalls mit einem C₁₋₄-Alkylrest mono- oder disubstituiert sein kann;
Z₁ und Z₂ stellen, gleich oder verschieden, ein Wasserstoffatom oder eine C₁₋₄-Alkyl-, Hydroxy-, Halogen- oder eine C₁₋₄-Alkoxygruppe dar;
oder die Salze dieser Verbindungen,
- mindestens eine Oxidationsfarbstoff-Vorstufenverbindung und
- mindestens ein oxidierendes Mittel enthält,
wobei der pH-Wert der auf die Fasern aufgebrachten Zusammensetzung unterhalb 7 liegt.

2. Verfahren gemäß Anspruch 1,
dadurch **gekennzeichnet**, daß
die Verbindung der Formel (I) ausgewählt ist aus:
(1) 5-Aminoindol,
(2) 4-Aminoindol,
(3) 2-Methyl-6-aminoindol,
(4) 2,3,4-Trimethyl-6-aminoindol,
(5) 2,3,7-Trimethyl-6-aminoindol,
(6) 2,3,5-Trimethyl-6-aminoindol,
(7) 6-Aminoindol,
(8) 7-Aminoindol,
(9) 6-(β-Hydroxyethylamino)indol,
(10) 2-Methyl-5-hydroxy-6-aminoindol,
(11) 2,3-Dimethyl-5-amino-6-hydroxyindol,
(12) 2,3-Dimethyl-5-methoxy-6-aminoindol,
(13) 2,3-Dimethyl-5-hydroxy-6-aminoindol,
(14) 2,3-Dimethyl-5-ethyl-6-aminoindol,
(15) 2,3-Dimethyl-5-chlor-6-aminoindol,
(16) 2,3-Dimethyl-5-acetylamino-6-methoxyindol,
(17) 2,3-Dimethyl-5-amino-6-methoxyindol,
(18) N-Methyl-6-(β-hydroxyethyl)aminoindol,
(19) 2,3,4,5-Tetramethyl-6-aminoindol,
(20) 6-N-Methylaminoindol,
(21) N-Methyl-5-aminoindol,
(22) N-Methyl-6-aminoindol,
(23) N-Methyl-4-aminoindol,
(24) 2,3-Dimethyl-4-aminoindol,
(25) 2,3-Dimethyl-7-aminoindol,
(26) 3-Methyl-6-aminoindol,
(27) 2-Methyl-3-ethyl-6-aminoindol,
(28) 2-Methyl-3-ethyl-7-aminoindol,
(29) 4-N-Methylaminoindol,
(30) 7-Ethyl-4-aminoindol,
(31) 7-Ethyl-6-aminoindol,
(32) 7-Ethyl-6-N-β-hydroxyethylaminoindol,
(33) 4-Methyl-6-aminoindol,
(34) 5-Methyl-6-aminoindol,
(35) 7-Methyl-4-aminoindol,
(36) 3-Methyl-7-ethyl-6-aminoindol,
(37) 5,7-Dimethyl-6-aminoindol
(38) 5,7-Diethyl-6-aminoindol,
(39) 2-Ethoxycarbonyl-5-methyl-7-aminoindol,
(40) 2-Ethoxycarbonyl-5-chlor-7-aminoindol,
(41) 2-Ethoxycarbonyl-5-ethoxy-7-aminoindol,
(42) 2-Ethoxycarbonyl-5-methoxy-7-aminoindol,
(43) 5-Methoxy-7-(4'-dimethylamino-1'-methylbutyl)aminoindol,
(44) 5-Methoxy-7-(4'-dimethylaminobutyl)aminoindol,
(45) 5-Methoxy-7-(4'-diethylamino-1'-methylbutyl)aminoindol,
(46) 5-Fluor-6-aminoindol,
(47) 5-Fluor-1-sec-butyl-6-aminoindol,
(48) 5-Fluor-1-n-propyl-6-aminoindol,
(49) 1-Methyl-2-methoxycarbonyl-5-methoxy-6-aminoindol,
(50) 2-Methoxycarbonyl-5-methoxy-6-aminoindol,
(51) 2-Ethoxycarbonyl-5-methoxy-6-aminoindol,
(52) 2-Carboxy-5-methoxy-6-aminoindol,
(53) 1,2-Dimethyl-5-hydroxy-6-aminoindol,
(54) 2-Methoxycarbonyl-4-methoxy-6-aminoindol
und aus den Salzen dieser Verbindungen.

3. Verfahren gemäß Anspruch 1 oder 2,
dadurch **gekennzeichnet**, daß
die Oxidationsfarbstoff-Vorstufenverbindungen aus p-Phenylendiaminen, p-Aminophenolen, heterozyklischen Vorstufenverbindungen vom para-Typ und aus Doppelbasen ausgewählt sind.

4. Verfahren gemäß Anspruch 3,
dadurch **gekennzeichnet**, daß
die p-Phenylendiamine aus Verbindungen der Formel: 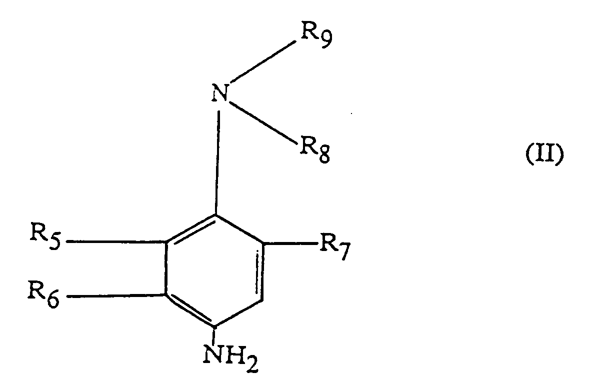 worin gilt:
R₅, R₆ und R₇ stellen, gleich oder verschieden, ein Wasserstoff- oder Halogenatom, einen Alkylrest mit 1 bis 4 Kohlenstoffatomen, einen Alkoxyrest mit 1 bis 4 Kohlenstoffatomen, einen Carboxy-, Sulfo- oder Hydroxyalkylrest mit 1 bis 4 Kohlenstoffatomen dar;
R₈ und R₉ stellen, gleich oder verschieden, ein Wasserstoffatom, einen Alkyl-, Hydroxyalkyl-, Alkoxyalkyl-, Carbamylalkyl-, Mesylaminoalkyl-, Acetylaminoalkyl-, Ureidoalkyl-, Carbalkoxyaminoalkyl-, Piperidinoalkyl- oder einen Morpholinoalkylrest dar, wobei diese Alkyl- oder Alkoxygruppen 1 bis 4 Kohlenstoffatome aufweisen, oder R₈ und R₉ bilden, zusammen mit dem Stickstoffatom, an das sie gebunden sind, auch einen Piperidino- oder Morpholino-Heterozyklus, mit der Maßgabe, daß R₅ oder R₇ ein Wasserstoffatom darstellen, wenn R₈ und R₉ kein Wasserstoffatom darstellen,
sowie aus den Salzen dieser Verbindungen ausgewählt sind.

5. Verfahren gemäß Anspruch 4,
dadurch **gekennzeichnet**, daß
die Verbindungen der Formel (II) aus p-Phenylendiamin, 2-Methyl-p-phenylendiamin, Methoxy-p-phenylendiamin, Chlor-p-phenylendiamin, 2,6-Dimethyl-p-phenylendiamin, 2,5-Dimethyl-p-phenylendiamin, 2,3-Dimethyl-p-phenylendiamin, 2-Methyl-5-methoxy-p-phenylendiamin, 2,6-Dimethyl-5-methoxy-p-phenylendiamin, N,N-Dimethyl-p-phenylendiamin, 3-Methyl-4-amino-N,N-diethylanilin, N,N-Di(β-hydroxyethyl)-p-phenylendiamin, 3-Methyl-4-amino-N,N-di(β-hydroxyethyl)anilin, 3-Chlor-4-amino-N,N-di(β-hydroxyethyl)anilin, 4-Amino-N,N-(ethyl, carbamylmethyl)anilin, 3-Methyl-4-amino-N,N-(ethyl,carbamylmethyl)anilin, 4-Amino-N,N-(ethyl,β-piperidinoethyl)anilin, 3-Methyl-4-amino-N,N-(ethyl,β-piperidinoethyl)anilin, 4-Amino-N,N-(ethyl, β-morpholinoethyl)anilin, 3-Methyl-4-amino,N,N-(ethyl, β-morpholinoethyl)anilin, 4-Amino-N,N-(ethyl, β-acetylaminoethyl)anilin, 4-Amino-N-(β-methoxyethyl)anilin, 3-Methyl-4-amino-N,N-(ethyl,β-acetylaminoethyl)anilin, 4-Amino-N,N-(ethyl,β-mesylaminoethyl)anilin, 3-Methyl-4-amino-N,N-(ethyl,β-mesylaminoethyl)anilin, 4-Amino-N,N-(ethyl,β-sulfoethyl)anilin, 3-Methyl-4-amino-N,N-(ethyl,β-sulfoethyl)anilin, N-((4'-Amino)phenyl)morpholin, N-((4'-Amino)phenyl)piperidin, 2-Hydroxyethyl-p-phenylendiamin, Fluor-p-phenylendiamin, Carboxy-p-phenylendiamin, Sulfo-p-phenylendiamin, 2-Isopropyl-p-phenylendiamin, 2-n-Propyl-p-phenylendiamin, 2-Hydroxymethyl-p-phenylendiamin, N,N-Dimethyl-3-methyl-p-phenylendiamin, N,N-(Ethyl,β-hydroxyethyl)-p-phenylendiamin, N-(Dihydroxypropyl)-p-phenylendiamin, N-4'-Aminophenyl-p-phenylendiamin, N-Phenyl-p-phenylendiamin in Form einer freien Base oder von Salzen ausgewählt sind.

6. Verfahren gemäß Anspruch 3,
dadurch **gekennzeichnet**, daß
die p-Aminophenole aus p-Aminophenol, 2-Methyl-4-aminophenol, 3-Methyl-4-aminophenol, 2-Chlor-4-aminophenol, 3-Chlor-4-aminophenol, 2,6-Dimethyl-4-aminophenol, 3,5-Dimethyl-4-aminophenol, 2,3-Dimethyl-4-aminophenol, 2-Hydroxymethyl-4-aminophenol, 2-(β-Hydroxyethyl)-4-aminophenol, 2-Methoxy-4-aminophenol, 3-Methoxy-4-aminophenol, 2,5-Dimethyl-4-aminophenol, 2-Methoxymethyl-4-aminophenol und aus 2-β-Hydroxyethoxymethyl-4-aminophenol ausgewählt sind.

7. Verfahren gemäß Anspruch 3,
dadurch **gekennzeichnet**, daß
die als doppelte bezeichneten Basen aus Bisphenylalkylendiaminen der Formel: 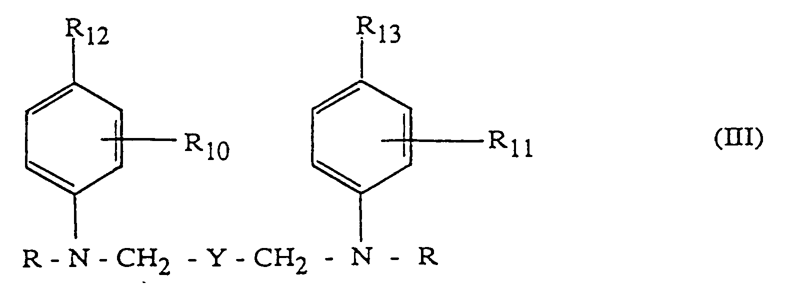 worin gilt:
R₁₂ und R₁₃ stellen, gleich oder verschieden, Hydroxyl- oder NHR₁₄-Gruppen dar, worin R₁₄ ein Wasserstoffatom oder eine Niedrigalkylrest bedeutet;
R₁₀ und R₁₁ stellen, gelich oder verschieden, entweder Wasserstoffatome oder Halogenatome oder auch Alkylgruppen dar;
R stellt ein Wasserstoffatom, eine Alkyl-, Hydroxyalkyl- oder Aminoalkylgruppe dar, deren Aminorest substituiert sein kann;
Y stellt einen Rest aus der Gruppe aus den folgenden Resten dar:
-(CH₂)ₙ-, -(CH₂)_{n'}-O-(CH₂)_{n'}-,
- (CH₂)_{n'}-CHOH-(CH₂)_{n'} , -(CH₂)_{n'}-N-CH₃-(CH₂)_{n'}-,
worin n eine ganze Zahl von 0 bis 8 und n' eine ganze Zahl von 0 bis 4 sind,
sowie aus deren Säureadditionssalzen ausgewählt sind.

8. Verfahren gemäß Anspruch 7,
dadurch **gekennzeichnet**, daß
die als doppelte bezeichnete Base aus den folgenden Verbindungen ausgewählt ist:
N,N'-Bis(β-hydroxyethyl)-N,N'-bis(4'-aminophenyl)-1,3-diaminopropan-2-ol, N,N'-Bis(β-hydroxyethyl)-N,N'-bis(4'-aminophenyl)ethylendiamin, N,N'-Bis(4-aminophenyl)tetramethylendiamin, N,N'-Bis(β-hydroxyethyl)-N,N'-bis(4-aminophenyl)tetramethylendiamin, N,N'-Bis(4-methylaminophenyl)tetramethylendiamin und aus N,N'-Bis(ethyl)-N,N'-bis(4'-amino-3'-methylphenyl)ethylendiamin.

9. Verfahren gemäß Anspruch 1 oder 2,
dadurch **gekennzeichnet**, daß
die Oxidationsfarbstoff-Vorstufenverbindungen Oxidationsfarbstoff-Vorstufenverbindungen vom ortho-Typ sind, ausgewählt aus o-Aminophenolen und o-Phenylendiaminen.

10. Verfahren gemäß jedem der Ansprüche 1 bis 9,
dadurch **gekennzeichnet**, daß
das oxidierende Mittel aus Wasserstoffperoxid, Harnstoffperoxid, Alkalimetallbromaten und aus Persalzen ausgewählt ist.

11. Verfahren gemäß jedem der Ansprüche 1 bis 10,
dadurch **gekennzeichnet**, daß
der pH-Wert der auf die keratinischen Fasern aufgetragenen Zusammensetzung 3 bis 6,9 beträgt.

12. Verfahren gemäß jedem der Ansprüche 1 bis 11,
dadurch **gekennzeichnet**, daß
die zur Färbung der keratinischen Fasern eingesetzte Zusammensetzung zusätzlich zu den heterozyklischen Kupplern der Familie der Aminoindole der Formel (I) weitere Kuppler enthält, die aus m-Diphenolen, m-Aminophenolen, m-Phenylendiaminen, m-N-Acylaminophenolen, m-Ureidophenolen, m-Carbalkoxyaminophenolen, α-Naphthol, aus Kupplern mit einer aktiven Methylengruppe, ausgewählt aus Diketoverbindungen und Pyrazolonen, aus heterozyklischen Kupplern oder aus 4-Hydroxyindol, 6-Hydroxyindol oder 7-Hydroxyindol ausgewählt sind.

13. Verfahren gemäß Anspruch 12,
dadurch **gekennzeichnet**, daß
die Kuppler aus 2,4-Dihydroxyphenoxyethanol, 2,4-Dihydroxyanisol, m-Aminophenol, Resorcin, den Monomethylether von Resorcin, 2-Methylresorcin, Pyrocatechol, 2-Methyl-5-N-(β -hydroxyethyl)aminophenol, 2-Methyl-5-N-(β-mesylaminoethylaminophenol,6-Hydroxybenzomorpholin, 2,4-Diaminoanisol, 2,4-Diaminophenoxyethanol, 6-Aminobenzomorpholin, (2-N-(β-Hydroxyethyl)amino-4-amino)-phenoxyethanol, 2-Amino-4-N-(β-hydroxyethyl)aminoanisol, (2,4-Diamino)phenyl-β,γ-dihydroxypropylether, 2,4-Diaminophenoxyethylamin, 1,3-Dimethoxy-2,4-diaminobenzol, 2-Methyl-5-aminophenol, 2,6-Dimethyl-3-aminophenol, 3,4-Methylendioxyphenol, 3,4-Methylendioxyanilin, 2-Chlorresorcin und deren Salzen ausgewählt sind.

14. Verfahren gemäß jedem der Ansprüche 1 bis 13,
dadurch **gekennzeichnet**, daß
die Zusammensetzung anionische, kationische, nicht-ionische oder amphotere oberflächenaktive Mittel oder deren Mischungen, Verdickungsmittel, Antioxidantien und/oder jeden weiteren kosmetisch geeigneten Hilfsstoff enthält.

15. Verfahren gemäß jedem der Ansprüche 1 bis 14,
dadurch **gekennzeichnet**, daß
das zur Färbung geeignete Milieu aus Wasser oder aus einer Mischung aus Wasser und einem Lösungsmittel zusammengesetzt ist, welches aus C₂₋₄-Niedrigalkanolen, Glycerin, Glycolen, Ethern von Glycolen, aromatischen Alkoholen oder aus deren Mischungen ausgewählt ist.

16. Mittel zur Färbung keratinischer Fasern und insbesondere der Haare,
dadurch **gekennzeichnet**, daß
es mindestens zwei Bestandteile umfaßt: einen Bestandteil (A) aus einer Zusammensetzung, die in einem zur Färbung geeigneten Milieu einen Aminoindol-Kuppler der in Anspruch 1 oder 2 definierten Formel (I) und eine in jedem der Ansprüche 1 und 3 bis 9 definierte Oxidationsfarbstoff-Vorstufenverbindung enthält, sowie einen Bestandteil (B) aus einer Zusammensetzung, die in einem zur Färbung geeigneten Milieu ein oxidierendes Mittel enthält, wobei der pH-Wert der Bestandteile (A) und (B) so eingestellt ist, daß nach Mischung in Mengenanteilen von 90 bis 10% für den Bestandteil (A) und von 10 bis 90% für den Bestandteil (B) die sich ergebende Zusammensetzung einen pH-Wert von weniger als 7 aufweist.

17. Mittel gemäß Anspruch 16,
dadurch **gekennzeichnet**, daß
der Bestandteil (A) einen pH-Wert von 3 bis 10,5 hat.

18. Mittel gemäß Anspruch 16 oder 17,
dadurch **gekennzeichnet**, daß
der Bestandteil (A) die Oxidationsfarbstoff-Vorstufenverbindungen sowie die Kuppler in Mengenanteilen von 0,05 bis 7 Gew.% enthält, bezogen auf das Gesamtgewicht des Bestandteils (A).

19. Mittel gemäß jedem der Ansprüche 16 bis 18,
dadurch **gekennzeichnet**, daß
die Konzentration an Verbindung der Formel (I) 0,012 bis 4 Gew.% ausmacht, bezogen auf das Gesamtgewicht des Bestandteils (A).

20. Mittel gemäß jedem der Ansprüche 16 bis 19,
dadurch **gekennzeichnet**, daß
der Bestandteil (A) oberflächenaktive Mittel in Mengenanteilen von 0,1 bis 55 Gew.%, Lösungsmittel zusätzlich zum Wasser in Mengenanteilen von 0,5 bis 40 Gew.%, Verdickungsmittel in Mengenanteilen von 0,1 bis 5 Gew.%, Antioxidantien in Mengenanteilen von 0,02 bis 1,5 Gew.% und/oder jeden weiteren kosmetisch geeigneten Hilfsstoff enthält.

21. Mittel gemäß jedem der Anspüche 16 bis 20,
dadurch **gekennzeichnet**, daß
der Bestandteil (B) einen pH-Wert in einem Bereich von mindestens 1 bis unterhalb 7 aufweist.

22. Verfahren zur Färbung keratinischer Fasern und insbesondere der Haare,
dadurch **gekennzeichnet**, daß
es eine erste Stufe umfaßt, wobei man ein in jedem der Ansprüche 16 bis 21 definiertes Färbemittel aufbewahrt und vorhält, und wobei man vor der Aufbringung die Bestandteile (A) und (B) in Mengenanteilen von 10 bis 90% für den Bestandteil (A) und von 90 bis 10% für den Bestandteil (B) vermischt, und zwar so, daß man eine Zusammensetzung erhält, die einen pH-Wert von weniger als 7 aufweist, und wobei man diese Mischung unmittelbar nach ihrer Zubereitung auf die keratinischen Fasern aufbringt.

23. Vorrichtung aus mehreren Teilen oder Kit zur Färbung,
dadurch **gekennzeichnet**, daß
sie mindestens zwei Teile umfassen, wovon ein erster Teil den in jedem der Ansprüche 16 bis 20 definierten Bestandteil (A) und der zweite Teil den in jedem der Ansprüche 16 bis 21 definierten Bestandteil (B) einschließen.

24. Vorrichtung gemäß Anspruch 23,
dadurch **gekennzeichnet**, daß
sie mit einem Element ausgerüstet ist, welches es ermöglicht, die erwünschte Mischung aus den Bestandteilen (A) und (B) freizusetzen und auf die Haare aufzubringen.

25. Verfahren gemäß jedem der Ansprüche 1 bis 15,
dadurch **gekennzeichnet**, daß
man auf die Haare die Zusammensetzung aufbringt und sie 3 bis 40 Minuten lang verweilen läßt, die Haare spült und gegebenenfalls eine Schamponierung vor einer erneuten Spülung durchführt und die Haare dann trocknet.

26. Verwendung der Aminoindol-Verbindungen der in Anspruch 1 oder 2 definierten Formel (I) als Kuppler zur Färbung keratinischer Fasern in saurem Milieu, zusammen mit Oxidationsfarbstoff-Vorstufenverbindungen.

27. Gebrauchsfertige Zusammensetzung zur Färbung keratinischer Fasern zur Anwendung in dem Verfahren gemäß jedem der Ansprüche 1 bis 15.

28. Zusammensetzung gemäß Anspruch 27,
welche die Verbindung der Formel (I) in Mengenanteilen von 0,01 bis 3,5 Gew.% enthält, bezogen auf das Gesamtgewicht der Zusammensetzung.

29. Färbezusammensetzung für keratinische Fasern, insbesondere für Haare,
dadurch **gekennzeichnet**, daß
sie in einem zur Färbung geeigneten Milieu mindestens eine Oxidationsfarbstoff-Vorstufenverbindung oder einen Kuppler der folgenden Formel: 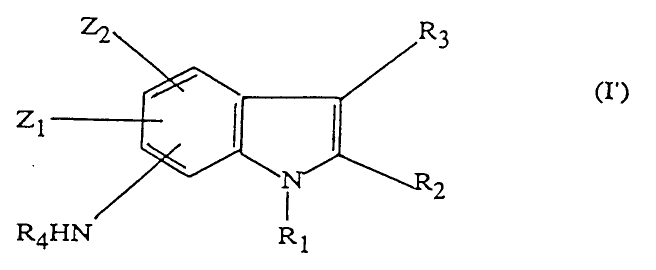 worin gilt:
R₁ bedeutet ein Wasserstoffatom oder eine C₁₋₄-Alkylgruppe;
R₂ und R₃ stellen, unabhängig voneinander, ein Wasserstoffatom, eine C₁₋₄-Alkyl- oder eine COOR'-Gruppe dar, wobei R' ein C₁₋₄-Alkylrest oder ein Wasserstoffatom ist;
R₄ stellt ein Wasserstoffatom, einen C₁₋₄-Alkylrest, eine Hydroxy-C₁₋₄-alkyl-, Polyhydroxy-C₂₋₄-alkyl-, Acetyl- oder eine Amino-C₁₋₆-alkylgruppe dar, wobei das Amin gegebenenfalls mit einem C₁₋₄-Alkylrest mono- oder disubstituiert sein kann;
Z₁ und Z₂ stellen, gleich oder verschieden, ein Wasserstoffatom oder eine C₁₋₄-Alkyl-, Hydroxy-, Halogen- oder eine C₁₋₄-Alkoxygruppe dar;
mit der Maßgabe, daß, wenn NHR₄ in Position 4, 6 oder 7 vorliegt, Z₁ und Z₂ nicht gleichzeitig ein Wasserstoffatom bedeuten, sowie die Salze dieser Verbndungen enthält, ausgenommen 6-Hydroxy-5-acetylamino-2,3-dimethylindol.

30. Färbemittel gemäß jedem der Ansprüche 16 bis 20,
dadurch **gekennzeichnet**, daß
der Bestandteil (A) als Kuppler ein Aminoindol der folgenden Formel: 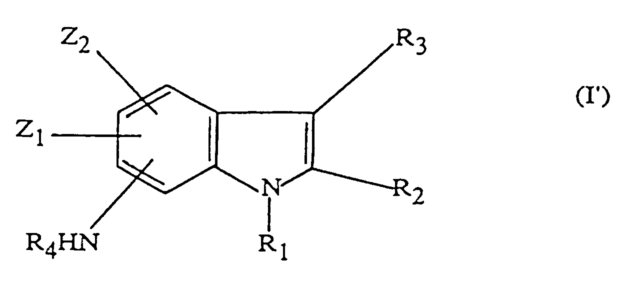 worin gilt:
R₁ bedeutet ein Wasserstoffatom oder eine C₁₋₄-Alkylgruppe;
R₂ und R₃ stellen, unabhängig voneinander, ein Wasserstoffatom, eine C₁₋₄-Alkyl- oder eine COOR'-Gruppe dar, wobei R' ein C₁₋₄-Alkylrest oder ein Wasserstoffatom ist; wobei mindestens einer der Reste R₂ und R₃ ein Wasserstoffatom bedeutet;
R₄ stellt eine C₁₋₄-Alkyl-, Hydroxy-C₁₋₄-alkyl- oder eine Polyhydroxy-C₂₋₄-alkylgruppe dar, wobei die -NHR₄-Gruppe die Positionen 4, 6 oder 7 besetzt;
Z₁ und Z₂ stellen, gleich oder verschieden, ein Wasserstoffatom, einen C₁₋₄-Alkylrest, einen Hydroxyrest, ein Halogenatom oder eine C₁₋₄-Alkoxygruppe dar;
mit der Maßgabe, daß, wenn NHR₄ in Position 4, 6 oder 7 vorliegt, Z₁ und Z₂ nicht gleichzeitig ein Wasserstoffatom bedeuten, sowie die Salze dieser Verbindungen enthält.

31. Verfahren zur Herstellung einer Verbindung der folgenden Formel: 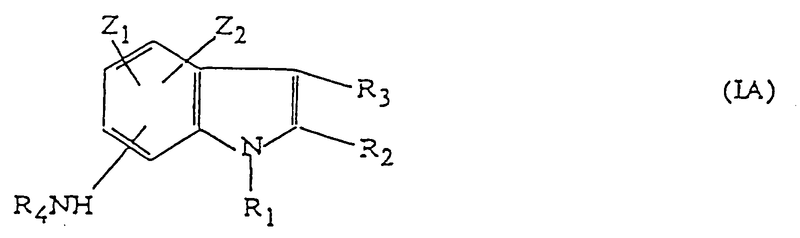 worin gilt:
R₁ bedeutet ein Wasserstoffatom oder eine C₁₋₄-Alkylgruppe;
R₂ und R₃ stellen, unabhängig voneinander, ein Wasserstoffatom, eine C₁₋₄-Alkykl- oder COOR'-Gruppe dar, worin R' ein Wasserstoffatom oder einen C₁₋₄-Alkylrest bedeutet, wobei mindestens einer der Reste R₂ und R₃ ein Wasserstoffatom bedeutet;
R₄ stellt eine C₁₋₄-Alkyl-, Hydroxy-C₁₋₄-alkyl- oder Polyhydroxy-C₂₋₄-alkylgruppe dar, wobei die -NHR₄-Gruppe die Positionen 4, 6 oder 7 besetzt;
Z₁ und Z₂ stellen, gleich oder verschieden, ein Wasserstoffatom, einen C₁₋₄-Alkylrest, einen Hydroxyrest, ein Halogenatom oder eine C₁₋₄-Alkoxygruppe dar, wobei mindestens eine der Gruppierungen Z₁ und Z₂ von Wasserstoff verschieden ist,
sowie der Salze dieser Verbindung,
dadurch **gekennzeichnet**, daß
die Verbindungen durch Tosylierung oder Formylierung einer Verbindung der Formel: 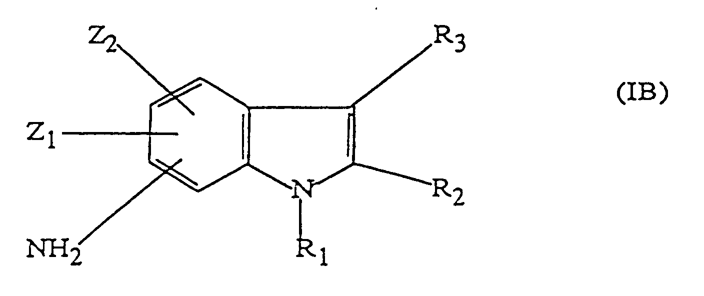 worin R₁, R₂, R₃, Z₁ und Z₂ die oben angegebenen Bedeutungen haben, unter anschließender Alkylierung mit einem Alkylhalogenid und dann unter gemäß herkömmlicher Verfahren durchgeführter Enttosylierung oder Entformylierung erhalten werden.

32. Verfahren zur Herstellung der Verbindungen 7-Ethyl-4-aminoindol und 7-Ethyl-6-aminoindol,
dadurch **gekennzeichnet**, daß
sie durch jeweilige Reduktion von 7-Ethyl-4-nitroindol bzw. von 7-Ethyl-6-nitroindol erhältlich sind.
